# EUROPEAN PATENT SPECIFICATION

(11) **EP 1 644 343 B1**
(45) Date of publication and mention of the grant of the patent: **04.07.2007**
(21) Application number: 04740421.5
(22) Date of filing: 29.06.2004
(51) Int. Cl.: C07D 239/95, A61K 31/517, C07D 405/12, C07D 401/12, C07D 403/12, C07D 487/04, A61P 13/00

(54) **5-SUBSTITUTED QUINAZOLINONE DERIVATIVES**
5-SUBSTITUIERTE CHINAZOLINONDERIVATE
DERIVES DE QUINAZOLINONE SUBSTITUES EN POSITION 5

(30) Priority: 02.07.2003 US 484536 P
(43) Date of publication of application: 12.04.2006
(73) Proprietor: F. HOFFMANN-LA ROCHE AG, 4070 Basel (CH)
(72) Inventor: Chin, Elbert, San Jose, California 95117 (US); COURNOYER, Richard, Leo, San Francisco, CA 94127 (US); KEITZ, Paul, Francis, Redwood City, CA 94062 (US); LEE, Eun, Kyung, Mountain View, CA 94040 (US); LOPEZ-TAPIA, Francisco, Javier, Union City, CA 94587 (US); MELVILLE, Chris, Richard, Palo Alto, CA 94306 (US); PADILLA, Fernando, Fremont, CA 94555 (US); WEINHARDT, Klaus, Kurt, Palo Alto, CA 94303 (US)
(74) Representative: Heiroth, Ulrike Hildegard
(86) International application number: PCT/EP2004/007027
(87) International publication number: WO 2005/005397

(56) References cited:
- WO-A-98/30560
- WO-A-02/053558
- LEONARDI A ET AL: "Synthesis, pharmacological evaluation, and structure-activity relationship and quantitative structure-activity relationship studies on novel derivatives of 2,4-diamino-6,7- dimethoxyquinazoline alpha1-adrenoceptor antagonists" JOURNAL OF MEDICINAL CHEMISTRY, AMERICAN CHEMICAL SOCIETY. WASHINGTON, US, vol. 42, no. 3, 11 February 1999 (1999-02-11), pages 427-437, XP002152416 ISSN: 0022-2623

## Description

This invention relates to quinazolinone compounds, more particularly, to substituted quinazolinone compounds and salts thereof which are useful as alpha-1-adrenergic receptor antagonists. The invention further relates to pharmaceutical compositions containing said compounds and to methods for their use as therapeutic agents.

Alpha-1-adrenergic receptors are G-protein coupled transmembrane receptors that mediate various actions of the sympathetic nervous system through the binding of the catecholamines, epinephrine, and norepinephrine (NE). Currently, several subtypes of the alpha-1 adrenergic receptors are known to exist for which the genes have been cloned: alpha-1A (previously known as alpha-1C), alpha-1B and alpha-1D.

Alpha-1 adrenoceptor antagonists have been shown in numerous clinical studies to be effective in relieving the symptoms associated with benign prostatic hypertrophy, also known as benign prostatic hyperplasia (BPH), an illness typically affecting men over fifty. The symptoms of the condition include, but are not limited to, increased difficulty in urination and sexual dysfunction. Drugs such as prazosin, indoramin, doxazosin and tamsulosin are in common clinical use for BPH, and are effective in reducing both "obstructive" symptoms (e.g. weak stream) and "irritative" symptoms (e.g. nocturia, urinary urge and frequency). However, these compounds are all non-subtype-selective and have the potential to cause significant side-effects, particularly cardiovascular effects such as postural hypotension, dizziness, and syncope, and CNS effects such as asthenia (tiredness). These effects can limit dosing and the clinical efficacy in reducing symptoms associated with BPH.

Pharmacological studies resulting in the subdivision of alpha-1 adrenoceptors into alpha-1A, alpha-1B, and alpha-1D adrenoceptors have led to the suggestion that development of subtype-selective antagonists may allow for an improved symptomatic treatment of BPH with a lower incidence of dose-limiting side-effects. Recently, much interest has been focused on the role of the alpha-1A adrenoceptor subtype in BPH, as studies have shown that this subtype predominates in the urethra and prostate of man and appears to be the receptor mediating NE-induced smooth muscle contraction in these tissues. See, e.g., Price et al., J. Urol. 150:546-551 (1993); Faure et al., Life Sci. 54:1595-1605 (1994); Taniguchi et al., Naunyn Schmiedeberg's Arch. Pharmacol. 355:412-416 (1997), Forray et al., Mol. Pharmacol. 45:703-708 (1994); Hatano et al., Br. J. Pharmacol. 113:723-728 (1994); and Marshall et al., Br J. Pharmacol. 115:781-786 (1995). Smooth muscle tone is believed to contribute substantially to the total urinary outflow obstruction observed in patients with BPH [Furuya et al., J. Urol. 128:836-839 (1982)]. Increased prostate mass is also a contributing factor. These observations have fuelled the hypothesis that an alpha-1A subtype-selective antagonist may, via a selective and significant decrease in outlet resistance, lead to improved pharmacotherapy for BPH.

However, in BPH, it is often the irritative symptoms which prompt the patient to seek treatment, and these irritative symptoms may be present in patients with no demonstrable obstruction (i.e. normal urine flow rates). Thus, it would be advantageous to provide a therapy for treating patients exhibiting obstructive symptoms and/or irritative symptoms. It is believed that reduction of obstructive and irritative symptoms in patients with BPH may be achieved via a combination of alpha-1A and alpha-1B subtype selectivity in a drug molecule. The lack of alpha-1D adrenoceptor antagonism is expected to lead to reduced or fewer side effects than those associated with the use of non-subtype-selective agents.

All publications, patents, and patent applications cited herein, whether *supra* or *infra,* are each hereby incorporated by reference in its entirety.

The present invention is directed to compounds of the Formula I 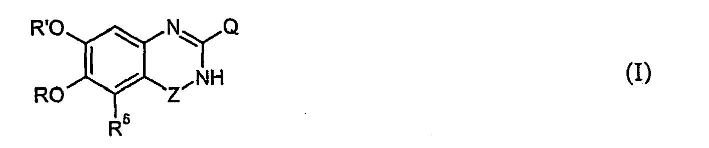 wherein
- Z: is -C(=O)-or-S(=O)₂-;
- R and R': are lower alkoxy;
- R⁵: is selected from halogen, cyano, hydroxy, optionally substituted phenyl, -R⁶, and -OR⁶;
R⁶ is alkyl, alkoxyalkyl, hydroxyalkyl, optionally substituted benzyloxyalkoxy optionally substituted phenoxy, aminoalkyl, optionally substituted aryl, optionally substituted heteroaryl, cycloalkyl, or cycloalkyloxy; and
- Q: is a monocyclic or bicyclic heterocyclic ring selected from (S), (T), (U), and (V) 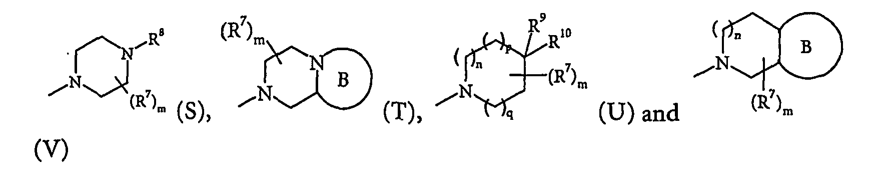
wherein
- B: is an optionally-substituted fused aryl or heteroaryl ring;
- R⁷: is attached to any available carbon atom of the piperidinyl or piperazinyl ring and at each occurrence is independently selected from alkyl, substituted alkyl, halogen, cyano, hydroxy, alkoxy, haloalkoxy, amino, and alkylamino; or alternatively, wherein Q is ring (T), two R⁷ groups attached to different carbon atoms may be taken together to form a carbon-carbon bridge of one to two bridgehead carbon atoms;
- R⁸: is -K-R¹⁴;
- R⁹ and R¹⁰: are (i) independently selected from -L-R¹⁵, or alternatively, (ii) R⁹ and R¹⁰ are taken together to form an optionally-substituted spiroryclic ring;
- K and L: are independently selected from a bond, optionally-substituted C₁₋₄alkylene, -M₁-O-M₂-, -M₁-C(=O)-M₂-, -M₁-C(O)₂-M₂-, -M₁-C(=O)NR¹⁶-M₂-, and -M₁-NR¹⁶-M₂-, wherein M₁ and M₂ are selected from a bond and optionally-substituted C₁₋₄alkylene;
- R¹⁴: and R¹⁵ are independently selected from hydrogen, optionally-substituted alkyl, aryl, heteroaryl, cycloalkyl, or heterocyclo, provided that if K or L is a bond or -NR¹⁶-, then R¹⁴ and R¹⁵ are not selected from phenyl, pyridyl, or pyrimidinyl having a para substituent that is CO₂R²², wherein R²² is selected from hydrogen, alkyl, aryl, araylalkyl, guanidinyl, hydroxy, alkoxy, aryloxy, and arylalkyloxy;
- R¹⁶: is selected from hydrogen and alkyl;
- *m*: is 0, 1, 2, 3, or 4;
- *n*: is 0 or 1;
- *p* is: 0, 1 or 2; and
- *q* is: 0 or 1; or
an isomer or pharmaceutically-acceptable salt, hydrate, or prodrug thereof.

The compounds of Formula (I) above are surprisingly advantageous in selectively antagonizing the alpha-1A and alpha-1B subtype receptors with selectively lesser activity in antagonizing the alpha-1D adrenergic receptor. Accordingly, said compounds of Formula (I) are surprisingly advantageous for use in treating diseases responsive to alpha-1A and alpha-1B receptor antagonism with reduced side effects.

Another aspect of this invention relates to the use of compounds of Formula (I) in the treatment of a subject having a disease state that is alleviated by treatment with an alpha 1A/B adrenergic receptor antagonist, which comprises administering to such a subject in need of treatment therefor, a therapeutically effective amount of at least one compound of Formula I.

Unless otherwise stated, the following terms used in this Application, including the specification and claims, have the definitions given below. It must be noted that, as used in the specification and the appended claims, the singular forms "a", "an," and "the" include plural referents unless the context clearly dictates otherwise.

In general, the nomenclature used in this Application is based on AutoNom^{®}, a Beilstein Institute computerized system for the generation of IUPAC systematic nomenclature.

Chemical structures shown herein were prepared using ISIS^{®} version 2.2. Any open valency shown on a carbon, nitrogen or oxygen in the structures herein indicates the presence of a hydrogen.

As used herein, the term "alkyl" means a linear or branched, saturated monovalent hydrocarbon moiety of one to eight carbon atoms (preferably one to six carbon atoms), *e.g.,* methyl, ethyl, *n*-propyl, 2-propyl, *tert-*butyl, pentyl, and the like. "Lower alkyl" means an alkyl of one to four carbon atoms. When a subscript is used herein following a carbon atom, the subscript refers to the number of carbon atoms the named group may contain. Thus, e.g., C₁₋₄alkyl means an alkyl of one to four carbon atoms (*i.e.,* lower alkyl) including methyl, ethyl, propyl, iso-propyl, butyl, and tert-butyl; hydroxy(C₁₋₄)alkyl means an alkyl of one to four carbon atoms substituted with a hydroxy group; and so forth.

"Alkylene" means a linear or branched, saturated divalent hydrocarbon moiety of one to eight (preferably one to six) carbon atoms, *e.g.,* methylene, ethylene, propylene, and the like. When reference is made to a linker group including an alkylene, *e.g.,* as in -M₁-C(=O)-M₂-, -M₁-C(O)₂-M₂-, -M₁-C(=O)NR-M₂-, etc. wherein M₁ and M₂ may be selected from optionally substituted alkylene, it should be understood that the alkylene may be a straight or branched-chain alkylene.

When the term "alkyl" is used as a suffix following another term, as in "phenylalkyl," or "hydroxyalkyl," this is intended to refer to an alkyl group, as defined above, being substituted with one or more (preferably one) substituent selected from the other, specifically-named group. Thus, "phenylalkyl" includes benzyl, phenylethyl, 2-phenylbutyl, and so forth. "Hydroxyalkyl" includes 2-hydroxyethyl, 1-(hydroxymethyl)-2-methylpropyl, 3,4-dihydroxybutyl, and so forth. Accordingly, as used herein, the term "hydroxyalkyl" is used to define a subset of heteroalkyl groups defined below.

The term "substituted alkyl" refers to an alkyl group as defined above having one, two, three or four substituents (preferably one to two substituents), independently selected from the group consisting of halo, haloalkoxy, trifluoromethyl, cyano, nitro, -R^{d}, -OR^{a}, -SR^{a}, -S(O)R^{c}, -S(O)₂R^{c}, -C(=O)R^{a}, -C(=O)NR^{a}R^{b}, -C(O)₂R^{a}, -C(O)₂NR^{a}R^{b}, -S(O)₂NR^{a}R^{b} and -NR^{a}R^{b}, wherein R^{a} and R^{b} are independently selected from hydrogen, C₁₋₆alkyl, benzyl, aryl, heteroaryl, cycloalkyl, and heterocyclo; R^{c} is selected from C₁₋₆alkyl, benzyl, aryl, heteroaryl, cycloalkyl, and heterocyclo; and R^{d} is selected from aryl, heteroaryl, cycloalkyl, and heterocyclo, and each of R^{a}, R^{b}, R^{c} and R^{d} in turn is optionally substituted with one, two, or three of alkyl, halo, haloalkyl, hydroxy, O(alkyl), haloalkoxy, cyano, amino, alkylamino, SO₂(alkyl), CO₂H, CO₂(alkyl), C(=O)H, C(=O)alkyl, -C(=NR^{e})-R^{f}, -N(R^{e})-C(=NR^{e})-R^{f}, -N=C(R^{e})-NR^{f}R^{g}, and -N=R^{f}, and/or a C₁₋₆alkyl substituted with one to two of halo, hydroxy, O(alkyl), haloalkoxy, trifluoromethyl, cyano, amino, alkylamino, -SO₂(alkyl), CO₂H, CO₂(alkyl), C(=O)H, and/or C(=O)alkyl, -C(=NR^{e})-R^{f}, -N(R^{e})-C(=NR^{e})-R^{f}, -N=C(R^{e})-NR^{f}R^{g}, and/or -N=R^{f}, wherein

R^{e} is hydrogen or lower alkyl, and R^{f} and R^{g} are selected from hydrogen, alkyl, C₁₋₄alkylene-OH, and C₁₋₄alkylene-O(alkyl).

The term "substituted lower alkyl" means an alkyl of one to four carbon atoms having one, two, or three substituents selected from halo, haloalkyl, hydroxy, O(alkyl), haloalkoxy, cyano, amino, alkylamino, SO₂(alkyl) CO₂H, CO₂(alkyl), C(=O)H, and/or C(=O)alkyl.

The term "substituted alkylene" means an alkylene group as defined above wherein one, two or three carbon atoms of the alkylene straight or branched chain is substituted with a group selected from halo, haloalkyl, hydroxy, O(alkyl), haloalkoxy, cyano, amino, alkylamino, SO₂(alkyl), CO₂H, CO₂(alkyl), C(=O)H, and/or C(=O)alkyl.

"Alkoxy" refers to the group OR', wherein R' is alkyl or substituted alkyl. A "lower alkoxy" is a group -OR' wherein R' is C₁₋₄alkyl.

"Amidinyl" means a group of the formula 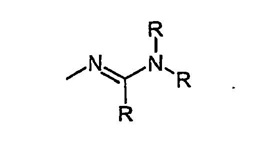 wherein each R is independently hydrogen or alkyl as defined herein. Amidinyl includes acetamidinyl, formamidinyl, butyramidinyl-, cyclobutanecarboxamidinyl-, furan-2-yl-carboxamidinyl-, N,N-dimethylacetamidinyl-, and the like. "Alkoxyalkyl" means a group R-OR' wherein R is alkylene as defined herein and R' is alkyl as defined herein.

When the term "oxy" is used as a suffix following another specifically-named group, as in "aryloxy", "heteroaryloxy," or "arylalkyloxy", this means that an oxygen atom is present as a linker to the other, specifically-named group. Thus, e.g., "aryloxy" refers to the group -O-R, wherein R is aryl; "heteroaryloxy" refers to the group -O-R', wherein R' is heteroaryl; and "arylalkyloxy" refers to the group -O-R", wherein R" is arylalkyl such as benzyl. Similarly, a "substituted aryloxy" means the group -O-R, wherein R is substituted aryl, and a "substituted heteroaryloxy" means the group -O-R', wherein R' is substituted heteroaryl. Phenoxy is an example of aryloxy, the phenyl moiety of which may be optionally substituted as defined below for "aryl".

"Alkylcarbonyl" means a group -(C=O)-R wherein R is alkyl as defined herein.

"Alkoxylcarbonyl" means a group -(C=O)-R wherein R is alkoxy as defined herein.

"Alkoxycarbonylalkyl" means a group -R'-(C=O)-R wherein R' is alkylene as defined herein and R is alkoxy as defined herein.

"Dialkylaminocarbonyl" means a group -(C=O)-NRR wherein R is alkyl as defined herein.

"Phenylaminocarbonyl means a group -(C=O)-NRR' wherein R is hydrogen or alkyl and R' is phenyl, which may be optionally substituted in the manner described below for "aryl".

"Cycloalkylalkylcarbonyl means a group (C=O)-R-R' wherein R is alkylene as defined herein and R' is cycloalkyl as defined herein.

"Amino" refers to the group NH₂. Thus, an aminoalkyl refers to an alkyl group having an amino substituent, *e.g.,* -CH₂-NH₂, -CH₂-CH(NH₂)-CH₃, -CH₂-CH(CH₂NH₂)-CH₃, and so forth. "Alkylamino" refers to monoalkylamino groups having the formula -NHR, as well as dialkylamino groups having the formula -NRR, wherein each R is an alkyl group. A "lower aminoalkyl" refers to a group -NHR' or -NR'R', wherein each R' is C₁₋₄alkyl. A "substituted alkylamino" refers to an alkylamino wherein the alkyl portion is substituted from a group selected from those recited above for substituted alkyl groups.

"Aminoalkyl" means a group of the formula R-NH₂, -R'-NHR or R'-NRR wherein R' is alkylene as defined herein and each R is alkyl as defined herein. The group -R'-NHR may be more specifically referred to herein as "alkylaminoalkyl", and the group R'-NRR may be more specifically referred to herein as "dialkylaminoalkyl".

"Alkylaminoalkylcarbonylaminoalky" means a group -R-NR'-(C=O)-R-NHR" wherein each R is alkylene as defined herein, R' is hydrogen or alkyl, and R" is alkyl.

The term "aryl" refers to a monovalent, monocyclic or bicyclic moiety in which at least one of the rings is an aromatic, carbocyclic moiety. Thus, the term "aryl" includes phenyl, 1-naphthyl, and 2-naphthyl. The term "aryl" also includes phenyl rings having fused thereto a second non-aromatic carbocyclic ring, or second fused heteroaryl or heterocyclic ring (thus, the term aryl includes groups such as benzothienyl, benzopyrazolyl, benzopiperadinyl, benzocyclohexyl, and the like), with the understanding, however, that in the case of bicyclic aryl groups, the point of attachment will be to a phenyl or benzo ring.

A "substituted aryl" is an aryl group as defined above having one to four (preferably one to two) substituents independently selected from the group consisting of halo, haloalkyl, haloalkoxy, cyano, hydroxy, alkoxy, -A₁-R^{p}, -A₁-NR^{q}R^{r}, -A₁-C(=O)R^{q}, -A₁-C(O)₂R^{q}, -A₁-C(=O)NR^{q}R^{r}, -A₁-C(O)₂NR^{q}R^{r}, -A₁-S(O)₀₋₂R^{q}, -A₁-NR^{s}S(O)₂R^{p}, -A₁-N(S(O)₂R^{q})₂, -A₁-S(O)₂NR^{q}R^{r}, -A₁-NR^{s}C(=O)NR^{q}R^{r}, -A₁-NR^{q}C(=O)R^{r}, -A₁-NR^{q}-A₂-NR^{r}C(=O)R^{t}, -A₁-C(=NH)-A₂-R^{p}, -A₁-NR^{s}-C(=NR^{s})-A₂-R^{p}, -A₁-N=CR^{s}-NR^{s}-A₂- R^{q} and/or -A₁-N=C-(R^{u})R^{v} wherein A₁ and A₂ are independently selected from a bond and optionally substituted C₁₋₄alkylene; R^{p} is selected from alkyl, aryl, heteroaryl, heterocyclo, and cycloalkyl; each R^{q}, R^{r} and R^{t} is independently selected from hydrogen, alkyl, aryl, heteroaryl, cycloalkyl, and heterocyclo, except when the substituent is -A₁-S(O)₁₋₂R^{q}, then R^{q} in these instances will not be hydrogen; each R^{s} is independently hydrogen, lower alkyl, or hydroxyC₁₋₄alkyl; and R^{u} and R^{v} are taken together to form a cycloalkyl or heterocyclo ring. In each instance, each of R^{p}, R^{q}, R^{r}, R^{t} and/or R^{u} and R^{v} in turn is optionally substituted with up to three groups selected from alkyl, halo, cyano, hydroxy, alkoxy, haloalkyl, haloalkoxy, amino, alkylamino, aminoalkyl, alkylaminoalkyl, hydroxyalkyl, alkoxyalkyl, -SO₂(alkyl), CO₂H, CO₂(alkyl), C(=O)H, C(=O)alkyl, pyrrolidinyl, and phenyl, said phenyl and pyrrolidinyl in turn being optionally substituted with one to two of lower alkyl, lower alkoxy, cyano and/or halogen. A preferred group of aryl substituents are those selected from -R^{p}, -(C₁₋₄alkylene)-R^{p}, -C(=NH)-R^{p}, -NR^{s}-C(=NR^{s})-R^{p}, -N=CR^{s}-NR^{s}-R^{p}, and -N=R^{p}, wherein R^{s} is hydrogen or lower alkyl; and R^{p} is selected from alkyl, pyrrolidinyl, pyrrolinyl, imidazolinyl, imidazolidinyl, morpholinyl, cyclobutyl, cyclopentyl, cyclohexyl, and furyl (except in the case of-N=R^{p}, then R^{p} will not be furyl), wherein each R^{p} in turn is optionally substituted with one to two groups selected from lower alkyl, halogen, cyano, halo(C₁₋₄)alkyl, halo(C₁₋₄)alkoxy, hydroxy, lower alkoxy, amino, (C₁₋₄alkyl)amino, (C₁₋₄alkyl)aminoalkyl, hydroxy(C₁₋₄)alkyl, (loweralkoxy)(C₁₋₄)alkyl, SO₂(C₁₋₄alkyl),-C(=O)H, -C(=O)(C₁₋₄ alkyl), pyrrolidinyl, and phenyl (said phenyl in turn being optionally substituted with one to two of lower alkyl, lower alkoxy, cyano, and/or halogen). "Optionally substituted aryl", such as optionally substituted phenyl means an aryl that is optionally substituted with from one to four (preferably one or two) of the above substituents.

The term "carbocyclic" means a cyclic moiety in which all ring atoms are carbon atoms, including saturated, partially unsaturated, and unsaturated rings.

The term "spirocyclic ring" refers to saturated and partially saturated carbocyclic or heterocyclic rings wherein the spirocyclic ring is attached to another moiety via a carbon ring atom. One or two ring carbon atoms of the "spirocyclic ring" may be replaced with a carbonyl (-C(=O)-) group. The term "spirocyclic ring" includes such rings having a second ring fused thereto, wherein the second fused ring may be an aryl, cycloalkyl, heteroaryl or heterocyclo ring, provided, however, that the term "spirocyclic ring" is not intended to encompass bicyclic ring systems in which a tetrahydrofuryl group has a six membered aromatic ring fused thereto (i.e., wherein the tetrahydrofuryl group is attached in a spiro fashion as in 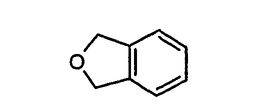 attached in a spiro fashion at the tetrahydrofuryl group). An "optionally-substituted spirocyclic ring" means the spirocyclic ring portion of this moeity may have one, two or three substituents selected from those recited herein for cycloalkyl and heterocyclo groups, and in the situation where the optionally-substituted spirocyclic ring has a second ring fused thereto, said second ring optionally may have one, two or three substituents selected from those recited herein for aryl, heteroaryl, cycloalkyl, and heterocyclo rings, as appropriate given the genus of which the second fused ring is a member.

The term "cycloalkyl" as used herein refers to saturated or partially unsaturated, monovalent, monocyclic carbocyclic moieties of three to seven ring carbon atoms and further includes such rings having a carbon-carbon bridge of one, two, or three bridgehead carbon atoms, and/or having a second ring fused thereto, with the understanding that the point of attachment will be to the non-aromatic carbocyclic ring moeity. Thus, the term "cycloalkyl" includes such rings as cyclopropyl, cyclopentyl, cyclopentenyl, cyclohexyl, cyclohexenyl, and the like. Additionally, one or two carbon atoms of a cycloalkyl group may optionally contain a carbonyl oxygen group, e.g., one or two atoms in the ring may be a moiety of the formula -C(=O)-.

"Benzylamino" means a group -NHR-R' or -NRR-R' wherein each R is alkyl, R' is benzyl.

"Benzyloxy" means a group -O-R wherein R is benzyl.

A "substituted cycloalkyl" is a cycloalkyl group as defined above having one to four (preferably one to two) substituents independently selected from the group of substituents recited above for substituted aryl groups.

"Cycloalkyloxy" means a group -OR wherein R is cycloalkyl as defined herein.

The term "amidinyl" as used herein refers to the group -N=C(R)NH₂, wherein R is hydrogen or lower alkyl. The term alkyl-amidinyl refers to the group -N=C(R)NR'R", wherein R is hydrogen or lower alkyl and R' and R" are selected from hydrogen and lower alkyl, provided that at least one of (or both of) R' and R" is lower alkyl.

"Guanidinyl" means a group -NR-(C=NR)-NRR wherein each R is independently hydrogen or alkyl.

"Furanylcarbonyl" means a group of the formula -(C=O)-R wherein R is furanyl, preferably furan-2-yl, the furanyl moiety of which may be optionally substituted. "Morpholinylcarbonyl" means a group of the formula -(C=O)-R wherein R is morpholinyl, preferably morpholin-4-yl.

"Imidazolinyl" means a 4,5-dihydro-imidazolyl. Imidazolinyl may be optionally substituted in the manner defined herein for heteroaryl.

"Imidazolylalkyl" means a group -R-R' wherein R is alkylene as defined herein and R' is imidazolyl, which may be optionally substituted as described for heteroaryl.

"Imidazolinylalkyl" means a group -R-R' wherein R is alkylene as defined herein and R' is imidazolinyl as defined herein.

"Iminomorpholinylmethyl" means a group -(C=NR)-R' wherein R is hydrogen or alkyl, and R' is morpholinyl, preferably morpholin-4-yl.

"Pyrrolidinylidineamino" means a group 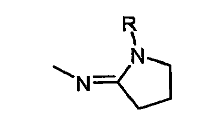 wherein R is hydrogen or alkyl.

The term "halo," "halide" or "halogen" when referring to a substituent means fluoro, chloro, bromo, or iodo (preferably fluoro or chloro).

The term "haloalkyl" means alkyl substituted with one or more same or different halo atoms, *e.g.*, -CHF₂, -CF₃, -CH₂CF₃, -CH₂CCl₃, and the like, and further includes those alkyl groups such as perfluoroalkyl in which all alkyl hydrogen atoms are replaced by fluorine atoms.

The term "haloalkoxy" means a haloalkyl group as defined above linked through an oxygen atom, e.g., it includes -O-CH₂Cl, -O-CF₃, -O-CH₂CF₃, -O-CH₂CCl₃, and the like.

"Hydroxyalkyl" means a group -R-OH wherein R is alkylene as defined herein.

"Heterocyclo" "heterocyclyl" or "heterocyclic" refers to a saturated or partially-unsaturated non-aromatic monocyclic or bicyclic moiety in which one or two ring atoms are heteroatoms selected from N, O, or S(O)ₓ (where x is an integer from 0 to 2), the remaining ring atoms being carbon atoms, and additionally, one or two carbon atoms may optionally contain a carbonyl oxygen group, e.g., one or two atoms in the ring may be a moiety of the formula -C(=O)-. Thus, the term heterocyclo includes rings such as tetrahydropyranyl, tetrahydrofuryl, piperidinyl, piperazinyl, morpholinyl, pyrrolidinyl, pyrrolinyl, imidazolidinyl, imidazolinyl, tetrahydropyrimidinyl and the like. In the case of a bicyclic heterocyclo, one of the two rings may be a carbocyclic, heteroaromatic or aromatic ring, with the understanding that in such cases the point of attachment will be to the non-aromatic heterocyclic ring. Heterocyclics may be optionally substituted one, two or three times with alkyl, alkoxy, halo or haloalkyl.

A "substituted heterocyclo" or "substituted heterocycle" refers to a heterocyclo group as defined above having one to four substituents (preferably one to two substituents) selected from the group of substituents recited above for substituted aryl groups.

"Heteroaryl" means a monovalent, monocyclic aromatic moiety of 5 to 6 ring atoms containing one, two, three, or four ring heteroatoms, each independently selected from N, O, N(O) or S, the remaining ring atoms being carbon, and it also includes such rings having a second ring fused thereto of five to six ring atoms, wherein the second fused ring may be aromatic or nonaromatic and may be carbocyclic, heterocyclic, or a heteroaryl ring, with the understanding, however, that in such cases the point of attachment will be to an aromatic ring containing at least one heteroatom. Thus, the term heteroaryl includes, but is not limited to, pyridyl, pyridinyl, furyl, thiophenyl, thiazolyl, isothiazolyl, triazolyl, imidazolyl, isoxazolyl, pyrrolyl, pyrazolyl, pyrimidinyl, benzofuryl, isobenzofuryl, benzothiazolyl, benzoisothiazolyl, benzotriazolyl, indolyl, isoindolyl, benzoxazolyl, quinolyl, isoquinolyl, benzimidazolyl, benzisoxazolyl, benzothiophenyl, dibenzofuran, and benzodiazepin-2-one-5-yl, and derivatives thereof.

A "substituted heteroaryl" is a heteroaryl ring as defined above having one to four (preferably one or two) substituents selected from the group of substituents recited above for substituted aryl groups. Preferred substituents for substituted heteroaryl groups include those selected from -R^{p}, -(C₁₋₄alkylene)-R^{p}, -C(=NH)-R^{p}, -NR^{s}-C(=NR^{s})-R^{p}, -N=CR^{s}-NR^{s}-R^{p}, and -N=R^{p}; wherein R^{s} is hydrogen or lower alkyl; and R^{p} is selected from alkyl, pyrrolidinyl, pyrrolinyl, imidazolinyl, imidazolidinyl, morpholinyl, cyclobutyl, cyclopentyl, cyclohexyl, and furyl (except in the case of -N=R^{p}, then R^{p} will not be furyl), wherein each R^{p} in turn is optionally substituted with one to two groups selected from lower alkyl, halogen, cyano, halo(C₁₋₄)alkyl, halo(C₁₋₄)alkoxy, hydroxy, lower alkoxy, amino, (C₁₋₄alkyl)amino, (C₁₋₄alkyl)aminoalkyl, hydroxy(C₁₋₄)alkyl, (loweralkoxy) (C₁₋₄)alkyl, SO₂(C₁₋₄alkyl), -C(=O)H, -C(=O) (C₁₋₄alkyl), pyrrolidinyl, and phenyl (said phenyl in turn being optionally substituted with one to two of lower alkyl, lower alkoxy, cyano, and/or halogen). "Optionally substituted heteroaryl", such as optionally substituted imidazolyl, means a heteroaryl that is optionally substituted with from one to four (preferably one or two) of the above substituents.

"Phenylcarbonyl" means a group -(C=O)-R wherein R is phenyl, which may be optionally substituted as described herein for "aryl".

"Sulfonamidyl" refers to the group -SO₂NRR', wherein R and R' are selected from hydrogen, alkyl, cycloalkyl, and lower substituted alkyl, and also is intended to include "reverse sulfonamides" wherein the group is attached to another moiety via the nitrogen atom, *i.e.,* groups of the formula -NR"SO₂R and/or -N(SO₂R)₂, wherein in this instance R" is hydrogen or lower alkyl, and R is alkyl, cycloalkyl, or lower substituted alkyl.

"Pyrrolidinylalkyl means a group -R-R' wherein R is alkylene as defined herein and R' is pyrrolidinyl, which may be optionally substituted as described herein for heterocyclo.

"Pyridinylalkyl" means a group -R-R' wherein R is alkylene as defined herein and R' is pyridinyl, which may be optionally substituted as described herein for heteroaryl.

"Leaving group" has the meaning conventionally associated with it in synthetic organic chemistry, *i.e.,* an atom or a group capable of being displaced by a nucleophile and includes halo (such as chloro, bromo, and iodo), alkanesulfonyloxy, arenesulfonyloxy, alkylcarbonyloxy (*e.g.,* acetoxy), arylcarbonyloxy, mesyloxy, tosyloxy, trifluoromethanesulfonyloxy, aryloxy (*e.g.,* 2,4-dinitrophenoxy), methoxy, N,O-dimethylhydroxylamino, and the like.

"Optional" or "optionally" means that the subsequently described event may but need not occur, and it includes instances where the event occurs and instances in which it does not. For example, "optionally-substituted cycloalkyl" refers to both cycloallcyl groups and substituted cycloalkyl groups, as defined above. When the term "optionally-substituted" precedes a number of different types of rings in one line or string, *e.g.,* as in "optionally-substituted cycloalkyl or heterocyclo", or "optionally-substituted carbocyclic or heterocyclic ring," or "optionally-substituted aryl, heteroaryl, cycloalkyl, or heterocyclo," it is intended that the term "optionally-substituted" modifies each of the rings identified in the line or string.

When the term "optionally-substituted" is used with respect to a particularly-named cyclic group, such as "optionally-substituted imidazolyl," or "optionally-substituted fused benzo or pyridyl," it should be understood that the optional substituents for such particularly-named rings may be selected from the group of substituents recited above with respect to the genus of which the particularly-named group is a member. Thus, e.g., an "optionally-substituted imidazolyl" maybe an unsubstituted imidazolyl or an imidazolyl group having one, two, or three substituents selected from those recited above for substituted heteroaryl groups. An optionally-substituted fused benzo ring will include an unsubstituted fused benzo ring, and a benzo ring having substituents selected from those recited above for substituted aryl groups.

An optionally-substituted benzyl group means a benzyl group wherein the phenyl portion of the group is unsubstituted or substituted as defined above in the definition for substituted aryl.

When reference is made herein to substituents on the quinazolinone core, e.g., the "five position substituent," the numbering of the ring atoms is intended to be as follows

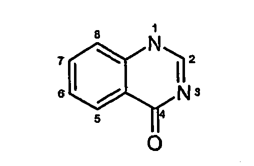

When reference is made herein to a tetrahydroisoquinolyl group, this means a derivative of 1,2,3,4-tetrahydroisoquinole wherein the ring atoms are numbered as follows

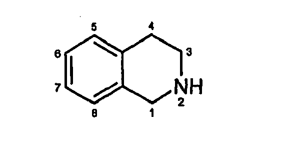

"Pharmaceutically acceptable excipient" means an excipient that is useful in preparing a pharmaceutical composition that is generally safe, non-toxic and neither biologically nor otherwise undesirable. The term includes excipients that are acceptable for veterinary use as well as human pharmaceutical use. A "pharmaceutically acceptable excipient" as used in the specification and claims includes both one and more than one such excipient.

"Pharmaceutically acceptable salt" of a compound means a salt that is generally safe, non-toxic and neither biologically nor otherwise undesirable, and which possesses the desired pharmacological activity of the parent compounds. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, N-methylglucamine, and the like.

The terms "pro-drug" and "prodrug" are used interchangeably herein and refer to any compound which releases an active parent drug according to Formula I *in vivo* when such prodrug is administered to a mammalian subject. Prodrugs of a compound of Formula I are prepared by modifying one or more functional group(s) present in the compound of Formula I in such a way that the modification(s) may be cleaved *in vivo* to release the parent compound. Prodrugs include compounds of Formula I wherein a hydroxy, amino, or sulfhydryl group in a compound of Formula I is bonded to any group that may be cleaved *in vivo* to regenerate the free hydroxyl, amino, or sulfhydryl group, respectively. Examples of prodrugs include, but are not limited to, esters *(e.g.* acetate, dialkylaminoacetates, formates, phosphates, sulfates and benzoate derivatives) and carbamates of hydroxy functional groups (*e.g. N,N*-dimethylcarbonyl), esters of carboxyl functional groups *(e.g.* ethyl esters, morpholinoethanol esters), *N*-acyl derivatives (*e.g.* N-acetyl), *N*-Mannich bases, Schiff bases and enaminones of amino functional groups, oximes, acetals, ketals, and enol esters of ketones and aldehyde functional groups in compounds of Formula I, and the like.

The prodrug can be metabolized before absorption, during absorption, after absorption, or at a specific site. Although metabolism occurs for many compounds primarily in the liver, almost all other tissues and organs, especially the lung, are able to carry out varying degrees of metabolism. Prodrug forms of compounds may be utilized, e.g., to improve bioavailability, improve subject acceptability such as by masking or reducing unpleasant characteristics such as bitter taste or gastrointestinal irritability, alter solubility such as for intravenous use, provide for prolonged or sustained release or delivery, improve ease of formulation, or provide site-specific delivery of the compound. Reference to a compound herein includes prodrug forms of a compound. Prodrugs are described in The Organic Chemistry of Drug Design and Drug Action, by Silverman, Academic Press, San Diego (1992), Chapter 8: "Prodrugs and Drug delivery Systems" pp.352-401; Design of Prodrugs, edited by Bundgaard, Elsevier Science, Amsterdam (1985); Design of Biopharmaceutical Properties through Prodrugs and Analogs, Ed. by Roche, American Pharmaceutical Association, Washington (1977); and Drug Delivery Systems, ed. by Juliano, Oxford Univ. Press, Oxford (1980).

"Solvate" means solvent addition form that contains either stoichiometric or non-stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate.

"Protecting group" refers to an atom or group of atoms that is attached to a reactive group in a molecule and masks, reduces, or prevents the reactivity of the group to which it is attached. Examples of protecting groups can be found in Green and Wuts, Protective Groups in Organic Chemistry (Wiley, 2nd ed. 1991), and Harrison et al., Compendium of Synthetic Organic Methods, Vols. 1-8 (John Wiley and Sons, 1971-1996). Representative amino protecting groups include formyl, acetyl, trifluoroacetyl, benzyl, benzyloxycarbonyl (CBZ), tert-butoxycarbonyl (Boc), trimethyl silyl (TMS), 2-trimethylsilyl-ethanesulfonyl (SES), trityl and substituted trityl groups, allyloxycarbonyl, 9-fluorenylmethyloxycarbonyl (FMOC), nitro-veratryloxycarbonyl (NVOC), and the like. Representative hydroxy protecting groups include those where the hydroxy group is either acylated or alkylated such as with benzyl or lower alkyl and trityl ethers as well as alkyl ethers, tetrahydropyranyl ethers, trialkylsilyl ethers, and allyl ethers.

Compounds that have the same molecular formula but differ in the nature or sequence of bonding of their atoms or the arrangement of their atoms in space are termed "isomers." Isomers that differ in the arrangement of their atoms in space are termed "stereoisomers". Stereoisomers that are not mirror images of one another are termed "diastereomers," and those that are non-superimposable mirror images of each other are termed "enantiomers". When a compound has an asymmetric center, e.g., if a carbon atom is bonded to four different groups, a pair of enantiomers is possible. An enantiomer can be characterized by the absolute configuration of its asymmetric centers which are described by the (R) and (S) sequencing rules of Cahn and Prelog, or by the manner in which the molecule rotates the plane of polarized light and designated as dextrorotatory or levorotatory (*i.e.,* as (+) or (-)-isomers respectively). A chiral compound can exist as either an individual enantiomer or as a mixture thereof. A mixture containing different enantiomers is called a "racemic mixture".

The compounds of this invention may possess one or more asymmetric centers; such compounds can therefore be produced as individual stereoisomers or as mixtures thereof. Unless indicated otherwise, the description or naming of a particular compound in the specification and claims is intended to include both individual enantiomers and mixtures (racemic or otherwise) thereof. The methods for the determination of stereochemistry and the separation of stereoisomers are well-known in the art (*see* March, Advanced Organic Chemistry, Chap. 4, 4th edition, John Wiley and Sons, New York [1992]).

"Tautomers" refers to compounds whose structures differ markedly in arrangement of atoms, but which exist in easy and rapid equilibrium. It is to be understood that compounds of Formula I may be depicted as different tautomers. For example, compounds of Formula I wherein Z is -C(O)-, may be depicted in the following tautomer forms

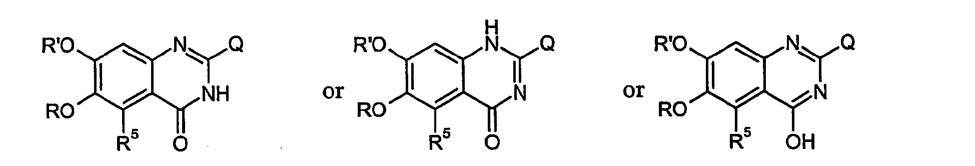

Compounds of Formula I may also contain other groups that exist in tautomeric equilibrium. For example some of the compounds contain an imidazolin-2-yl amino group which may be in equilibrium with a imidazolin-2-ylidenamino group

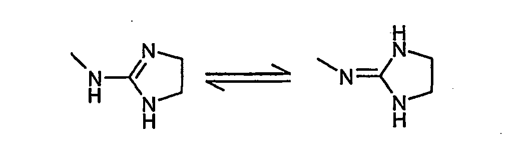

It should also be understood that when compounds have tautomeric forms, all tautomeric forms are intended to be within the scope of the invention, and the naming of the compounds does not exclude any tautomer form.

"Treating" or "treatment" of a disease includes: (1) preventing the disease, *i.e.,* causing the clinical symptoms of the disease not to develop in a mammal that may be exposed to or predisposed to the disease but does not yet experience or display symptoms of the disease; (2) inhibiting the progression of the disease, *i.e.,* arresting or reducing the development of the disease or its symptoms; and (3) relieving the disease, *i.e.,* causing regression of the disease or its symptoms.

"A therapeutically effective amount" means the amount of a compound that, when administered to a mammal for treating a disease, is sufficient to effect a treatment for the disease. The "therapeutically effective amount" will vary depending on such factors as the compound being administered, the type of disease being treated, the progression or severity of the disease state, and the age, weight, and general health of the mammal being treated.

"Patient" means mammals and non-mammals. Mammals means any member of the mammalia class including, but not limited to, humans, non-human primates such as chimpanzees and other apes and monkey species; farm animals such as cattle, horses, sheep, goats, and swine; domestic animals such as rabbits, dogs, and cats; and laboratory animals such as rats, mice, and guinea pigs. Examples of non-mammals include, but are not limited to, birds, reptiles, and the like.

"Pharmacological effect" as used herein encompasses effects produced in the patient that achieve the intended purpose of a therapy. In one preferred embodiment, a pharmacological effect means that primary indications of the patient being treated are prevented, alleviated, or reduced. For example, a pharmacological effect would be one that results in the prevention, alleviation or reduction of primary indications in a treated patient. In another preferred embodiment, a pharmacological effect means that disorders or symptoms of the primary indications of the patient being treated are prevented, alleviated, or reduced.

"Disease state" means any disease, condition, symptom, or indication.

"Disorders of the urinary tract" or "uropathy" refer to pathologic changes in the urinary tract and symptoms thereof. Disorders of the urinary tract include overactive bladder (also known as detrusor hyperactivity), outlet obstruction, outlet insufficiency, pelvic hypersensitivity. incontinence, benign prostatic hypertrophy or hyperplasia (BPH), prostatitis, detrusor hyperreflexia, urinary frequency, nocturia, urinary urgency, pelvic hypersensitivity, urge incontinence, urethritis, prostatodynia, cystitis, and idiophatic bladder hypersensitivity.

"Overactive bladder" or "Detrusor hyperactivity" includes, but is not limited to, changes symptomatically manifested as urgency, frequency, reduced bladder capacity, and incontinence episodes; changes urodynamically manifested as changes in bladder capacity, micturition threshold, unstable bladder contractions, and sphincteric spasticity; and symptoms usually manifested in detrusor hyperreflexia (neurogenic bladder), in conditions such as outlet obstruction, outlet insufficency, pelvic hypersensitivity, or in idiopathic conditions such as detrusor instability.

"Outlet obstruction" includes, but is not limited to, benign prostatic hypertrophy or benign prostatic hyperplasia (BPH), urethral stricture disease, tumors and the like. It is usually symptomatically manifested as obstructive (low flow rates, difficulty in initiating urination, and the like), or irritative (urgency, suprapubic pain, and the like).

"Outlet insufficiency" includes, but is not limited to, urethral hypermobility, intrinsic sphincteric deficiency, or mixed incontinence. It is usually symptomatically manifested as stress incontinence.

"Pelvic Hypersensitivity" includes but is not limited to, pelvic pain, interstitial (cell) cystitis, prostadynia, prostatitis, vulvadynia, urethritis, orchidalgia, and the like. It is symptomatically manifested as pain, inflammation or discomfort referred to the pelvic region, and usually includes symptoms of overactive bladder.

"Sexual dysfunction" means the inability to achieve a normal sexual response and includes such conditions in males and females. Thus, it includes male erectile dysfunction (MED) and female sexual dysfunction (FSD).

"Disease states associated with the Central Nervous System (CNS) " or "CNS disease states" mean neurological and/or psychiatric changes in the CNS, e.g., brain and spinal cord, which manifest in a variety of symptoms. Examples of CNS disease states include, but are not limited to, migraine headache; cerebrovascular deficiency; psychoses including paranoia, schizophrenia, attention deficiency, and autism; obsessive/compulsive disorders including anorexia and bulimia; posttraumatic stress disorders, sleep disorders, convulsive disorders including epilepsy and withdrawal from addictive substances; cognitive diseases including Parkinson's disease and dementia; and anxiety/depression disorders such as anticipatory anxiety (e.g., prior to surgery, dental work and the like), depression, mania, seasonal affective disorder (SAD), and convulsions and anxiety caused by withdrawal from addictive substances such as opiates, benzodiazepines, nicotine, alcohol, cocaine, and other substances of abuse; and improper thermoregulation.

The compounds of this invention demonstrate selectivity for the alpha-1A/B subtype over the alpha-1D subtype. The compounds of this invention may reduce both obstructive and irritative symptoms in patients with BPH. The attenuated antagonism of alpha 1D-adrenoceptor is expected to lead to reduced or fewer side effects than those associated with the use of non-subtype-selective agents.

According to one aspect of the invention, preferred compounds are those having Formula (I) wherein R⁵ is selected from C₁₋₄alkyl, halogen, hydroxy, C₁₋₄alkoxy, - O(CH₂)ᵣNH₂, -O(CH₂)ᵣOH, -O(CH₂)ᵣO(C₁₋₄alkyl), -O(CH₂)ᵣO(phenyl), - O(CH₂)ᵣO(benzyl), -O(CH₂)ₛcycloalkyl, -O(CH₂)ₛ(phenyl), -(CH₂)ₛcycloalkyl, and - (CH₂)ₛ(phenyl), wherein each of said phenyl, benzyl, and cycloalkyl rings is optionally substituted with one to two of lower alkyl, substituted lower alkyl, cyano, and/or halogen; *r* is 1 or 2; and s is 0,1 or 2. More preferably R⁵ is selected from methyl, ethyl, n-propyl, isopropyl, halogen, methoxy, and ethoxy. Even more preferred are compounds wherein R⁵ is selected from fluoro, chloro, methyl, ethyl, isopropyl, methoxy, and ethoxy. Most preferred are compounds wherein R⁵ is methoxy or methyl.

According to another aspect of the invention, preferred compounds are those having the Formula (I), wherein *m* is 0.

According to another aspect of the invention, preferred compounds are those having the Formula (I), wherein R and R' are both CH₃.

In one embodiment the present invention provides a compound of the Formula I wherein Z is -C(=O); R and R' are CH₃; R⁵ is selected from C₁₋₄alkyl, halogen, cyano, hydroxy, C₁₋₄alkoxy, -O(CH₂)ᵣNH₂, -O(CH₂)ᵣOH, -O(CH₂)ᵣO(C₁₋₄alkyl), - O(CH₂)ᵣO(benzyl), -O(CH₂)ₛcycloalkyl,-O(CH₂)ₛ(phenyl) and -(CH₂)ₛ(phenyl), wherein each of said phenyl is optionally substituted with one to two of lower alkyl, cyano, trifluoromethyl, and/or halogen; R⁷ is attached to any available carbon atom of the piperidinyl or piperazinyl ring and at each occurrence is independently selected from alkyl, halogen, cyano, hydroxy, alkoxy, haloalkoxy, amino, and alkylamino; K and L are independently selected from C₁₋₄alkylene, -M₁-O-M₂-, -M₁-C(=O)-M₂-, -M₁-C(O)₂-M₂-, -M₁-C(=O)NR¹⁶-M₂-, -M₁-S(O)₂-M₂-, and -M₁-S(O)₂NR¹⁶-M₂-, wherein M₁ and M₂ are selected from a bond and C₁₋₄alkylene, except when K is -M₁-O-M₂-, then M₁ is not a bond; R¹⁴ and R¹⁵ are independently selected from hydrogen, alkyl, aryl, heteroaryl, cycloalkyl, or heterocyclo, provided, however, that if K is -M₁-S(O)₂-, then R¹⁴ is not hydrogen, wherein each R¹⁴ and R¹⁵ group in turn is optionally substituted with one to three groups selected from R¹⁹ and R²⁰; R¹⁶ is hydrogen or C₁₋₄alkyl; R¹⁹ and R²⁰ are independently selected from lower alkyl, halogen, cyano, halo(C_{1- 4})alkyl, halo(C₁₋₄)alkoxy, hydroxy, lower alkoxy, amino, (C₁₋₄alkyl)amino, (C₁₋₄alkyl)amino(C₁₋₄)alkyl, hydroxy(C₁₋₄)alkyl, (loweralkoxy)(C₁₋₄)alkyl, SO₂(C₁₋₄alkyl),-C(=O)H, -C(=O)(C₁₋₄alkyl), sulfonamidyl, CO₂H, CO₂(C₁₋₄alkyl), guanidinyl, alkyl-guanidinyl, pyrrolidinyl, and phenyl (said phenyl in turn being optionally substituted with one to two of lower alkyl, lower alkoxy, cyano, and/or halogen); *m* is 0,1, or 2; *n, p* and *q* are each 1; *r* is 2, 3 or 4; and *s* is 0, 1 or 2.

In still another embodiment the present invention provides a compound of the formula I wherein R⁵ is selected from methyl, ethyl, n-propyl, isopropyl, halogen, cyano, methoxy, ethoxy, hydroxy, 2-methoxyethoxy, 2-hydroxyethoxy, 2-aminoethyoxy, cyclopropylmethoxy, phenoxy, 2-benzyloxyethoxy, and 4-fluorophenyl.

In still another embodiment the present invention provides a compound of the formula I wherein Z is -C(=O)-. In yet another embodiment the present invention provides a compound of the formula I wherein Z is -C(=O)- and *m* is 0. In yet another embodiment the present invention provides a compound of the formula I wherein Z is - C(=O)-, *m* is 0 and *n, p* and *q* are each 1.

In another embodiment the present invention provides a compound of formula I wherein Q is a group 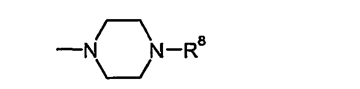 wherein R⁸ is
(i) K-R¹⁴;
   K is selected from a bond, -C(=O)-, -C(=O)C₁₋₂alkylene-, -C(O)₂-, -C₁₋₂alkylene-C(O)₂-, -C(=O)NR¹⁶-, and -C₁₋₂akylene-C(=O)NR¹⁶-, wherein R¹⁶ is hydrogen or methyl; and R¹⁴ is selected from lower alkyl, furyl, cyclopentyl, phenyl, pyridyl, imidazolyl, and imidazolinyl, wherein each R¹⁴ in turn is optionally substituted with one to three groups selected from R¹⁹; and R¹⁹ is selected from lower alkyl, lower alkoxy, halogen, cyano, trifluoromethyl, and phenyl (said phenyl in turn being optionally substituted with one to two of methyl, halogen, and/or cyano);
(ii) selected from -C(=O)furyl, -C(=O)alkyl, -C(O)₂alkyl, -C₁₋₂alkylene-C(O)₂-(alkyl), -C₁₋₂alkylene-C(=O)NH(alkyl), -C₁₋₂alkylene-C(=O)N(lower alkyl)(alkyl), -C(=O)NH (phenyl), phenyl, -C(=O)C₁₋₂alkylene(cyclopentyl), pyridyl, imidazolyl, and imidazolinyl, wherein each of said phenyl, pyridyl, imidazolyl, and imidazolinyl groups is in turn optionally substituted with one to three of lower alkyl, trifluoromethyl, halogen, cyano, methoxy, and phenyl, said phenyl in turn optionally substituted with one to two of methyl, halogen, and/or cyano;
(iii) selected from alkylcarbonyl, furanylcarbonyl, alkoxycarbonyl, alkoxycarbonylalkyl, dialkylaminocarbonyl, optionally substituted:phenylaminocarbonyl, cycloalkylalkylcarbonyl, optionally substituted phenyl, optionally substituted pyridinyl, optionally substituted imidazolyl, and optionally substituted imidazolinyl; or
(iv) selected from 4-methyl-pentanoyl-, furan-2-carbonyl-, ethoxycarbonyl-, ethoxycarbonylmethyl-, 3-trifluoromethylphenyl-, dimethylaminocarbonylmethyl-, 3-chlorophenyl-, cyclopentyl-methylcarbonyl-, 3-fluorophenyl-aminocarbonyl-, 3,4-difluorophenyl-aminocarbonyl-, 3-cyanophenyl-aminocarbonyl-, pyridin-2-yl-, imidazol-2-yl-, 6-methyl-pyridin-2-yl-, 4-methyl-pyridin-2-yl, 1-oxypiridin-2-yl, 1-methyl-imidazolin-2-yl-, 1-methyl-imidazol-2-yl-, 1,4,5-trimethyl-imidazol-2-yl-, 4-methoxy-pyridin-2-yl-, 3-methylbutyryl-, 3-trifluoromethyl-4-chlorophenyl-, 1-(3-fluorophenyl)-imidazolin-2-yl-, and 1-isopropyl-imidazolin-2-yl-.

In another embodiment the present invention provides a compound of formula I
wherein Q is 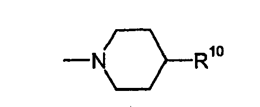 wherein R¹⁰ is
(i) selected from alkyl, pyrrolidinyl, -C₁₋₂alkylene(pyrrolidinyl), -C(=O)phenyl, -C₁₋₂alkylene(phenyl), -C₁₋₂alkylene(pyridyl), -N(H)(C₁₋₂alkylene)(phenyl), -N(CH₃)(C₁₋₂alkylene)(phenyl), -C₁₋₂alkylene(imidazolyl), -O-(C₁₋₂alkylene)(phenyl), and tetrahydropyrimidinyl, wherein each of said pyrrolidinyl and tetrahydropyrimidinyl groups optionally has one to two carbon ring atoms replaced with a carbonyl group, and each of said pyrrolidinyl, phenyl, pyridyl, imidazolyl, and tetrahydropyrimidinyl groups optionally is substituted with one to three of lower alkyl, halogen, cyano, methoxy, methoxy(C₁₋₂)alkyl, amino, (C₁₋₄alkyl)amino, sulfonamidyl, CO₂H, CO₂(C₁₋₄alkyl),-N=C(CH₃)N(CH₃)₂, and/or pyrrolidinyl;
(ii) selected from alkyl, optionally substituted pyrrlolidinyl, optionally substituted pyrrolidinylalkyl, optionally substituted tetrahydropyrimidinyl, optionally substituted benzyl, optionally substituted phenylcarbonyl, optionally substituted benzylamino, optionally substituted imidazolylalkyl, optionally substituted imidazolinylalkyl, optionally substituted pyridinylakyl, optionally substituted phenylalkylamino, optionally substituted benzyloxy, optionally substituted phenyl, and optionally substituted morpholinylcarbonyl; or
(iii) selected from 2-(2-methoxymethylpyrrolidin-1-yl)-ethyl-, 2-oxo-tetrahydropyrimidin-1-yl-, 2-oxo-pyrrolidin-1-yl-, benzyl, 4-chlorophenylcarbonyl, 3-(pyrrolidin-1-yl)-benzylamino-, 2-(2-amino-imidazol-1-yl)-ethyl-, 2-(2-methyl-imidazolin-1-yl)-ethyl-, 2-(imidazol-1-yl)-ethyl-, 2-dimethylaminosulfonyl-benzyloxy-, 2-fluoro-benzyl-N-methylamino-, propyl-, pyridin-2-yl-methyl-, 2-fluoro-benzylamino-, 2-chloro-benzylamino-, 2-methoxy-benzylamino-, 2-methyl-benzylamino-, 3-methoxycarbonyl-benzyloxy-, 3-carboxy-benzyloxy-, 3-(N,N-di-methanesulfonyl)-amino-benzyloxy-, 3-methanesulfonylamino-benzyloxy-, 3-N,N-dimethylacetamidinyl-benzyloxy-, 3-(2-methylimidazolin-1-yl)-phenyl-, morpholin-1-yl-carbonyl-, 3-[(2-methylaminocarbonyl)-ethyl]-phenyl-, and 3-N,N-dimethylacetamidinyl-phenyl-,

In another embodiment the present invention provides a compound of formula I
wherein Q is the group 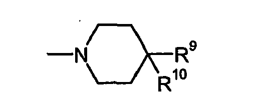 wherein R⁹ and R¹⁰ are taken together
(i) to form a spirocyclic ring selected from one of 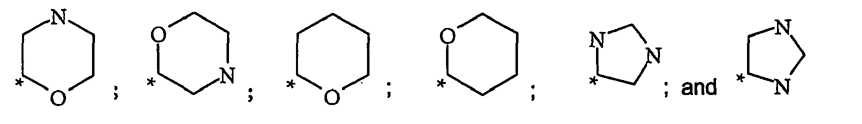 wherein * represents the point of attachment to the carbon ring atom to which R⁹ and R¹⁰ are attached, and wherein each of said spirocyclic rings optionally has one to two carbon ring atoms replaced with a carbonyl group, and/or optionally has a benzo ring fused thereto, and wherein each of said spirocyclic rings and/or benzo rings is optionally substituted with one to three groups selected from lower alkyl, aminoalkyl, alkylaminoalkyl, and phenyl, wherein said phenyl group is in turn optionally substituted with one to two of halogen, cyano, lower alkoxy, and/or lower alkyl; or
(ii) to define one of 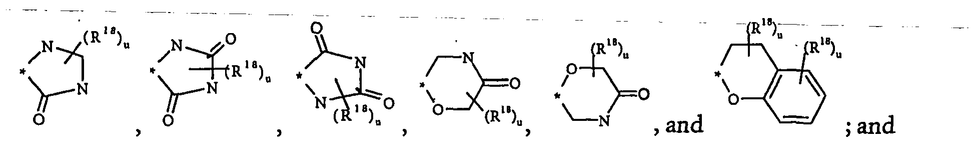 R¹⁸ is at each occurrence selected from hydrogen, lower alkyl, (C₁₋₄alkyl)amino(C₁₋₄)alkyl, and phenyl optionally substituted with one to two of halogen and/or lower alkoxy; and *u* is 0,1, 2 or 3.

In another embodiment the present invention provides a compound of formula I
wherein Q is the group 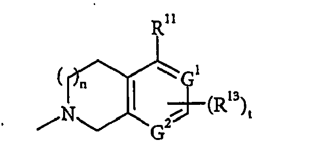 wherein G¹ and G² are carbon or nitrogen; R¹¹ is -J-R¹⁷ ; R¹³ is selected from lower alkoxy, guanidinyl and alkylguanidinyl; J is selected from a bond, -C₁₋₂alkylene-, -C(=NH)-, -NR¹⁶⁻C(=NH)-, -N=CR¹⁶-NR^{16a}-, and -N=; R¹⁶ and R^{16a} are hydrogen or lower alkyl; R¹⁷ is selected from hydrogen, alkyl, pyrrolidinyl, pyrrolinyl, imidazolyl, imidazolinyl, imidazolidinyl, morpholinyl, cycloalkyl, and furyl, except R¹⁷ is not furyl when J is -N=; and wherein each of said R¹⁷ groups is optionally substituted with one to three groups selected from R²¹; R²¹ is selecetd from lower alkyl, lower alkoxy, halogen and cyano; and t is 0,1 or 2.

In another embodiment the present invention provides a compound of formula I
wherein Q is 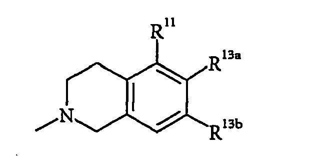 wherein R¹¹ is selected from hydrogen, pyrrolidinyl, pyrrolinyl, imidazolyl, imidazolinyl, imidazolidinyl, morpholinyl, -N=C(CH₃)N(CH₃)₂, -N=(pyrrolidinyl), -N=(imidazolidinyl), -C₁₋₂alkylene(imidazolinyl), -C(=NH)(morpholinyl), -N(H)-C(=NH)-(alkyl), -N(H)-C(=NH)-(cyclobutyl), and -N(H)-C(=NH)-(furyl), wherein each of said pyrrolidinyl, pyrrolinyl, imidazolyl, imidazolinyl, imidazolidinyl, and morpholinyl groups is in turn optionally substituted with one to three of methyl, ethyl, propyl, methoxy, ethoxy, and/or methoxy(C₁₋₂alkyl); and R^{13a} and R^{13b} are selected from hydrogen, methoxy and -N=C(CH₃)N(CH₃)₂.

In another embodiment the present invention provides a compound of formula I
wherein Q is the group 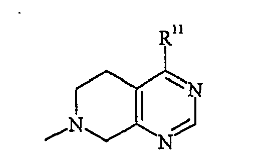 wherein R¹¹ is selected from hydrogen, pyrrolidinyl, pyrrolinyl, imidazolyl, imidazolinyl, imidazolidinyl, morpholinyl, -N=C(CH₃)N(CH₃)₂, -N=(pyrrolidinyl), -N=(imidazolidinyl), -C₁₋₂alkylene-(imidazolinyl), -C(=NH)-(morpholinyl), -N(H)-C(=NH)-(alkyl), -N(H)-C(=NH)-(cyclobutyl), and -N(H)-C(=NH)-(furyl), wherein each of said pyrrolidinyl, pyrrolinyl, imidazolyl, imidazolinyl, imidazolidinyl, morpholinyl, cyclobutyl, and/or furyl groups is in turn optionally substituted with one to three of methyl, ethyl, propyl, methoxy, ethoxy, and/or methoxy(C₁₋₂alkyl).

In another embodiment the present invention provides a compound of formula I
wherein Q is the group 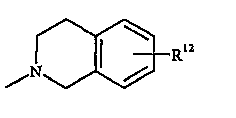 wherein R¹² is selected from
(i) hydrogen, alkoxy, dialkylaminoalkyl, alkylaminoalkyl, optionally substituted imidazolidin-2-ylideneamino, alkylaminoalkylcarbonylaminoalkyl, optionally substituted pyrrolidinylidineamino, acetamidinyl, optionally substituted imidazolylalkyl, optionally substituted imidazolyl, optionally substituted imidazolinyl, iminomorpholinylmethyl, amidinyl, and morpholinyl; or
(ii) hydrogen, methoxy, N-ethyl-N-methylamino-methyl-, N-methylamino-methyl-, 1,3-dimethyl-imidazolidin-2-ylidineamino-, N-methylamino-methylcarbonyl-N-methylamino-methyl-, 1-methyl-pyrrolidin-ylidineamino-, N,N-dimethylacetamidinyl-, 2-(imidazolin-2-yl)-ethyl-, imidazolin-2-yl-, imino-morpholin-4-yl-methyl-, butyramidinyl-, cyclobutanecarboxamidinyl-, furan-2-yl-carboxamidinyl-, 1-methylimidazolin-2-yl-, 2,4,4-trimethyl-imidazolin-1-yl-, 1-methyl-imidazol-2-yl-, 1-(2-methoxy)-ethoxy-imidazolin-2-yl-, 1-isopropyl-imidazolin-2-yl-, 2,4-dimethyl-imidazolin-2-yl-, and morpholin-4-yl-.

According to another aspect of the invention, preferred compounds are those having the Formula (Ia) 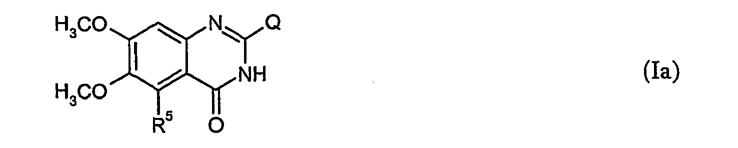 wherein
- R⁵: is selected from C₁₋₄alkyl, halogen, cyano, hydroxy, C₁₋₄alkoxy, -O(CH₂)ᵣNH₂, -O(CH₂)ᵣOH, -O(CH₂)ᵣO(C₁₋₄alkyl), -O(CH₂)ᵣO(benzyl), -O(CH₂)ₛcycloalkyl, -O(CH₂)ₛ(phenyl), and -(CH₂)ₛ(phenyl), wherein each of said phenyl is optionally substituted with one to two of lower alkyl, cyano, trifluoromethyl, and/or halogen;
and
- Q: is selected from (S¹), (T¹), (U¹) and (V¹) 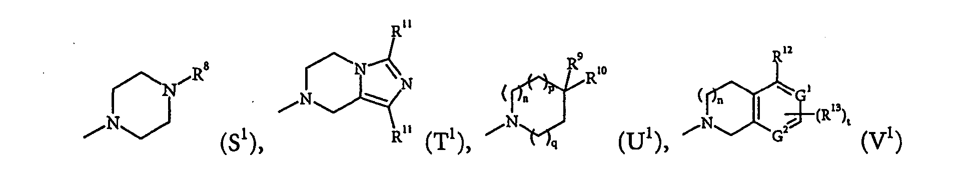
R⁸ is -K-R¹⁴;
R⁹ is hydrogen or alkyl;
R¹⁰ is-L-R¹⁵, or R¹⁰ together with R⁹ may form an optionally substituted spiroryclic ring of five or six members that optionally includes one or two heteroatoms selected from O, N and S;
each R¹¹ is independently selected from hydrogen, alkyl or cycloalkyl;
R¹² is selected from hydrogen, halogen, cyano, alkoxy and -J-R¹⁷;
G¹ and G² are selected from CR¹³ and nitrogen;
R¹³ is selected from hydrogen, halogen, cyano, and alkoxy;
J is selected from a bond, -C₁₋₄alkylene-, -C(=NR¹⁶)-, -NR¹⁶-C(=NR¹⁶)-, -N=CR¹⁶-NR^{16a}-, and -N=;
K is selected from a bond, C₁₋₄alkylene, -M₁-C(=O)-M₂-, -M₁-C(O)₂-M₂- and -M₁-C(=O)NR¹⁶-M₂-, wherein M₁ and M₂ are selected from a bond and C₁₋₄alkylene;
L is selected from a bond, C₁₋₄alkylene, -M₁-O-M₂-, and -M₁-NR¹⁶-M₂-,
R¹³ is selected from hydrogen, halo, alkyl, haloalkyl and alkoxy;
R¹⁴ is selected from alkyl, cycloalkyl, optionally substituted phenyl, optionally substituted pyridinyl, and optionally substituted imidazolyl;
R¹⁵ is selected from alkyl, optionally substituted phenyl, optionally substituted pyrrolidinyl, optionally substituted imidazolyl, optionally substituted pyridinyl, and tetrahydropyrimidyl;
R¹⁷ is selected from hydrogen, alkyl, amino, alkylamino, dialkylamino, cycloalkyl, optionally substituted furanyl, optionally substituted imidazolinyl, morpholinyl, and optionally substituted imidazolidinyl;
R¹⁶ and R^{16a} are selected from hydrogen and lower alkyl;
n is 1;
p is 0,1 or 2;
q is 1; and
*t* is 0, 1 or 2, except if both G¹ and G² are nitrogen, then *t* is 0 or 1.

In certain embodiments of formula (I) and formula (Ia):
K-R¹⁴ taken together may be selected from alkylcarbonyl, furanylcarbonyl, alkoxycarbonyl, alkoxycarbonylalkyl, dialkylaminocarbonyl, optionally substituted phenylaminocarbonyl, cycloalkylalkylcarbonyl, optionally substituted phenyl, optionally substituted pyridinyl, optionally substituted imidazolyl, and optionally substituted imidazolinyl;
L-R¹⁵ taken together may beselected from alkyl, optionally substituted pyrrlolidinyl, optionally substituted pyrrolidinylalkyl, optionally substituted tetrahydropyrimidinyl, optionally substituted benzyl, optionally substituted phenylcarbonyl, optionally substituted benzylamino, optionally substituted imidazolylalkyl, optionally substituted imidazolinylalkyl, optionally substituted pyridinylalkyl, optionally substituted phenylalkylamino, optionally substituted benzyloxy, optionally substituted phenyl, and optionally substituted morpholinylcarbonyl; and
J-R¹⁷ taken together may be selected from hydrogen, alkoxy, dialkylaminoalkyl, alkylaminoalkyl, optionally substituted imidazolidin-2-ylideneamino, alkylaminoalkylcarbonylaminoalkyl, optionally substituted pyrrolidinylidineamino, acetamidinyl, optionally substituted imidazolylalkyl, optionally substituted imidazolyl, optionally substituted imidazolinyl, iminomorphofinylmethyl, amidinyl, and morpholinyl.

Where K-R¹⁴ taken together is alkylcarbonyl K-R¹⁴ may be 4-methyl-pentanoyl-.

Where K-R¹⁴ taken together is furanylcarbonyl K-R¹⁴ may be furan-2-carbonyl-. Where K-R¹⁴ taken together is alkoxycarbonyl, K-R¹⁴ may be ethoxycarbonyl.

Where K-R¹⁴ taken together is dialkylaminocarbonyl, K-R¹⁴ may be dimethylaminocarbonylmethyl-.

Where K-R¹⁴ taken together is optionally substituted phenylaminocarbonyl, K-R¹⁴ may be 3-fluorophenylaminocarbonyl-, 3,4-difluorophenylaminocarbonyl-, or 3-cyanophenylaminocarbonyl-.

Where K-R¹⁴ taken together is cycloalkylalkylcarbonyl, K-R¹⁴ may be cyclopentyl-methylcarbonyl-.

Where K-R¹⁴ taken together is optionally substituted phenyl, K-R¹⁴ may be 3-trifluoromethylphenyl-, 3-chlorophenyl-, 3-trifluoromethyl-4-chlorophenyl-, or 1-(3-fluorophenyl)-imidazolin-2-yl-.

Where K-R¹⁴ taken together is optionally substituted pyridinyl, K-R¹⁴ maybe 4-methoxy-pyridin-2-yl-, 4-methyl-pyridin-2-yl-, 5-methyl-pyridin-2-yl-, 6-methyl-pyridin-2-yl-, pyridin-2-yl, or 1-oxypiridin-2-yl.

Where K-R¹⁴ taken together is optionally substituted imidazolyl, K-R¹⁴ maybe imidazol-2-yl-, 1-methyl-imidazol-2-yl-, or 1,4,5-trimethyl-imidazol-2-yl-.

Where K-R¹⁴ taken together is optionally substituted imidazolinyl, K-R¹⁴ may be 1-methyl-imidazolin-2-yl-, imidazolin-2-yl-, or 1-isopropyl-imidazolin-2-yl-.

Where L-R¹⁵ taken together is optionally substituted pyrrlolidinyl, L-R¹⁵ may be 2-oxopyrrolidin-1-yl-.

Where L-R¹⁵ taken together is optionally substituted pyrrolidinylalkyl, L-R¹⁵ may be 2-(2-methoxymethylpyrrolidin-1-yl)-ethyl-.

Where L-R¹⁵ taken together is optionally substituted tetrahydropyrimidinyl, L-R¹⁵ taken may be 2-oxo-tetrahydropyrimidin-1-yl-.

Where L-R¹⁵ taken together is optionally substituted phenylcarbonyl, L-R¹⁵ may be 4-chlorophenylcarbonyl.

Where L-R¹⁵ taken together is optionally substituted benzylamino, L-R¹⁵ may be 3-(pyrrolidin-1-yl)-benzylamino-, 2-fluoro-benzyl-N-methylamino-, 2-fluoro-benzylamino-, 2-chloro-benzylamino-, 2-methoxy-benzylamino- or 2-methyl-benzylamino-.

Where L-R¹⁵ taken together is optionally substituted imidazolylalkyl, L-R¹⁵ may be 2-(2-amino-imidazol-1-yl)-ethyl-, or 2-(imidazol-1-yl)-ethyl-.

Where L-R¹⁵ taken together is optionally substituted imidazolinylalkyl, L-R¹⁵ may be 2-(2-methyl-imidazolin-1-yl)-ethyl-.

Where L-R¹⁵ taken together is optionally substituted pyridinylalkyll, L-R¹⁵ may be pyridin-2-yl-methyl-.

Where L-R¹⁵ taken together is optionally substituted benzyloxy, L-R¹⁵ maybe 2-dimethylaminosulfonyl-benzyloxy-, 2-dimethylaminosulfonyl-benzyloxy, 3-methoxycarbonyl-benzyloxy-, 3-carboxy-benzyloxy-, 3-(N,N-di-methanesulfonyl)-amino-benzyloxy-, 3-methanesulfonylamino-benzyloxy-, or 3-N,N-dimethylacetamidinyl-benzyloxy-.

Where L-R¹⁵ taken together is optionally substituted phenyl, L-R¹⁵ maybe 3-(2-methyl-imidazolin-1-yl)-phenyl-, 3-[(2-methylaminocarbonyl)-ethyl]-phenyl-, or 3-N,N-di-methylacetamidinyl-phenyl-.

Where L-R¹⁵ taken together is optionally substituted morpholinylcarbonyl, L-R¹⁵ may be morpholin-1-yl-carbonyl-.

Where J-R¹⁷ taken together is dialkylaminoalkyl, J-R¹⁷ may be N-ethyl-N-methylamino-methyl-.

Where J-R¹⁷ taken together is alkylaminoalkyl, J-R¹⁷ may be N-methylamino-methyl-,

Where J-R¹⁷ taken together is optionally substituted imidazolidin-2-ylideneamino, J-R¹⁷ may be 1,3-dimethyl-imidazolidin-2-ylidineamino-.

Where J-R¹⁷ taken together is alkylaminoalkylcarbonylaminoalky, J-R¹⁷ may be N-methylamino-methylcarbonyl-N-methylamino-methyl-.

Where J-R¹⁷ taken together is optionally substituted pyrrolidinylidineamino, J-R¹⁷ may be 1-methyl-pyrrolidin-ylidineamino-.

Where J-R¹⁷ taken together is optionally substituted imidazolylalkyl, J-R¹⁷ may be 2-(imidazolin-*2*-yl)-ethyl-.

Where J-R¹⁷ taken together is optionally substituted imidazolyl, J-R¹⁷ maybe 1-methyl-imidazol-2-yl-.

Where J-R¹⁷ taken together is optionally substituted imidazolinyl, J-R¹⁷ maybe imidazolin-2-yl-, 1-methyl-imidazolin-2-yl-, 2,4,4-trimethyl-imidazolin-1-yl-, 1-(2-methoxy)-ethoxy-imidazolin-2-yl-, 1-isopropyl-imidazolin-2-yl-, or 2,4-dimethyl-imidazolin-2-yl-.

Where J-R¹⁷ taken together is iminomorpholinylmethyl, J-R¹⁷ may be imino-morpholin-4-yl-methyl-.

Where J-R¹⁷ taken together is amidinyl, J-R¹⁷ may bebutyramidinyl-, cyclobutanecarboxamidinyl-, furan-2-yl-carboxamidinyl-, or N,N-dimethylacetamidinyl-.

Where J-R¹⁷ taken together is morpholinyl, J-R¹⁷ may be morpholin-4-yl-

In certain embodiments of formula (I) and formula (Ia), K-R¹⁴ taken together may be selected from 4-methyl-pentanoyl-, furan-2-carbonyl-, ethoxycarbonyl-, ethoxycarbonylmethyl-, 3-trifluoromethylphenyl-, dimethylaminocarbonylmethyl-, 3-chlorophenyl-, cyclopentyl-methylcarbonyl-, 3-fluorophenyl-aminocarbonyl-, 3,4-difluorophenyl-aminocarbonyl-, 3-cyanophenyl-aminocarbonyl-, pyridin-2-yl-, imidazol-2-yl-, 6-methyl-pyridin-2-yl-,4-methyl-pyridin-2-yl,1-oxypiridin-2-yl,1-methyl-imidazolin-2-yl-, 1-methyl-imidazol-2-yl-, 1,4,5-trimethyl-imidazol-2-yl-, 4-methoxy-pyridin-2-yl-, 3-methylbutyryl-,3-trifluoromethyl-4-chlorophenyl-,1-(3-fluorophenyl)-imidazolin-2-yl-, and 1-isopropyl-imidazolin-2-yl-.

In other embodiments of formula (I) and formula (Ia), L-R¹⁵ taken together may be selected from 2-(2-methoxymethylpyrrolidin-1-yl)-ethyl-, 2-oxo-tetrahydropyrimidin-1-yl-, 2-oxo-pyrrolidin-1-yl-, benzyl, 4-chlorophenylcarbonyl, 3-(pyrrolidin-1-yl)-benzylamino-, 2-(2-amino-imidazol-1-yl)-ethyl-, 2-(2-methyl-imidazolin-1-yl)-ethyl-, 2-(imidazol-1-yl)-ethyl-, 2-dimethylaminosulfonyl-benzyloxy-, 2-fluoro-benzyl-N-methylamino-, propyl-, pyridin-2-yl-methyl-, 2-fluoro-benzylamino-, 2-chloro-benzylamino-, 2-methoxy-benzylamino-, 2-methyl-benzylamino-, 3-methoxycarbonyl-benzyloxy-, 3-carboxy-benzyloxy-, 3-(N,N-di-methanesulfonyl)-amino-benzyloxy-, 3-methanesulfonylamino-benzyloxy-, 3-N,N-dimethylacetamidinyl-benzyloxy-, 3-(2-methylimidazolin-1-yl)-phenyl-, morpholin-1-yl-carbonyl-, 3-[(2-, methylaminocarbonyl)-ethyl] -phenyl-, and 3-N,N-dimethylacetamidinyl-phenyl.

In still other embodiments of formula (I) and formula (Ia), J-R¹⁷ taken together may be selected from hydrogen, methoxy, N-ethyl-N-methylamino-methyl-, N-methylamino-methyl-, 1,3-dimethyl-imidazolidin-2-ylidineamino-, N-methylamino-methylcarbonyl-N-methylamino-methyl-, 1-methyl-pyrrolidin-ylidineamino-, N,N-dimethylacetamidinyl-, 2-(imidazolin-2-yl)-ethyl-, imidazolin-2-yl-, imino-morpholin-4-yl-methyl-, butyramidinyl-, cyclobutanecarboxamidinyl-, furan-2-yl-carboxamidinyl-, 1-methyl-imidazolin-2-yl-, 2,4,4-trimethyl-imidazolin-1-yl-, 1-methyl-imidazol-2-yl-, 1-(2-methoxy)-ethoxy-imidazolin-2-yl-, 1-isopropyl-imidazolin-2-yl-, 2,4-dimethyl-imidazolin-2-yl-, and morpholin-4-yl-.

According to another aspect of the invention, certain preferred compounds are those having the above formulae (Ia) wherein Q is the group (S¹).

Within the group of compounds wherein Q is (S) or more preferably (S¹), preferred are those compounds wherein R⁸ is K-R¹⁴; K is selected from a bond, C₁₋₂alkylene, -M₁-C(=O)-M₂-, -M₁-C(O)₂-M₂-, and -M₁-C(=O)NR¹⁶-M₂-,wherein M₁ and M₂ are selected from a bond and C₁₋₂alkylene; and R¹⁶ is hydrogen or lower alkyl; R¹⁴ is selected from hydrogen, lower alkyl, furyl, cycloalkyl, phenyl, pyridyl, imidazolyl, and imidazolinyl, wherein each R¹⁴ in turn is optionally substituted with one to three groups selected from R¹⁹; and R¹⁹ is selected from lower alkyl, halogen, cyano, halo(C₁₋₄)alkyl, halo(C₁₋₄)alkoxy, hydroxy, lower alkoxy, amino, (C₁₋₄alkyl)amino, (C₁₋₄alkyl)amino(C₁₋₄)alkyl, hydroxy(C₁₋₄)alkyl, (loweralkoxy)(C₁₋₄)alkyl, SO₂(C₁₋₄alkyl), -C(=O)H, -C(=O) (C₁₋₄ alkyl), pyrrolidinyl, and phenyl (said phenyl in turn being optionally substituted with one to two of lower alkyl, lower alkoxy, cyano, and/or halogen).

Within the group of preferred compounds wherein Q is (S) or more preferably (S¹), even more preferred are compounds wherein R⁸ is K-R¹⁴; K is selected from a bond, -C(=O)-, -C(=O)C₁₋₂alkylene-, -C(O)₂-, -C₁₋₂alkylene-C(O)₂-, -C(=O)NR¹⁶-, and -C₁₋₂alkylene-C(=O)NR¹⁶-, wherein R¹⁶ is hydrogen or methyl; R¹⁴ is selected from lower alkyl, furyl, cyclopentyl, phenyl, pyridyl, imidazolyl, and imidazolinyl, wherein each R¹⁴ in turn is optionally substituted with one to three groups selected from R¹⁹; and R¹⁹ is selected from lower alkyl, lower alkoxy, halogen, cyano, trifluoromethyl, and phenyl (said phenyl in turn being optionally substituted with one to two halogen).

Within this group of preferred compounds wherein Q is the group (S) or more preferably (S¹), even more preferred are compounds wherein R⁸ is selected from -C(=O)furyl, -C(=O)alkyl, -C(O)₂alkyl, -C₁₋₂alkylene-C(O)₂-(alkyl), -C₁₋₂aklene-C(=O)NH(alkyl), -C₁₋₂alkylene-C(=O)N(lower alkyl)(alkyl), -C(=O)NH(phenyl), phenyl, -C(=O)C₁₋₂alkylene(cyclopentyl), pyridyl, imidazolyl, and imidazolinyl, wherein each of said phenyl, pyridyl, imidazolyl, and imidazolinyl groups is in turn optionally substituted with one to three of lower alkyl, trifluoromethyl, halogen, cyano, methoxy, and/or phenyl, said phenyl in turn being optionally substituted with one to two of methyl, halogen, and/or cyano.

Within this group of preferred compounds, most preferred are those compounds wherein R¹⁴ is furyl optionally substituted with one to two of lower alkyl, trifluoromethyl, halogen, and/or cyano, and most preferred are those compounds wherein R⁸ is -C(=O)(furyl) .

According to another aspect of the invention, certain preferred compounds are those having the above formulae (Ia) wherein Q is a group of formula S¹, and wherein R⁸ is selected from alkylcarbonyl, furanylcarbonyl, alkoxycarbonyl, alkoxycarbonylalkyl, dialkylaminocarbonyl, optionally substituted phenylaminocarbonyl, cycloalkylalkylcarbonyl, optionally substituted phenyl, optionally substituted pyridinyl, optionally substituted imidazolyl, and optionally substituted imidazolinyl. In certain embodiments, R⁸ may be selected from 4-methyl-pentanoyl-, furan-2-carbonyl-, ethoxycarbonyl-, ethoxycarbonylmethyl-, 3-trifluoromethylphenyl-, dimethylaminocarbonylmethyl-, 3-chlorophenyl-, cyclopentyl-methylcarbonyl-, 3-fluorophenyl-aminocarbonyl-, 3,4-difluorophenyl-aminocarbonyl-, 3-cyanophenyl-aminocarbonyl-, pyridin-2-yl-, imidazol-2-yl-, 6-methyl-pyridin-2-yl-, 4-methyl-pyridin-2-yl, 1-oxypiridin-2-yl, 1-methyl-imidazolin-2-yl-, 1-methyl-imidazol-2-yl-, 1,4,5-trimethyl-imidazol-2-yl-, 4-methoxy-pyridin-2-yl-, 3-methylbutyryl-, 3-trifluoromethyl-4-chlorophenyl-, 1-(3-fluorophenyl)-imidazolin-2-yl-, and 1-isopropyl-imidazolin-2-yl-.

According to another aspect of the invention, preferred compounds are those having the above formulae (I) and/or (Ia) wherein Q is the group (T¹). Within this group of preferred compounds, more preferred are compounds wherein R¹¹ is cycloallcyl, more preferably cyclohexyl, and wherein R¹² is hydrogen.

According to another aspect of the invention, preferred compounds are those having the above formulae (I) and/or (Ia) wherein Q is the group (U¹). Within this group of preferred compounds wherein Q is the group (U¹), one subset of further preferred compounds are those wherein *n, p* and *q* are each 1; R⁹ is hydrogen or lower alkyl; R¹⁰ is -L-R¹⁵; L is selected from a bond, C₁₋₂alkylene, -C(=O)-, -NH(C₁₋₂alkylene)-, -N(lower alkyl) (C₁₋₂alkylene)-, and -O-(C₁₋₂alkylene)-; R¹⁵ is selected from alkyl, pyrrolidinyl, phenyl, pyridyl, imidazolyl, and tetrahydropyrimidinyl, wherein each of said pyrrolidinyl and tetrahydropyrimidinyl groups optionally has one to two carbon ring atoms replaced with a carbonyl group, and each of said R¹⁵ groups is optionally substituted with one to three groups selected from R²⁰; and R²⁰ is at each occurrence independently selected from lower alkyl, halogen, cyano, lower alkoxy, (loweralkoxy)(C₁₋₄)alkyl, amino, (C₁₋₄alkyl)amino, sulfonamidyl, CO₂H, CO₂(C₁₋₄alkyl), amidinyl, alkyl-amidinyl, and pyrrolidinyl.

Within this group of preferred compounds wherein Q is the group (U¹), even further preferred compounds are those wherein *n, p* and *q* are each 1; R⁹ is hydrogen; and R¹⁰ is selected from alkyl, pyrrolidinyl, -C₁₋₂alkylene(pyrrohdinyl), -C(=O)phenyl, C₁₋₂alkylene(phenyl), -C₁₋₂alkylene(pyridyl), -NH(C₁₋₂alkylene)(phenyl), -N(CH₃)(C₁₋₂alkylene)(phenyl), -C₁₋₂alkylene(imidazolyl), -O-(C₁₋₂alkylene)(phenyl), and tetrahydropyrimidinyl, wherein each of said pyrrolidinyl and tetrahydropyrimidinyl groups optionally has one to two carbon ring atoms replaced with a carbonyl group, and each of said pyrrolidinyl, phenyl, pyridyl, imidazolyl, and tetrahydropyrimidinyl groups optionally is substituted with one to three of lower alkyl, halogen, cyano, methoxy, methoxy(C₁₋₂)alkyl, amino, (C₁₋₄alkyl)amino, sulfonamidyl, CO₂H, CO₂(C₁₋₄alkyl),-N=C(CH₃)N(CH₃)₂, and/or pyrrolidinyl.

According to another aspect of the invention, preferred compounds are those having the above formulae (I) and/or (Ia) wherein Q is 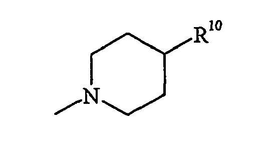 and wherein R¹⁰ is selected from alkyl, optionally substituted pyrrlolidinyl, optionally substituted pyrrolidinylalkyl, optionally substituted tetrahydropyrimidinyl, optionally substituted benzyl, optionally substituted phenylcarbonyl, optionally substituted benzylamino, optionally substituted imidazolylalkyl, optionally substituted imidazolinylalkyl, optionally substituted pyridinylalkyl, optionally substituted phenylalkylamino, optionally substituted benzyloxy, optionally substituted phenyl, and optionally substituted morpholinylcarbonyl.

R¹⁰ may be selected from 2-(2-methoxymethylpyrrolidin-1-yl)-ethyl-, 2-oxo-tetrahydro-pyrimidin-1-yl-, 2-oxo-pyrrolidin-1-yl-, benzyl, 4-chlorophenylcarbonyl, 3-(pyrrolidin-1-yl)-benzylamino-, 2-(2-amino-imidazol-1-yl)-ethyl-, 2-(2-methyl-imidazolin-1-yl)-ethyl-, 2-(imidazol-1-yl)-ethyl-, 2-dimethylaminosulfonyl-benzyloxy-, 2-fluoro-benzyl-N-methylamino-, propyl-, pyridin-2-yl-methyl-, 2-fluoro-benzylamino-, 2-chloro-benzylamino-, 2-methoxy-benzylamino-, 2-methyl-benzylamino-, 3-methoxycarbonyl-benzyloxy-, 3-carboxy-benzyloxy-, 3-(N,N-di-methanesulfonyl)-amino-benzyloxy-, 3-methanesulfonylamino-benzyloxy-, 3-N,N-dimethylacetamidinyl-benzyloxy-, 3-(2-methylimidazolin-1-yl)-phenyl-, morpholin-1-yl-carbonyl-, 3-[(2-methylaminocarbonyl)-ethyl] -phenyl-, and 3-N,N-dimethylacetamidinyl-phenyl-.

According to another aspect of the invention, preferred compounds are those having the above formulae (I) and/or (Ia) wherein Q is the group (U¹), and R⁹ and R¹⁰ are taken together to form a spirocyclic ring, more preferably a spirocyclic ring selected from one of 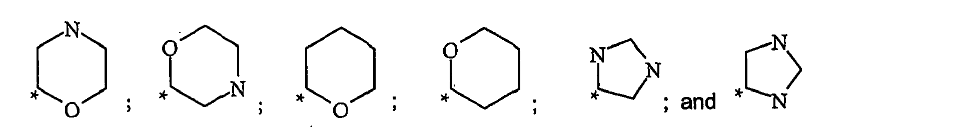 wherein * represents the point of attachment to the carbon ring atom to which R⁹ and R¹⁰ are attached, and each of said spirocyclic rings optionally has one to two carbon ring atoms replaced with a carbonyl group, and/or optionally has a benzo ring fused thereto, and wherein each of said spirocyclic rings and/or fused benzo rings is optionally substituted with one to two groups selected from lower alkyl, aminoalkyl, alkylaminoalkyl, and phenyl, wherein said phenyl group is in turn optionally substituted with one to two of halogen, cyano, lower alkoxy, and/or lower alkyl, more preferably with fluoro, chloro, and/or methoxy.

In certain embodiments, R⁹ and R¹⁰ taken together define one of 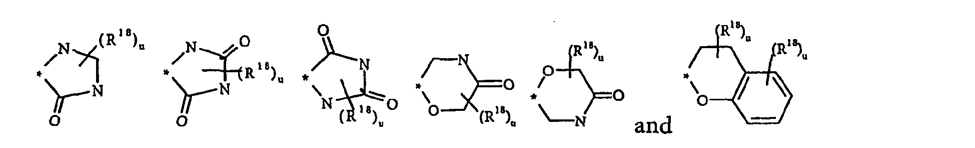 and R¹⁸ is at each occurrence selected from hydrogen, lower alkyl, (C₁₋₄alkyl)amino-(C₁₋₄)alkyl, and phenyl optionally substituted with one to two of halogen and/or lower alkoxy; and u is 0, 1, 2 or 3.

According to another aspect of the invention, preferred compounds are those having the above formulae (I) and/or (Ia) wherein Q is the group (V¹). Within this group of compounds, one subset of further preferred compounds are those wherein n is 1; G¹ and G² are both carbon; R¹¹ is -J-R¹⁷; R¹³ is selected from lower alkoxy, amidinyl and alkylamidinyl; J is selected from a bond, -C₁₋₂alkylene-, -C(=NH)-, -NR¹⁶-C(=NH)-, -N=CR¹⁶-NR^{16a}-, and -N=; R¹⁶ and R^{16a} are hydrogen, methyl, or ethyl; R¹⁷ is selected from hydrogen, alkyl, pyrrolidinyl, pyrrolinyl, imidazolyl, imidazolinyl, imidazolidinyl, morpholinyl, cycloalkyl, and furyl, except R¹⁷ is not furyl when J is -N=; and wherein each of said R¹⁷ groups is optionally substituted with one to three groups selected from R²¹; R²¹ is selected from lower alkyl, lower alkoxy, halogen and cyano; and t is 0, 1 or 2.

Further preferred within this group of preferred compounds [wherein Q is the group (V¹)], are those compounds wherein Q is

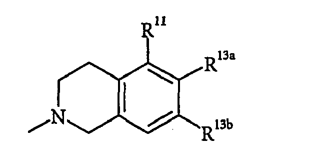

R¹¹ is selected from hydrogen, pyrrolidinyl, pyrrolinyl, imidazolyl, imidazolinyl, imidazolidinyl, morpholinyl, -N=C(CH₃)N(CH₃)₂, -N=(pyrrolidinyl), -N=(imidazolidinyl), -C₁₋₂alkylene(imidazolinyl), -C(=NH)(morpholinyl), -N(H)-C(=NH)-(alkyl), -N(H)-C(=NH)-(cyclobutyl), and -N(H)-C(=NH)-(furyl), wherein each of said pyrrolidinyl, pyrrolinyl, imidazolyl, imidazolinyl, imidazolidinyl, morpholinyl groups, cyclobutyl, and furyl groups is in turn optionally substituted with one to three of methyl, ethyl, propyl, methoxy, ethoxy, and/or methoxy(C₁₋₂alkyl); and R^{13a} and R^{13b} are selected from hydrogen, methoxy and -N=C(CH₃)N(CH₃)₂.

According to another aspect of the invention, preferred compounds are those compounds wherein Q is the group 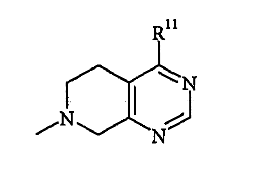 and R¹¹ is selected from hydrogen, pyrrolidinyl, pyrrolinyl, imidazolyl, imidazolinyl, imidazolidinyl, morpholinyl, -N=C(CH₃)N(CH₃)₂, -N=(pyrrolidinyl), N=(imidazolidinyl), -C₁₋₂alkylene(imidazolinyl), -C(=NH)(morpholinyl), -N(H)-C(=NH)-(alkyl), -N(H)-C(=NH)-(cyclobutyl), and -N(H)-C(=NH)-(furyl), wherein each of said pyrrolidinyl, pyrrolinyl, imidazolyl, imidazolinyl, imidazolidinyl, and morpholinyl rings is in turn optionally substituted with one to three of methyl, ethyl, propyl, methoxy, ethoxy, and/or methoxy(C₁₋₂alkyl). More preferred within this group of compounds are those wherein R¹¹ is morpholinyl.

In certain embodiments of formula (I) and formula (Ia) wherein Q is the group

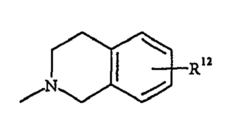

R¹² may be selected from hydrogen, alkoxy, dialkylaminoalkyl, alkylaminoalkyl, optionally substituted imidazolidin-2-ylideneamino, alkylaminoalkylcarbonylaminoalkyl, optionally substituted pyrrolidinylidineamino, acetamidinyl, optionally substituted imidazolylalkyl, optionally substituted imidazolyl, optionally substituted imidazolinyl, iminomorpholinylmethyl, amidinyl, and morpholinyl. In specific embodiments R¹² may be selected from hydrogen, methoxy, N-ethyl-N-methylamino-methyl-, N-methylamino-methyl-,1,3-dimethyl-imidazolidin-2-ylidineamino-, N-methylamino-methylcarbonyl-N-methylamino-methyl-, 1-methyl-pyrrolidin-ylidineamino-, N,N-dimethylacetamidinyl-, 2-(imidazolin-2-yl)-ethyl-, imidazolin-2-yl-, imino-morpholin-4-yl-methyl-, butyramidinyl-, cyclobutanecarboxamidinyl-, furan-2-yl-carboxamidinyl-, 1-methyl-imidazolin-2-yl-, 2,4,4-trimethyl-imidazolin-1-yl-, 1-methyl-imidazol-2-yl-, 1-(2-methoxy)-ethoxy-imidazolin-2-yl-,1-isopropyl-imidazolin-2-yl-,2,4-dimethyl-imidazolin-2-yl-, and morpholin-4-yl-.

In certain embodiments, the compounds of the invention are of the formula (Im) 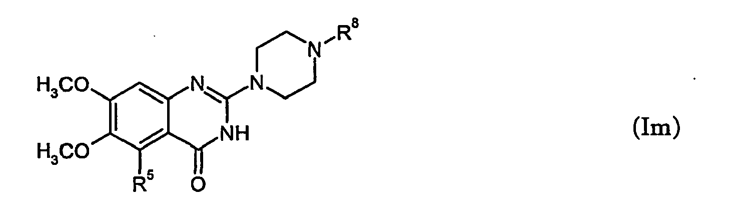 wherein
R⁵ is hydrogen, alkyl or alkoxy; and
R⁸ may be selected from alkylcarbonyl, furanylcarbonyl, alkoxycarbonyl, alkoxycarbonylalkyl, dialkylaminocarbonyl, optionally substituted phenylaminocarbonyl, cycloalkylalkylcarbonyl, optionally substituted phenyl, optionally substituted pyridinyl, optionally substituted imidazolyl, and optionally substituted imidazolinyl. In specific embodiments R⁸ maybe selected from 4-methyl-pentanoyl-, furan-2-carbonyl-, ethoxycarbonyl-, ethoxycarbonylmethyl-, 3-trifluoromethylphenyl-, dimethylaminocarbonylmethyl-, 3-chlorophenyl-, cyclopentyl-methylcarbonyl-, 3-fluorophenyl-aminocarbonyl-, 3,4-difluorophenyl-aminocarbonyl-, 3-cyanophenyl-aminocarbonyl-, pyridin-2-yl-, imidazol-2-yl-, 6-methyl-pyridin-2-yl-, 4-methyl-pyridin-2-yl, 1-oxypiridin-2-yl, 1-methyl-imidazolin-2-yl-, 1-methyl-imidazol-2-yl-, 1,4,5-trimethyl-imidazol-2-yl-, 4-methoxy-pyridin-2-yl-, 3-methylbutyryl-, 3-trifluoromethyl-4-chlorophenyl-, 1-(3-fluorophenyl)-imidazolin-2-yl-, and 1-isopropyl-imidazolin-2-yl-.

In certain embodiments, the compounds of the invention are of the formula (In) 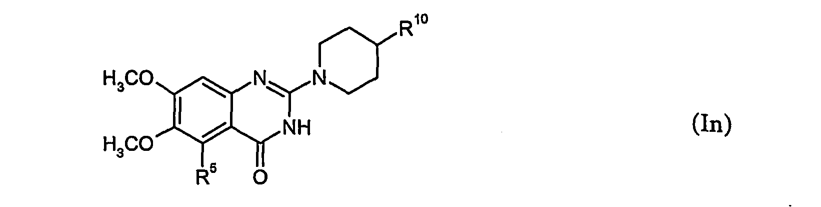 wherein
R⁵ is hydrogen, alkyl or alkoxy; and
R¹⁰ is selected from alkyl, optionally substituted pyrrlolidinyl, optionally substituted pyrrolidinylalkyl, optionally substituted tetrahydropyrimidinyl, optionally substituted benzyl, optionally substituted phenylcarbonyl, optionally substituted benzylamino, optionally substituted imidazolylalkyl, optionally substituted imidazolinylalkyl, optionally substituted pyridinylalkyl, optionally substituted phenylalkylamino, optionally substituted benzyloxy, optionally substituted phenyl, and optionally substituted morpholinylcarbonyl. In specific embodiments R¹⁰ may be selected from 2-(2-methoxymethylpyrrolidin-1-yl)-ethyl-, 2-oxo-tetrahydropyrimidin-1-yl-, 2-oxopyrrolidin-1-yl-, benzyl, 4-chlorophenylcarbonyl, 3-(pyrrolidin-1-yl)-benzylamino-, 2-(2-amino-imidazol-1-yl)-ethyl-, 2-(2-methyl-imidazolin-1-yl)-ethyl-, 2-(imidazol-1-yl)-ethyl-, 2-dimethylaminosulfonyl-benzyloxy-, 2-fluoro-benzyl-N-methylamino-, propyl-, pyridin-2-yl-methyl-, 2-fluoro-benzylamino-, 2-chloro-benzylamino-, 2-methoxy-benzylamino-, 2-methyl-benzylamino-, 3-methoxycarbonyl-benzyloxy-, 3-carboxy-benzyloxy-, 3-(N,N-di-methanesulfonyl)-amino-benzyloxy-, 3-methanesulfonylamino-benzyloxy-, 3-N,N-dimethylacetamidinyl-benzyloxy-, 3-(2-methylimidazolin-1-yl)-phenyl-, morpholin-1-yl-carbonyl-, 3-[(2-methylaminocarbonyl)-ethyl]-phenyl-, and 3-N,N-dimethylacetamidinyl-phenyl-.

In certain embodiments, the compounds of the invention are of the formula (Is) 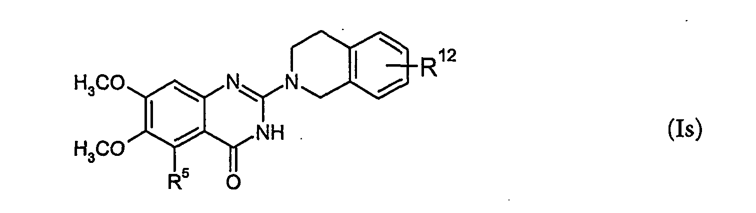 wherein:
R⁵ is hydrogen, alkyl or alkoxy; and
R¹² is selected from hydrogen, alkoxy, dialkylaminoalkyl, alkylaminoalkyl, optionally substituted imidazolidin-2-ylideneamino, alkylaminoallrylcarbonylaminoalkyl, optionally substituted pyrrolidinylidineamino, acetamidinyl, optionally substituted imidazolylalkyl, optionally substituted imidazolyl, optionally substituted imidazolinyl, iminomorpholinylmethyl, amidinyl, and morpholinyl. In specific embodiments R¹² may be selected from hydrogen, methoxy, N-ethyl-N-methylamino-methyl-, N-methylamino-methyl-, 1,3-dimethyl-imidazolidin-2-ylidineamino-, N-methylamino-methylcarbonyl-N-methylamino-methyl-, 1-methyl-pyrrolidin-ylidineamino-, N,N-dimethylacetamidinyl-, 2-(imidazolin-2-yl)-ethyl-, imidazolin-2-yl-, imino-morpholin-4-yl-methyl-, butyramidinyl-, cyclobutanecarboxamidinyl-, furan-2-yl-carboxamidinyl-, 1-methyl-imidazolin-2-yl-, 2,4,4-trimethyl-imidazolin-1-yl-, 1-methyl-imidazol-2-yl-, 1-(2-methoxy)-ethoxy-imidazolin-2-yl-, 1-isopropyl-imidazolin-2-yl-, 2,4-dimethyl-imidazolin-2-yl-, and morpholin-4-yl-.

In certain embodiments, the compounds of the invention are of the formula (It) 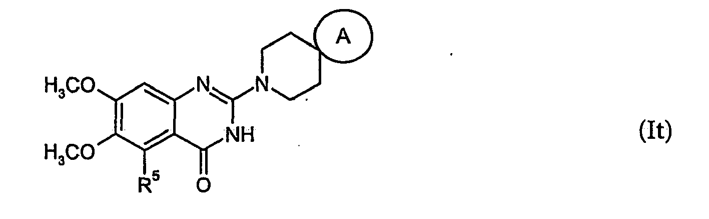 wherein
R⁵ is hydrogen, alkyl or alkoxy; and
A is a five or six-membered ring selected from: 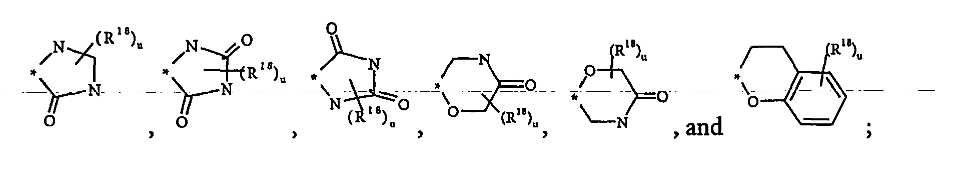 u is 0, 1 or 2;
* is the point of attachment for each ring A; and
R¹⁸ is at each occurrence selected from hydrogen, alkyl, alkylaminoalkyl, and phenyl optionally substituted with one to two of halogen and/or alkoxy.

According to another aspect of the invention, combinations of the preferred groups described herein form other preferred embodiments. In this manner, a variety of preferred compounds are embodied within the present invention. For example, another group of preferred compounds, selected from a combination of preferred groups recited above, are those compounds having a formula selected from, 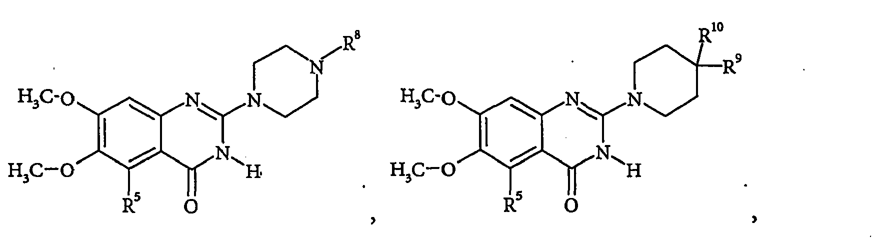 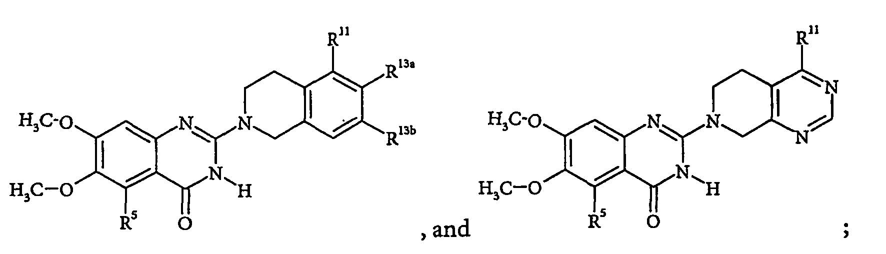 wherein R⁵ is selected from C₁₋₄alkyl, halogen, hydroxy, C₁₋₄alkoxy, -O(CH₂)ᵣNH₂, -O(CH₂)ᵣOH, -O(CH₂)ᵣO(C₁₋₄alkyl), -O(CH₂)ᵣO(phenyl), -O(CH₂)ᵣO(benzyl), -O(CH₂)ₛcycloalkyl, -O(CH₂)ₛ(phenyl), -(CH₂)ₛcycloalkyl, and -(CH₂)ₛ(phenyl), wherein each of said phenyl, benzyl, and cycloalkyl rings is optionally substituted with one to two of lower alkyl, substituted lower alkyl, cyano, and/or halogen, *r* is 1 or 2, and *s* is 0, 1 or 2; and R⁸, R⁹, R¹⁰, R¹¹, R^{13a}, and R^{13b} are selected from preferred and further preferred groups recited above.

Even more preferred are compounds as immediately defined above wherein R⁵ is selected from methyl, ethyl, n-propyl, isopropyl, halogen, methoxy, and ethoxy. Even more preferred are compounds wherein R⁵ is selected from methyl and methoxy.

Thus, further combination of preferred compounds may be selected from the preferred groups recited above.

Alpha-1 adrenoceptors mediate the contractile state of smooth muscle tissue and are present in the human prostate, bladder neck and urethra. Alpha-1 adrenoceptor stimulation also produces contraction of urethral and bladder neck smooth muscle, leading to increased resistance in urinary outflow. Thus, alpha-1 adrenoceptor antagonists may be useful in treating disorders of the urinary tract, as previously defined.

Alpha-1B adrenoceptors are present in the liver, heart and cerebral cortex and are believed to be involved in mediating vascular contractile and blood pressure responses. Alpha-1B adrenoceptors are also present in areas of the spinal cord which receive input from sympathetic neurons originating in the pontine micturition center and are presumed to be involved in the regulation of bladder function. Additionally, alpha-1B adrenoceptor antagonists are useful as analgesic/ antihyperalgesic therapies for treating pain, including symptoms of acute pain, inflammatory pain, neuropathic pain (including thermal and mechanical hyperalgesia as well as thermal and mechanical allodynia), complex regional pain syndromes (including reflex sympathetic dystrophy, causalgia and sympathetically maintained pain and the like).

However, it must be noted that in BPH, it is often the irritative symptoms which prompt the patient to seek treatment, and that these irritative symptoms may be present even in patients with no demonstrable obstruction (*i.e.* normal urine flow rates). By combining both alpha-1A and alpha-1B subtype selectivity in a compound, a reduction of both obstructive and irritative symptoms in patients with BPH may be achieved. Lower levels or lack of alpha-1D adrenoceptor antagonism should lead to reduced or fewer side effects than those associated with the use of non-subtype-selective agents.

In a preferred embodiment, the compounds of this invention are useful for treating disorders and symptoms which can be ameliorated by blockade of alpha1A/B adrenoceptors, such as reduction or alleviation of urinary tract disorders, e.g., pelvic hypersensitivity (including interstitial cystitis, prostatitis, pelvic pain syndrome, infectious cystitis, prostatodynia, and the like), overactive bladder, urinary frequency, nocturia, urinary urgency, detrusor hyperreflexia, outlet obstruction, BPH, prostatitis, urge incontinence, urethritis, idiophatic bladder hypersensitivity, sexual dysfunction, and the like.

In another preferred embodiment, the compounds of this invention are useful for treating disorders and symptoms which can be ameliorated by blockade of alpha-1A/B adrenoceptors, such as reduction or alleviation of pain disorders, e.g. inflammatory pain, neuropathic pain, cancer pain, acute pain, chronic pain or complex regional pain syndromes.

In a more preferred embodiment, the compounds of this invention are useful for treating disorders and symptoms which can be ameliorated by blockade of both alpha-1A and alpha-1B adrenoceptors with diminished blockade of alpha-1D adrenoceptors, such as reduction or alleviation of both outlet obstruction, such as benign prostatic hypertrophy, and irritative symptoms associated with it, such as pain.

In another preferred embodiment, the compounds of this invention are useful for the improvement of sexual dysfunction including male erectile dysfunction (MED) and female sexual dysfunction (FSD).

These and other therapeutic uses are described, e.g., in Goodman & Gilman's, The Pharmacological Basis of Therapeutics, ninth edition, McGraw-Hill, New York, 1996, Chapter 26, 601-616; and Coleman, Pharmacological Reviews 46:205-229 (1994).

The present invention includes pharmaceutical compositions comprising at least one compound of the present invention, or an individual isomer, racemic or non-racemic mixture of isomers or a pharmaceutically acceptable salt or solvate thereof, together with at least one pharmaceutically acceptable carrier, and optionally other therapeutic and/or prophylactic ingredients.

In general, the compounds of the present invention will be administered in a therapeutically effective amount by any of the accepted modes of administration for agents that serve similar utilities. Suitable dosage ranges are typically 1-500 mg daily, preferably 1-100 mg daily, and most preferably 1-30 mg daily, depending upon numerous factors such as the severity of the disease to be treated, the age and relative health of the subject, the potency of the compound used, the route and form of administration, the indication towards which the administration is directed, and the preferences and experience of the medical practitioner involved. One of ordinary skill in the art of treating such diseases will be able, without undue experimentation and in reliance upon personal knowledge and the disclosure of this Application, to ascertain a therapeutically effective amount of the compounds of the present invention for a given disease.

In general, compounds of the present invention will be administered as pharmaceutical formulations including those suitable for oral (including buccal and sublingual), rectal, nasal, topical, vaginal, or parenteral (including intramuscular, intraarterial, intrathecal, subcutaneous and intravenous) administration or pulmonary in a form suitable for administration by inhalation or insufflation. The preferred manner of administration is generally oral using a convenient daily dosage regimen which can be adjusted according to the degree of affliction.

A compound or compounds of the present invention, together with one or more conventional adjuvants, carriers, or diluents, may be placed into the form of pharmaceutical compositions and unit dosages. The pharmaceutical compositions and unit dosage forms may be comprised of conventional ingredients in conventional proportions, with or without additional active compounds or principles, and the unit dosage forms may contain any suitable effective amount of the active ingredient commensurate with the intended daily dosage range to be employed. The pharmaceutical compositions may be employed as solids, such as tablets or filled capsules, semisolids, powders, sustained release formulations, or liquids such as solutions, suspensions, emulsions, elixirs, or filled capsules for oral use; or in the form of suppositories for rectal or vaginal administration; or in the form of sterile injectable solutions for parenteral use. Formulations containing about one (1) to about 20 milligram of active ingredient or, more broadly, about 0.01 to about one hundred (100) milligrams, per tablet, are accordingly suitable representative unit dosage forms.

The compounds of the present invention may be formulated in a wide variety of oral administration dosage forms. The pharmaceutical compositions and dosage forms may comprise a compound or compounds of the present invention or pharmaceutically acceptable salts thereof as the active component. The pharmaceutically acceptable carriers may be either solid or liquid. Solid form preparations include powders, tablets, pills, capsules, cachets, suppositories, and dispersible granules. A solid carrier may be one or more substances which may also act as diluents, flavoring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. The powders and tablets preferably contain from about one (1) to about seventy (70) percent of the active compound. Suitable carriers include but are not limited to magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier, providing a capsule in which the active component, with or without carriers, is surrounded by a carrier, which is in association with it. Similarly, cachets and lozenges are included. Tablets, powders, capsules, pills, cachets, and lozenges may be as solid forms suitable for oral administration.

Other forms suitable for oral administration include liquid form preparations including emulsions, syrups, elixirs, aqueous solutions, aqueous suspensions, or solid form preparations which are intended to be converted shortly before use to liquid form preparations. Emulsions may be prepared in solutions, e.g., in aqueous propylene glycol solutions or may contain emulsifying agents, e.g., such as lecithin, sorbitan monooleate, or acacia. Aqueous solutions can be prepared by dissolving the active component in water and adding suitable colorants, flavors, stabilizing, and thickening agents. Aqueous suspensions can be prepared by dispersing the finely divided active component in water with viscous material, such as natural or synthetic gums, resins, methylcellulose, sodium carboxymethylcellulose, and other well known suspending agents. Solid form preparations include solutions, suspensions, and emulsions, and may contain, in addition to the active component, colorants, flavors, stabilizers, buffers, artificial and natural sweeteners, dispersants, thickeners, solubilizing agents, and the like.

The compounds of the present invention may be formulated for parenteral administration (*e.g.*, by injection, e.g. bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, e.g. solutions in aqueous polyethylene glycol. Examples of oily or nonaqueous carriers, diluents, solvents or vehicles include propylene glycol, polyethylene glycol, vegetable oils (*e.g.*, olive oil), and injectable organic esters (*e.g.*, ethyl oleate), and may contain formulatory agents such as preserving, wetting, emulsifying or suspending, stabilizing and/or dispersing agents. Alternatively, the active ingredient may be in powder form, obtained by aseptic isolation of sterile solid or by lyophilisation from solution for constitution before use with a suitable vehicle, *e.g.*, sterile, pyrogen-free water.

The compounds of the present invention may be formulated for topical administration to the epidermis as ointments, creams or lotions, or as a transdermal patch. Ointments and creams may, e.g., be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or coloring agents. Formulations suitable for topical administration in the mouth include lozenges comprising active agents in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

The compounds of the present invention may be formulated for administration as suppositories. A low melting wax, such as a mixture of fatty acid glycerides or cocoa butter is first melted and the active component is dispersed homogeneously, e.g., by stirring. The molten homogeneous mixture is then poured into convenient sized molds, allowed to cool, and to solidify.

The compounds of the present invention may be formulated for vaginal administration. Pessaries, tampons, creams, gels, pastes, foams or sprays may contain in addition to the active ingredient, such carriers as are known in the art to be appropriate.

The compounds of the present invention may be formulated for nasal administration. The solutions or suspensions are applied directly to the nasal cavity by conventional means, e.g., with a dropper, pipette or spray. The formulations may be provided in a single or multidose form. In the case of a dropper or pipette, dosing may be achieved by the patient administering an appropriate, predetermined volume of the solution or suspension. In the case of a spray, this may be achieved e.g. by means of a metering atomizing spray pump.

The compounds of the present invention may be formulated for aerosol administration, particularly to the respiratory tract and including intranasal administration. The compound will generally have a small particle size e.g. on the order of five (5) microns or less. Such a particle size may be obtained by means known in the art, e.g. by micronization. The active ingredient is provided in a pressurized pack with a suitable propellant such as a chlorofluorocarbon (CFC), e.g., dichlorodifluoromethane, trichlorofluoromethane, or dichlorotetrafluoroethane, or carbon dioxide or other suitable gas. The aerosol may conveniently also contain a surfactant such as lecithin. The dose of drug may be controlled by a metered valve. Alternatively the active ingredients may be provided in a form of a dry powder, e.g. a powder mix of the compound in a suitable powder base such as lactose, starch, and starch derivatives such as hydroxypropylmethyl cellulose, and polyvinylpyrrolidine (PVP). The powder carrier will form a gel in the nasal cavity. The powder composition may be presented in unit dose form e.g. in capsules or cartridges of *e.g.,* gelatin or blister packs from which the powder may be administered by means of an inhaler.

When desired, formulations can be prepared with enteric coatings adapted for sustained or controlled release administration of the active ingredient. For example, the compounds of the present invention can be formulated in transdermal or subcutaneous drug delivery devices. These delivery systems are advantageous when sustained release of the compound is necessary and when patient compliance with a treatment regimen is crucial. Compounds in transdermal delivery systems are frequently attached to an skin-adhesive solid support. The compound of interest can also be combined with a penetration enhancer, *e.g.,* Azone (1-dodecylazacycloheptan-2-one). Sustained release delivery systems are inserted subcutaneously into to the subdermal layer by surgery or injection. The subdermal implants encapsulate the compound in a lipid soluble membrane, *e.g.,* silicone rubber, or a biodegradable polymer, *e.g.,* polylactic acid.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Other suitable pharmaceutical carriers and their formulations are described in Remington: The Science and Practice of Pharmacy 1995, edited by Martin, Mack Publishing Company, 19th edition, Easton, Pennsylvania. Representative pharmaceutical formulations containing a compound of the present invention are described in Examples below.

Throughout the application, and in the following Schemes and Examples herein, the following abbreviations are used for ease of reference: BOC: tert-Butoxycarbonyl; BPH: Benign prostatic hypertrophy or benign prostatic hyperplasia; CBZ: Carbobenzyloxy; CNS: Central nervous system; DCE: Dichloroethane; DCM: Dichloromethane; DMF: *N,N*-Dimethylformamide; DMSO: Dimethylsulfoxide; EtOH: Ethanol; EtOAc: Ethyl Acetate; Hal: Halogen or halide; L: Leaving group; MeOH: Methanol; P: Protective group; Pd/C: Palladium on carbon; RT: room temperature; TEA: triethylamine; THF: Tetrahydrofuran

Compounds of the present invention may be made by the methods depicted in the illustrative synthetic reaction schemes shown and described below.

The starting materials and reagents used in preparing these compounds generally are either available from commercial suppliers, such as Aldrich Chemical Co., or are prepared by methods known to those skilled in the art following procedures set forth in references such as Fieser and Fieser's Reagents for Organic Synthesis; Wiley & Sons: New York, 1991, Volumes 1-15; Rodd's Chemistry of Carbon Compounds, Elsevier Science Publishers, 1989, Volumes 1-5 and Supplementals; and Organic Reactions, Wiley & Sons: New York, 1991, Volumes 1-40. The following synthetic reaction schemes are merely illustrative of some methods by which the compounds of the present invention may be synthesized, and various modifications to these synthetic reaction schemes may be made and will be suggested to one skilled in the art having referred to the disclosure contained in this Application.

The starting materials and the intermediates of the synthetic reaction schemes may be isolated and purified if desired using conventional techniques including but not limited to filtration, distillation, crystallization, chromatography, and the like. Such materials maybe characterized using conventional means, including physical constants and spectral data.

Unless specified to the contrary, the reactions described herein preferably take place at atmospheric pressure over a temperature range from about -78°C to about 150°C, more preferably from about 0°C to reflux, and most preferably and conveniently at about room (or ambient) temperature, *e.g.,* about 20°C.

Schemes 1 to 9 describe methods to prepare compounds of Formula I. Scheme 10 describes a method to prepare intermediates (1) useful in Schemes 1-9 to prepare compounds of formula (I).

### Scheme 1

Scheme 1 describes a method of preparing a compound of Formula Ib wherein X is carbon or nitrogen, fused ring B is optionally present, and Z, R, R', R⁵, R⁷, *m* and *n* are as defined in the claims herein. 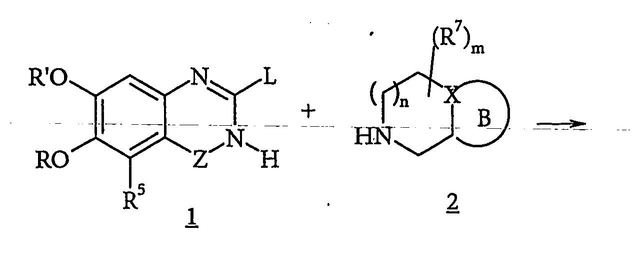 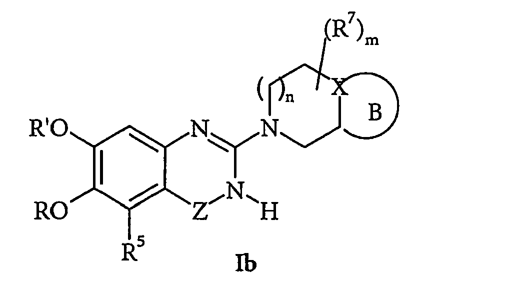 Reacting the free amine of compound 2, with compound 1 in an inert solvent such as lower alkanol, methoxyethanol, DMSO or DMF, optionally in the presence of a base such as, but not limited to sodium carbonate, sodium bicarbonate, triethyl amine, tributylamine and the like, can give a compound of Formula Ib.
Compounds 1 wherein L is a leaving group such as halogen, preferably chloro, or SCH₃ (as in Ex. G-1, below) can be prepared according to Cronin et al., J. Med. Chem. 11:136-138 (1968) or as described in WO 02/053558 A1, incorporated herein by reference. Alternatively, 2-chloro-quinazoline compounds 1 can be prepared as described below in Scheme 10.

### Scheme 2

Scheme 2 describes a method of preparing a compound of Formula Ic wherein R¹¹ is attached to the pyrimidinyl ring via a nitrogen atom (*i.e.,* R¹¹ is NRR), and the remaining variables are as defined herein.

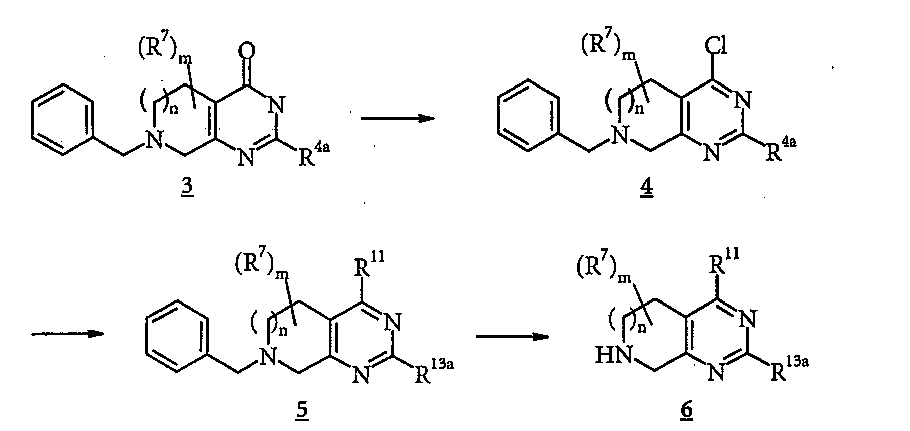

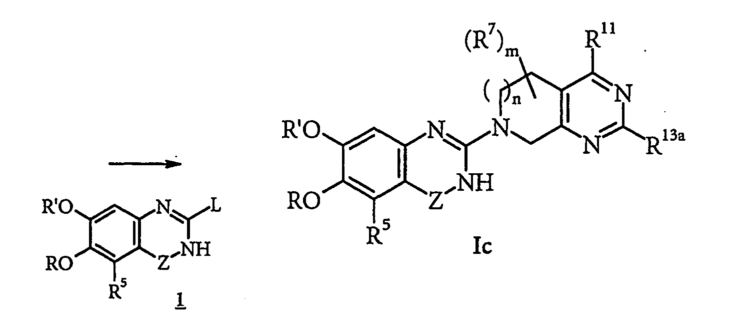

Compounds 3 can be prepared according to Ozdowska et al., Rocz.Chem. 50:1771-1775 (1976), and halogenated with phosphorous oxychloride to yield the chloro derivative 4, which can be reacted with an appropriate amine (*i.e.*, to provide the desired group R¹¹) in an inert solvent such as an alkanol, methoxyethanol, DMSO or DMF to yield the substituted 5,6,7,8-tetrahydro-pyrido[3,4-*d*]pyrimidine 5. The benzyl group of compound 5 can be removed by procedures known to one skilled in the art to yield the free base 6. A detailed description of the techniques applicable to protective groups and their removal can be found in Greene and Wuts, Protective Groups in Organic Synthesis, Wiley and Sons, New York (1991). For example a method of debenzylation can be carried out with a suitable catalyst (*e.g.,* 10% Pd/C) in the presence of ammonium formate and in an appropriate solvent, typically an alcohol, preferably MeOH/EtOH, at about 20 °C to about 100°C, and more preferably at reflux. Compounds of Formula Ic can be obtained by reacting the free amine 6 with a quinazolinone derivative of Formula 1, wherein L is a leaving group, preferably a halo group, and even more preferably a chloro group, in an inert solvent such as an alkanol, preferably n-butanol or methoxyethanol, by procedures known to one skilled in the art.

### Scheme 3

Scheme 3 describes a method of preparing a compound of Formula Id wherein the variables are as defined herein. 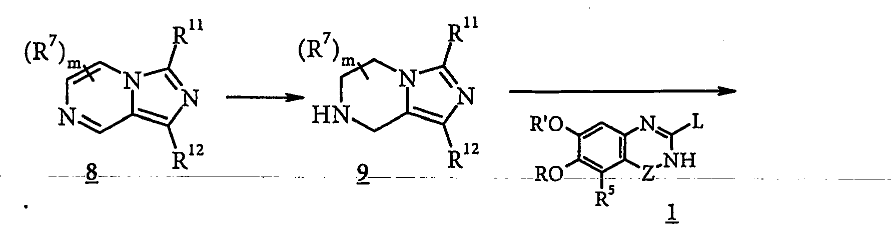 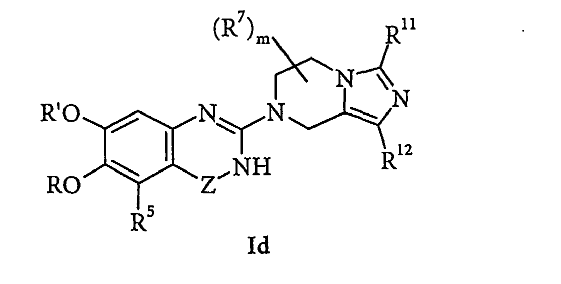 Compound 8 [prepared according to Abushanab et al. J. Heterocycl. Chem. 12:211 (1975)] can be hydrogenated in the presence of a catalyst, preferably Adam's catalyst (platinum (IV)oxide) to give compound 9. Suitable solvents are alkanols, preferably EtOH. Compounds of Formula Id can be obtained by reaction of the free amine 9, with a quinazolinone derivative of Formula 1, wherein L is a leaving group as described in the previous schemes.

### Scheme 4

Scheme 4 describes a method of preparing a compound of Formula Ie wherein the variables are as defined herein.

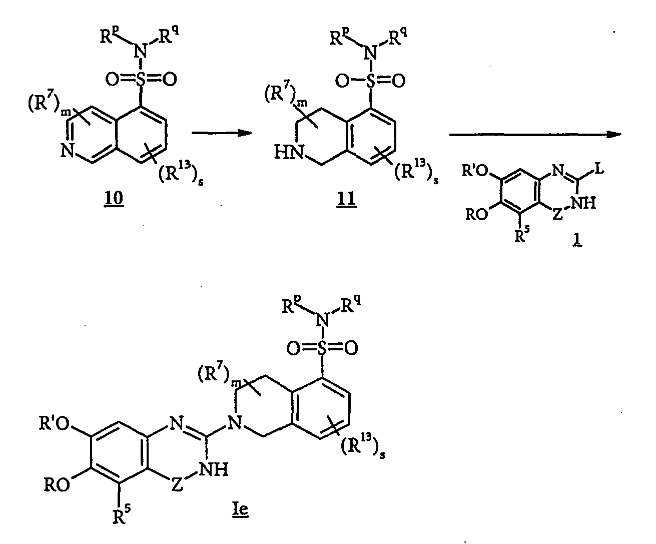

Compounds 10 [prepared according to Morikawa et al., Chem. Pharm. Bull. 40:770-773 (1992)], can be hydrogenated in the presence of a catalyst, preferably platinum oxide to give compounds 11. Suitable solvents are alkanols, preferably MeOH. Compounds of Formula Ie are obtained by reaction of the free amine 11, with a quinazolinone derivative 1, wherein L is a leaving group as described in Scheme 2.

### Scheme 5

Scheme 5 describes a method of preparing a compound of Formula If wherein R¹¹ is benzyl, and the remaining variables are as defined in the claims herein.

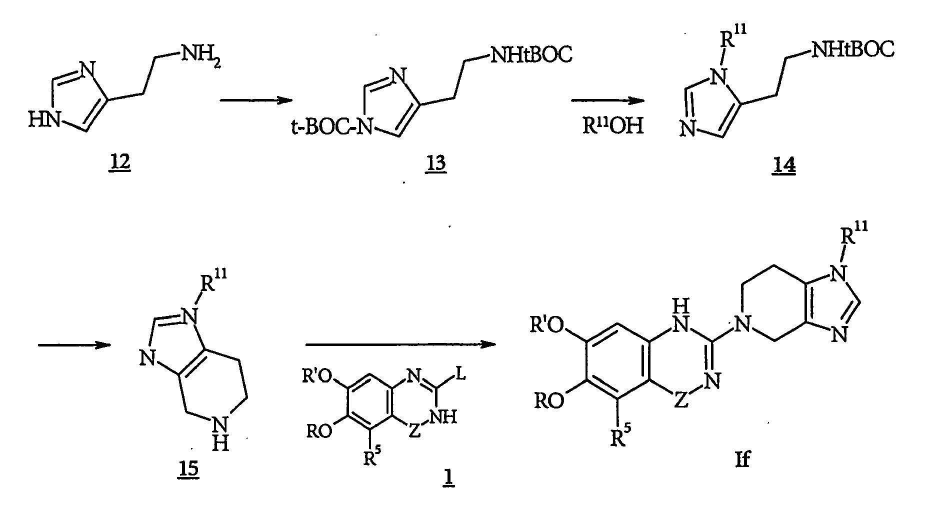

Protection of the amino groups of histamine 12 with di-*tert*-butyl dicarbonate under conditions well known to the skilled artisan can give compounds 13. Formation of the amine of Formula 14 can be effected when the protected histamine 13 is treated with a solution of benzyl alcohol and a base such as diisopropylethyl amine to which a solution of triflic anhydride in an anhydrous **h**alogenated solvent, such as DCM, has been added. Deprotection of 14 in the presence of an acid, preferably in trifluoroacetic acid in a solvent such as DCM, followed by Mannich cyclization preferably with formaldehyde in the presence of an aqueous acid such as hydrochloric acid, provides amines 15.
Compounds of Formula If can be obtained by reaction of the free amine 15, with a quinazolinone derivative of Formula 1*,* wherein L is a leaving group as described in the previous schemes.

### Scheme 6

Scheme 6 describes a method of preparing a compound of Formula Ig wherein R¹¹ is phenyl and the remaining variables are as defined in the claims herein.

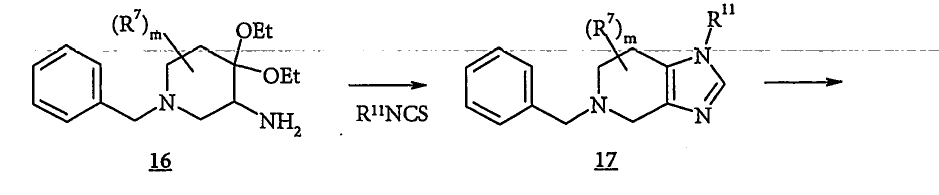

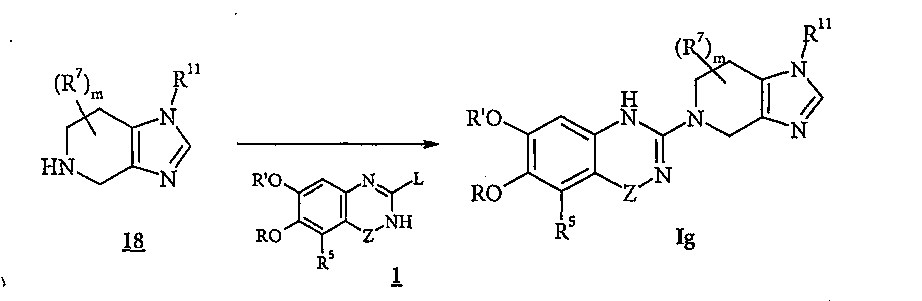

Compounds 16 (prepared as described in Tetrahedron, 1995, at 13447-13453), can be treated with a phenyl isothiocyanate of formula R¹¹NCS in an inert solvent such as chloroform or DMF, preferably chloroform, followed by acid catalyzed cyclization with a diluted acid such as hydrochloric acid, to the imidazolethione, which is desulfurized by methods known in the art such as by oxidation with hydrogen peroxide or by reduction with Raney Nickel to give compounds 17. Deprotection of the amino group in conditions well known in the art, such as by catalytic hydrogenation, i.e. 10% Pd/C, palladium hydroxide, palladium acetate, etc., in the presence of ammonium formate and in an appropriate solvent, typically an alcohol (*e.g.*, EtOH, MeOH, isopropanol, any appropriate mixture of alcohols), preferably in the presence of Pd/C gives amines 18. Compounds of Formula Ig can be obtained by reaction of the free amine 18, with a quinazolinone derivative 1, wherein L is a leaving group, as described in the previous schemes.

### Scheme 7

Scheme 7 describes a method of preparing a compound of Formula Ih wherein R¹⁷ is attached to the methylene group via a nitrogen atom, and the remaining variables are as defined herein.

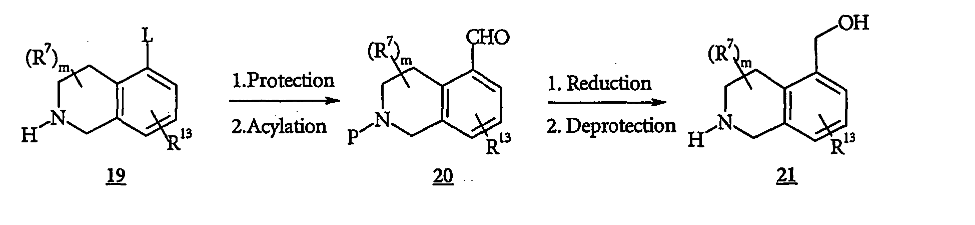

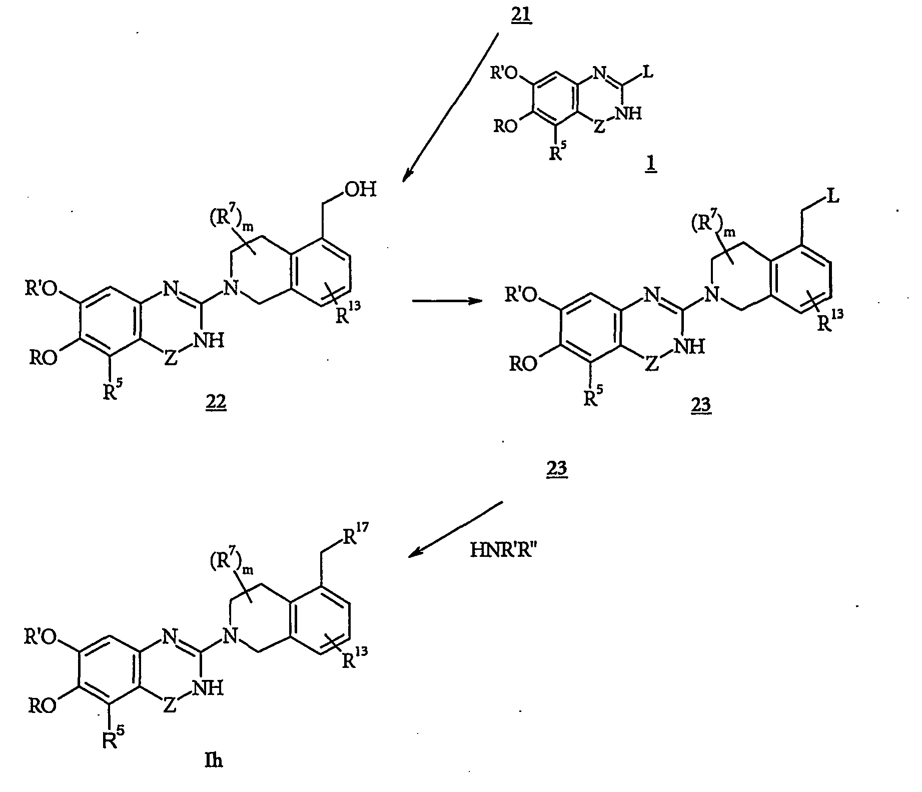

The amine functionality of a compound of Formula 19, wherein L is a halide, is protected with a protective group such as benzyl, BOC, carbamate, or CBZ, under conditions well known in the art. Formylation with a *N',N*'-disubstituted formyl amide such as *N*-formylmorpholine in the presence ofbutyllithium can afford an aldehyde of formula 20. Reduction of the aldehyde with a metallic hydride such as lithium aluminum hydride or sodium borohydride, followed by deprotection by methods well known in the art, such as with ammonium formate and Pd/C in a solvent such as MeOH in the case of benzyl, can afford the alcohol of general Formula 21. Reacting the free amine of Formula 21 with compounds 1 wherein L is a leaving group such as halogen in an inert solvent affords compounds 22. The hydroxy group can be converted to a leaving group such as a halide with halogen acids such as hydrobromic acid or with inorganic acid halides such as,. e.g., SOCl₂, POBr₃, or POCl₃ to afford compounds 23, which can further undergo amination with an amine (*i.e.,* R¹⁷ being an amine) to give a compound of general Formula Ih.

### Scheme 8

Scheme 8 describes a method of preparing compounds of Formula Ii and Ij, wherein the variables are as defined herein.

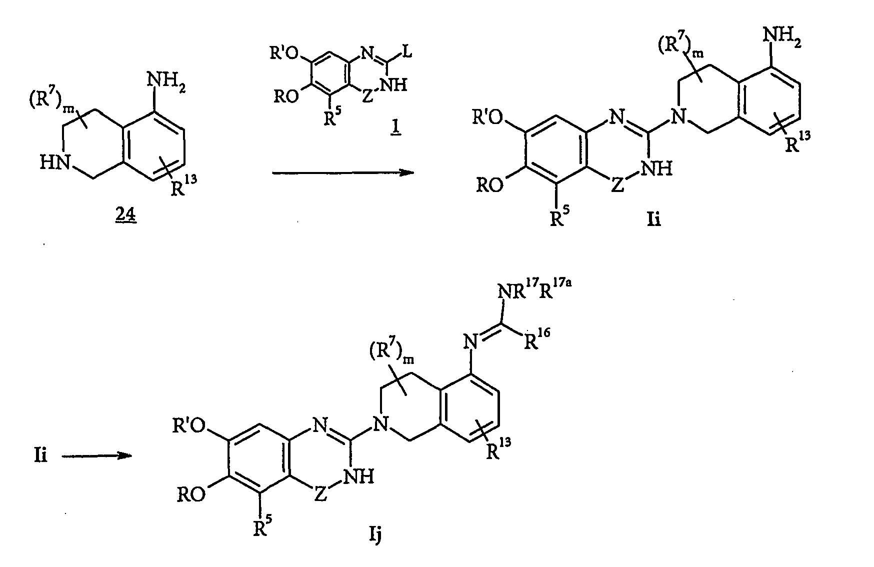

Compounds of formula Ii can be prepared from compounds 24 (prepared as described in WO 95/13274) with the quinazoline derivative 1, as described in Scheme 1.
Compounds of Formula Ij, wherein R¹¹ is -N=CR¹⁶-NR^{17a}R¹⁷, can be prepared by reacting compounds of formula Ii, with a disubstituted amide and phosphorous oxychloride.

### Scheme 9

Scheme 9 describes a method of preparing a compound of Formula Ik wherein the variables are as defined herein.

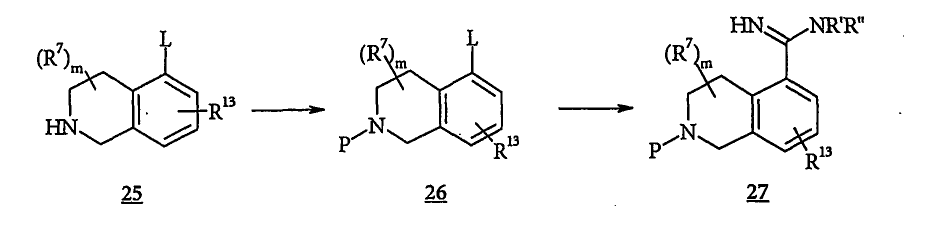

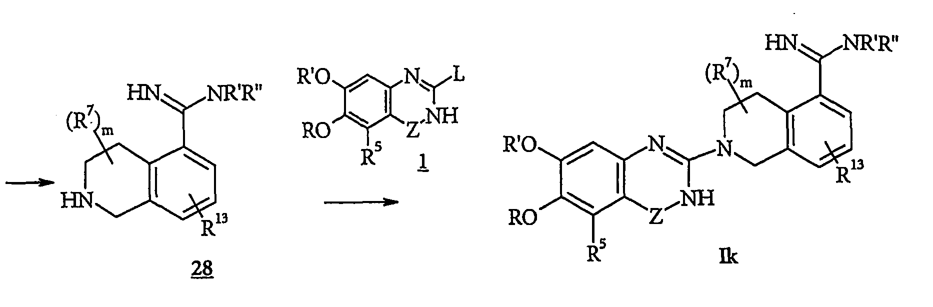

After protection of the amino group of compound of Formula 25, wherein L is a halogen, preferably bromo or chloro, following procedures well known in the art as described herein to afford compound of Formula 26, the halogen group can be replaced with an amidine group of general formula -C(=NH)-NR'R", by treatment with butyllitium followed by an aminocarbonitrile compound to give an imino amine of general Formula 27. Removal of the amino protecting group, e.g. with an acid, such as trifluoroacetic acid if the protective group is BOC, and coupling with the quinazoline derivative 1 afford compounds of Formula Ik

### Scheme 10

Scheme 10 illustrates a method for making 2-chloro-quinazolin-4-one compounds 34, 1 (wherein L is Cl and Z is C(=O)), used as starting material in Schemes 1 through 9.

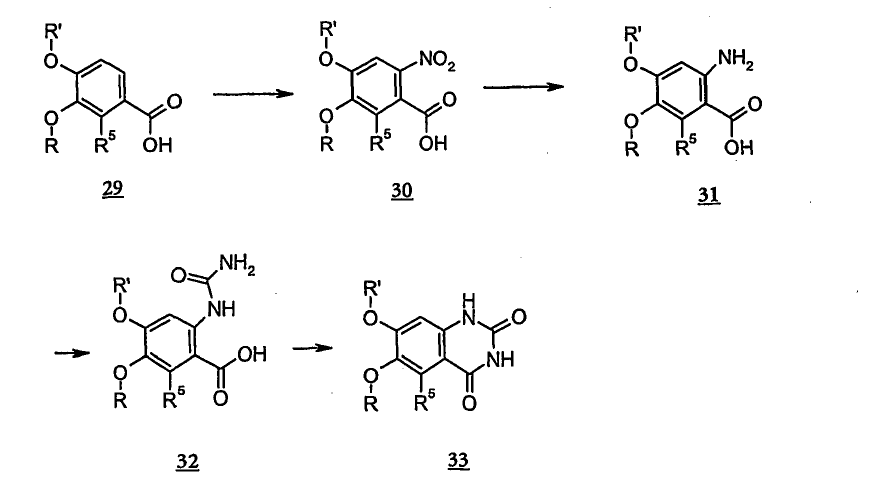

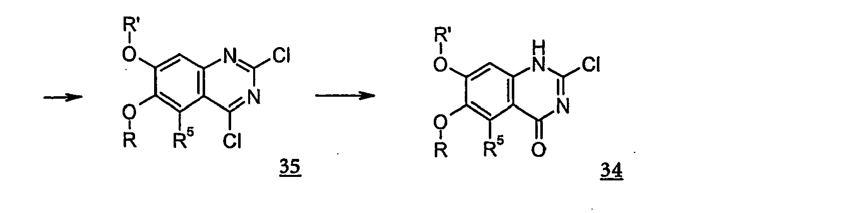

The procedure of Scheme 10 is described by Cronin et al., J. Med. Chem. 11:136-138 (1968) and by WO 02/053558 A1. Nitro-acids (30) are commercially available, or can be readily prepared by one skilled in the field from carboxylic acids (29) using several methods, including that of Kowalczyk et al., Organic Process Research and Development 1:355-358 (1997). Carboxylic acids (29) are commercially available.
Nitro acids (30) are dissolved in water by addition of base (e.g., NaOH, KOH, LiOH). A heterogenous catalyst on an inert support is added (e.g., palladium on carbon), and the reaction mixture is exposed to a hydrogen atmosphere either directly (hydrogen gas) or indirectly (transfer hydrogenation technique using e.g., formate salts, hydrazine, etc., as the hydrogen source). The nitro-group is thereby converted to an amino group to provide compounds (31).
Compounds (31) can be converted to a urea (32) by addition of a cyanate salt (e.g., KOCN, NaOCN) and an acid (e.g., HCl, HOAc). The urea (32) is then cyclized to a dione derivative (33) by adding a base (e.g., NaOH, KOH) and heating the reaction mixture. The dione (33) is precipitated by adding an acid (e.g., HCl, HOAc) to the reaction mixture, and the dione (33) may be isolated such as by filtration. Other acids also may be used, e.g., any acid that will generate HOCN in-situ from the cyanate salt and the acid.
Dione intermediate (33) is converted to dichloroquinazoline (35) by combining (33) with a chlorinating and dehydrating agent (e.g., phosphorous oxychloride) in an organic solvent (e.g. acetonitrile) and heating the reaction mixture. The dichloroquinazoline (35) is isolated by quenching the reaction mixture into water and filtering the precipitated product, or by quenching the reaction mixture into a mixture of water and a water-immiscible solvent (e.g. methylene chloride), and extracting the product into the organic solvent. The solvent is evaporated to provide compound (35).
Compound (35) is then combined with a base (e.g., KOH, NaOH) in a mixture of water and a solvent like THF. At the end of the reaction, the organic solvent is partially removed by distillation, an acid (e.g. HOAc) is added, and the compound (34) is collected via filtration.
Dione intermediates (33) are commercially available or can be readily prepared by one skilled in the field, e.g., as described in Mizuno et al., Heteroatom Chemistry 11:428-433 (2000); Mizuno et al., Tetrahedron Letters 41:1051-1051 (2000); US 6,376,667-B1; US 6,048,864; WO 97/23462; EP 775,697; and so forth.

### EXAMPLES

The following preparations and examples are provided to enable those skilled in the art to more clearly understand and to practice the present invention. However, these Examples should not be considered as limiting the scope of the invention, but merely as being illustrative and representative thereof.

### Example A-1: 5,6,7-Trimethoxy-2-[4-(4-methyl-pentanoyl)-piperazin-1-yl]-1H quinazolin-4-one

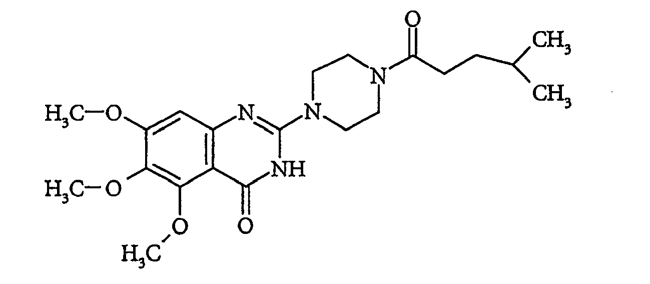

### Step 1: Preparation of 4-(4-methyl-pentanoyl)-piperazine-1-carboxylic acid tert-butyl ester

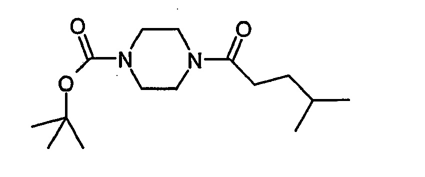

A stirred mixture of EtOAc (70 mL) containing 1.6 g (8.6 mmol) of 1-BOC piperazine and 25 mL of saturated aqueous sodium carbonate was treated dropwise with 1.2 g (9 mmol) of 4-methylvaleryl chloride. After 2 h of stirring, an additional 0.2 g (1.5 mmol) of 4-methylvaleryl chloride was added and stirring was continued for an additional 2 h. The layers were separated and the organic phase was dried over potassium carbonate, filtered, and concentrated to furnish 2.45 g of 4-(4-methyl-pentanoyl)-piperazine-1-carboxylic acid tert-butyl ester. Mp 88-90.2°C.

### Step 2: Preparation of 4-methylpentanoylpiperazine

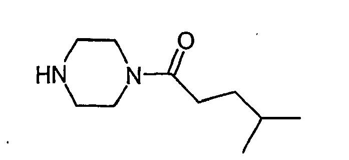

4-(4-Methyl-pentanoyl)-piperazine-1-carboxylic acid tert-butyl ester from Step 1 (2.45 g) was dissolved in 15 mL of hot EtOH and was treated with 15 mL of EtOH containing 1.5 g of HCl. The solution was kept at reflux for 5 min, and concentrated to approximately 15 mL. Addition of diethyl ether precipitated the HCl salt of 4-methylpentanoylpiperazine, 1.5 g (79%). Mp 186.2-187.5 °C.

### Step 3: Example A-1

A mixture containing 300 mg of 2-chloro-5,6,7-trimethoxyquinazolin-4-one (1.1 mmol), 253 mg of of 4-methylpentanoylpiperazine from Step 2 (1.15 mmol), 400 mg TEA (4 mmol) and 3-4 mL *N*-methylpyrrolidinone was stirred at 80 °C for 6 h. The *N*-methylpyrrolidinone was removed *in vacuo* and the remainder was treated with hot EtOH, and the insoluble white free base of Example A-1 was collected. This was further purified by trituation with water and recrystallization from EtOH to furnish 140 mg of Example A-1 (30%). Mp 211.4-211.7 °C,
ms 418.49 (M+H). Anal. (C₂₁H₃₀N₄O₅) Calcd.: C, 60.27; H, 7.23; N, 13.39. Found: C, 60.19; H, 7.17; N, 13.45. The hydrochloride salt of Example A-1 was prepared from ethanol-diethyl ether. Mp 198-201°C, Anal. (C₂₁H₃₀N₄O₅•HCl) Calcd.: C, 55.44; H, 6.87; N, 12.32. Found: C, 55.30; H, 6.80; N, 12.39.

### EXAMPLES A-2 to A-21

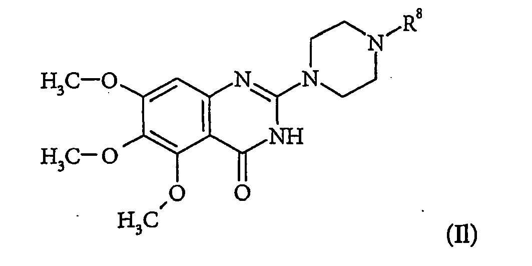

Compounds having the above formula (II), wherein R⁸ has the values reported in Table 1 were prepared following the same or similar method as described in A-1 except a differently-substituted piperazine was coupled with the chloroquinazolinone in the last step.

**TABLE 1**

| Ex. | R⁸ | MW |
|---|---|---|
| A-2 | 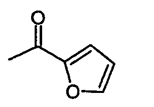 | 414.42 |
| A-3 | 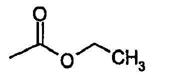 | 392.41 |
| A-4 | 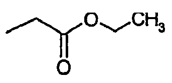 | 406.44 |
| A-5 | 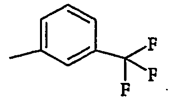 | 464.44 |
| A-6 | 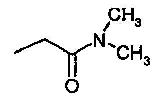 | 405.45 |
| A-7 | 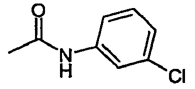 | 473.91 |
| A-8 | 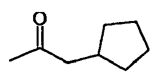 | 430.50 |
| A-9 | 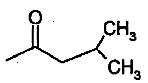 | 404.46 |
| A-10 | 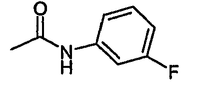 | 457.46 |
| A-11 | 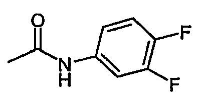 | 475.45 |
| A-12 | 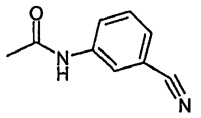 | 464.48 |
| A-13 | 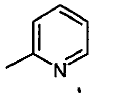 | 397.43 |
| A-14 | 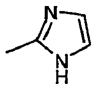 | 386.41 |
| A-15 | 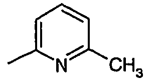 | 411.46 |
| A-16 | 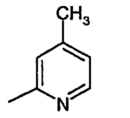 | 411.46 |
| A-17 | 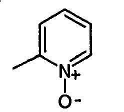 | 413.43 |
| A-18 | 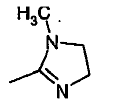 | 402.45 |
| A-19 | 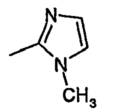 | 400.44 |
| A-20 | 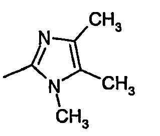 | 428.49 |
| A-21 | 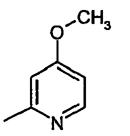 | 427.46 |

### EXAMPLES A-22 to A-40

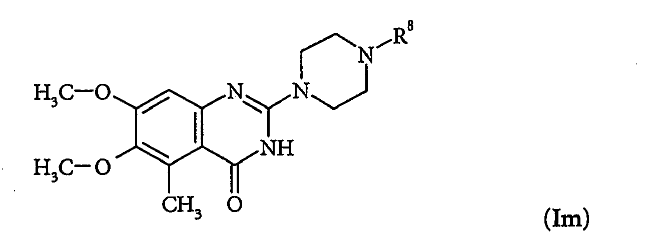

Compounds having the above formula (Im) wherein R⁸ has the values reported in Table 2 were prepared following the same or similar method as described in A-1 except in the last step, an appropriately-substituted piperazinyl compound was coupled with 2-chloro-6,7-dimethoxy-5-methylquinazolin-4-one instead of 2-chloro-5,6,7-trimethoxyquinazolin-4-one.

**TABLE 2**

| Ex. | R⁸ | MW |
|---|---|---|
| A-22 | 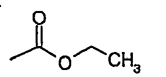 | 376.41 |
| A-23 | 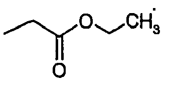 | 390.44 |
| A-24 | 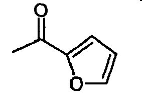 | 398.42 |
| A-25 | 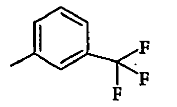 | 448.44 |
| A-26 | 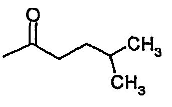 | 402.49 |
| A-27 | 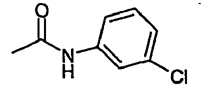 | 457.92 |
| A-28 | 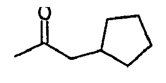 | 414.50 |
| A-29 | 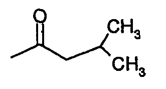 | 388.46 |
| A-30 | 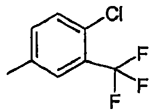 | 482.89 |
| A-31 | 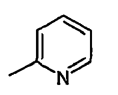 | 381.43 |
| A-32 | 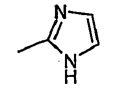 | 370.41 |
| A-33 | 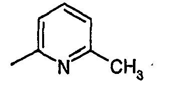 | 395.46 |
| A-34 | 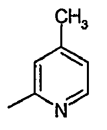 | 395.46 |
| A-35 | 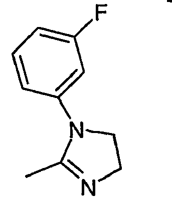 | 466.51 |
| A-36 | 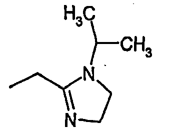 | 428.53 |
| A-37 | 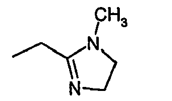 | 400.48 |
| A-38 | 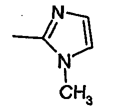 | 384.44 |
| A-39 | 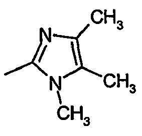 | 412.49 |
| A-40 | 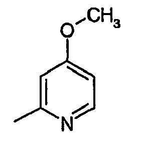 | 411.46 |

Additional compounds prepared according to the procedure of Example A-1 are shown in Table 9.

### Example B-1: 2-(3-Cyclohexyl-5,6-dihydro-8H-imidazo[1,5-a]pyrazin-7-yl)-6,7-dimethoxy-5-methyl-1H-quinazolin-4-one

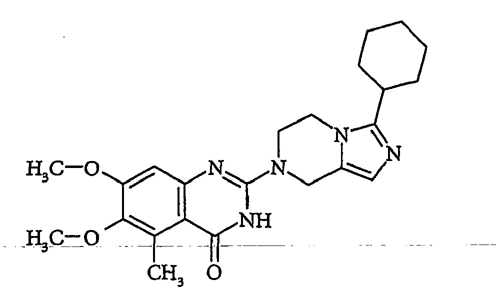

Example B-1 was made following the same or similar procedure as Example A-1, except 3-cyclohexyl-5,6-dihydro-8H-imidazo[1,5-a]pyrazine was coupled to the 2-chloroquinazolinone in the last step. MW = 423.51.

### Example C-1: 6,7-Dimethoxy-2-{4-[2-(2-methoxymethyl-pyrrolidin-1-yl)-ethyl]-piperidin-1-yl}-5-methyl-1H-quinazolin-4-one

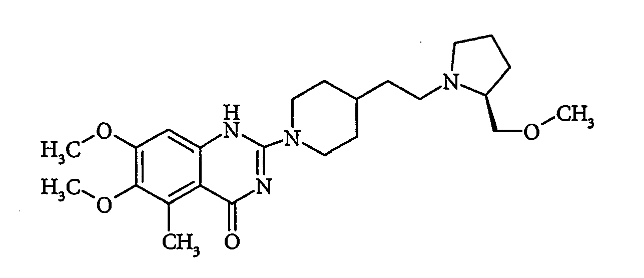

### Step 1: Preparation of 4-(2-hydroxy-ethyl)-piperidine-1-carboxylic acid benzyl ester

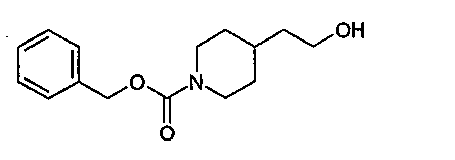

Benzyl chloroformate (12 mL, 84.1 mmol) was added dropwise via syringe to a stirred solution of 2-piperidin-4-ylethanol (10.04 g, 77.4 mmol) and TEA (11.8 mL, 84.7 mmol) in 100 mL of acetonitrile. The reaction was stirred at rt in a water bath overnight. The suspension was filtered and the filtrate was diluted with EtOAc. The solution was washed with brine and the organic extracts were dried with magnesium sulfate. The crude reaction mixture was concentrated to give 16.43 g (81%) of 4-(2-hydroxy-ethyl)-piperidine-1-carboxylic acid benzyl ester as an orange/yellow oil. ¹H NMR (CDCl₃) 1.1-1.7 (m, 5H), 1.5 (t, 2H, *J*= 6.4), 2.7 (t, 2H, *J*= 12), 3.7 (t, 2H, *J*= 6.5), 4.2 (m, 2H), 5.1 (s, 2H), 7.25-7.4 (m, 5H).

### Step 2: Preparation of 4-(2-bromo-ethyl)-piperidine-1-carboxylic acid benzyl ester

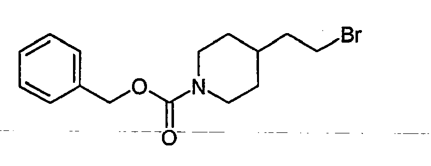

A solution of triphenylphosphine (4.4 g, 16.8 mmol) in DCM (10 mL) was added via addition funnel to a solution of 4-(2-hydroxy-ethyl)-piperidine-1-carboxylic acid benzyl ester from Step 1 (4.0 g, 15.3 mmol) and carbon tetrabromide (5.48 g, 16.5 mmol) in DCM (20 mL) at 0 °C. The reaction was gradually warmed to rt and stirred overnight.
The reaction mixture was concentrated and chromatographed (6:1 hexane/EtOAc) to give 4.27 g (86%) of 4-(2-bromo-ethyl)-piperidine-1-carboxylic acid benzyl ester as a light tan oil. TLC R*f* 0.5 (4:1 hexane/EtOAc) ¹H NMR (CDCl₃) 1.1 (m, 2H), 1.7 (m, 3H), 1.8 (t, 2H, *J=* 6.8), 2.8 (t, 2H, *J*= 12), 3.4 (t, 2H, *J*= 6.8), 4.2 (m, 2H), 5.1 (s, 2H), 7.3 (m, 5H).

### Step 3: Preparation of (S)-4-[2-(2-methoxymethyl-pyrrolidin-1-yl)-ethyl]-piperidine-1-carboxylic acid benzyl ester

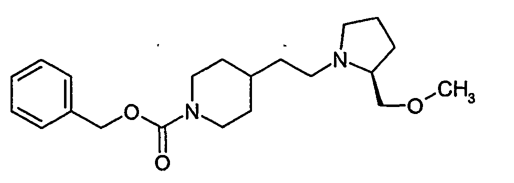

(S)-(+)-2-(Methoxymethyl)pyrrolidine (320 µL, 2.59 mmol) was added to a stirred solution of 4-(2-bromo-ethyl)-piperidine-1-carboxylic acid benzyl ester from Step 2 (797 mg, 2.44 mmol) and TEA (360 µL, 2.58 mmol) in acetonitrile (12 mL). The yellow solution was stirred overnight at rt and concentrated under reduced pressure. The crude residue was diluted with EtOAc, washed with saturated sodium bicarbonate solution and brine, and dried with magnesium sulfate. The solvent was removed to give 591 mg (67%) of (S)-4-[2-(2-methoxymethyl-pyrrolidin-1-yl)-ethyl]-piperidine-1-carboxylic acid benzyl ester as a yellow oil. MS (ES+) *m*/*z* 361 (M+H).

### Step 4: Preparation of (S)-4-[2-(2-methoxymethyl-pyrrolidin-1-yl)-ethyl]-piperidine

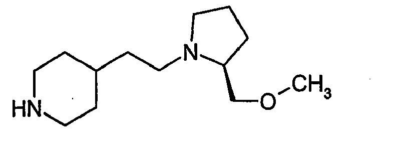

A solution of (S)-4-[2-(2-methoxymethyl-pyrrolidin-1-yl)-ethyl]-piperidine-1-carboxylic acid benzyl ester from Step 3 (592 mg, 1.64 mmol), palladium (10% on carbon)(85 mg, 0.08 mmol) in MeOH (5 mL) and EtOH (2 mL) was stirred at rt under an atmosphere of hydrogen. The reaction mixture was filtered and the filtrate concentrated *in vacuo* to give 372 mg (quantitative yeild) of (S)-4-[2-(2-methoxymethyl-pyrrolidin-1-yl)-ethyl]-piperidine as a light orange oil. MS (ES+) m/z 227(M+H).

Step 5: A solution of (S)-4-[2-(2-methoxymethyl-pyrrolidin-1-yl)-ethyl]-piperidine from step 4 (196 mg, 0.867 mmol) and 2-chloro-6,7-dimethoxy-5-methylquinazolin-4-one (220 mg, 0.866 mmol) in methoxyethanol (3 mL) was heated at 80 °C overnight. The reaction mixture was concentrated and chromatographed (5% MeOH/DCM) to give 104 mg (27%) of Example C-1 above as a white solid. Mp = 83.1-110.9 °C; TLC R*f* 0.35 (10% EtOH/DCM); [α]²⁴_{D} -27.1 ° (C 5.18, MeOH); IR (KBr) vₘₐₓ 3437, 2928,1650,1586,1464 cm-1; ¹H NMR (DMSO-*d*₆) 1.2 (m, 2H), 1.5-1.9 (m, 8H), 2.0 (m, 1H), 2.6 (s, 3H), 2.7 (m, 2H), 2.9 (t, 2H, *J*= 12), 3.3 (s, 3H), 3.4 (m, 2H), 3.6 (s, 3H), 3.8 (s, 3H), 4.3 (d, 2H, *J* =12), 6.6 (s, 1H); MS (ES+) m/z 445 (M+H); Anal. (C₂₄H₃₆N₄O₄ 0.92 CH₂Cl₂) C: calcd, 57.26; found, 57.22; H: calcd, 7.30; found, 7.31; N: calcd, 10.72; found, 10.93.

### EXAMPLES C-2 to C-12

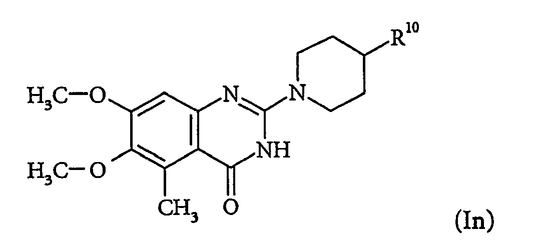

Compounds having the above formula (In), wherein R¹⁰ has the values reported in Table 3 were prepared following the same or similar method as described e.g. C-1, except a differently-substituted piperidinyl compound was used in place of 1-(2-methoxymethyl-pyrrolidin-1-yl)-2-(piperidin-4-yl)ethyl.

**TABLE 3**

| Ex. | R¹⁰ | MW |
|---|---|---|
| C-2 | 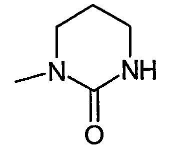 | 401.46 |
| C-3 | 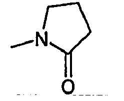 | 386.45 |
| C-4 | 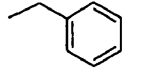 | 393.48 |
| C-5 | 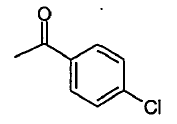 | 441.91 |
| C-6 | 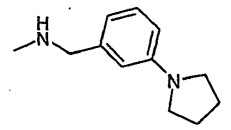 | 477.61 |
| C-7 | 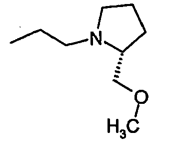 | 444.57 |
| C-8 | 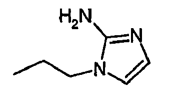 | 412.49 |
| C-9 | 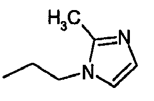 | 413.52 |
| C-10 | 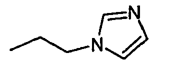 | 397.48 |
| C-11 | 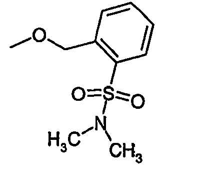 | 516.61 |
| C-12 | 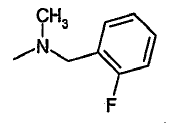 | 440.52 |

Additional compounds prepared according to the procedure of Example C-1 are shown in Table 9.

### EXAMPLES D-1 to D-20

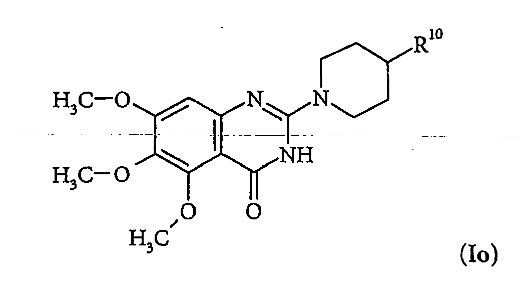

Compounds having the above formula (Io), wherein R¹⁰ has the values reported in Table 4 were prepared following the same or similar method as described e.g. C-1, except 2-chloro-6,7-dimethoxy-5-methylquinazolin-4-one was replaced with 2-chloro-5,6,7-trimethoxyquinazolin-4-one, and an appropriately-substituted piperidinyl compound was used.

**TABLE 4**

| Ex. | R¹⁰ | MW |
|---|---|---|
| D-1 | 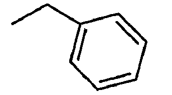 | 409.48 |
| D-2 | 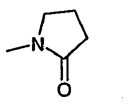 | 402.45 |
| D-3 | 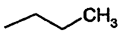 | 361.44 |
| D-4 | 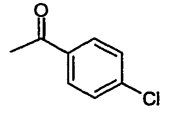 | 457.91 |
| D-5 | 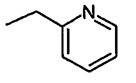 | 410.47 |
| D-6 | 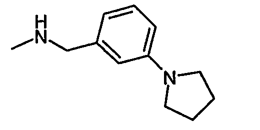 | 493.60 |
| D-7 | 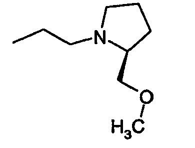 | 460.57 |
| D-8 | 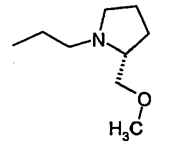 | 460.57 |
| D-9 | 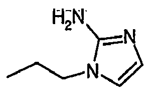 | 428.49 |
| D-10 | 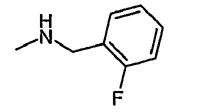 | 442.49 |
| D-11 | 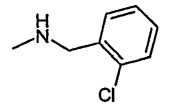 | 458.94 |
| D-12 | 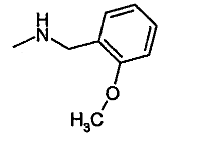 | 454.52 |
| D-13 | 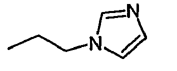 | 413.48 |
| D-14 | 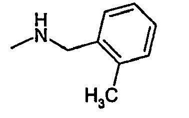 | 438.52 |
| D-15 | 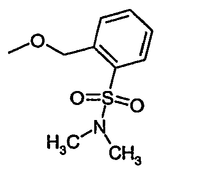 | 532.61 |
| D-16 | 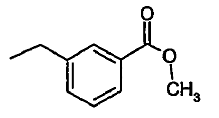 | 467.51 |
| D-17 | 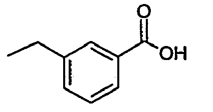 | 453.49 |
| D-18 | 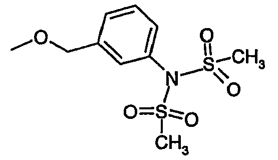 | 596.68 |
| D-19 | 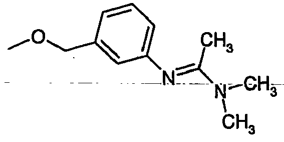 | 509.60 |
| D-20 | 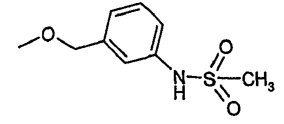 | 518.59 |

### EXAMPLES E-1 to E-15

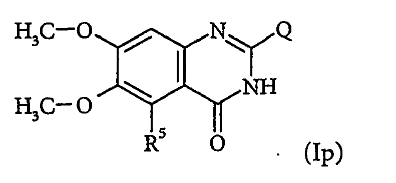

Compounds having the above formula (Ip), wherein R⁵ and Q have the values reported in Table 5 were prepared following the same or similar methods described above.

**TABLE 5**

| Ex. | R⁵ | Q | MW |
|---|---|---|---|
| E-1 | -CH₃ | 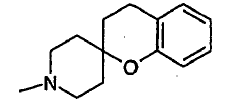 | 421.49 |
| E-2 | -OCH₃ | 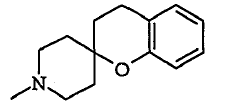 | 437.49 |
| E-3 | -CH₃ | 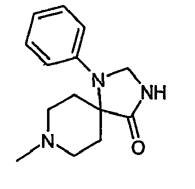 | 449.51 |
| E-4 | -CH₃ | 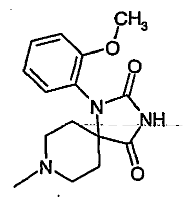 | 493.52 |
| E-5 | -CH₃ | 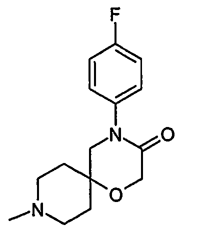 | 482.51 |
| E-6 | -OCH₃ | 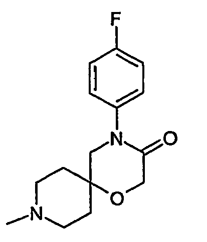 | 498.51 |
| E-7 | -OCH₃ | 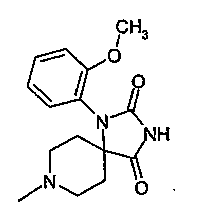 | 509.52 |
| E-8 | -OCH₃ | 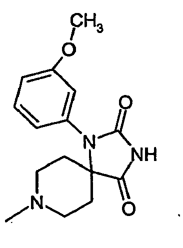 | 509.52 |
| E-9 | -OCH₃ | 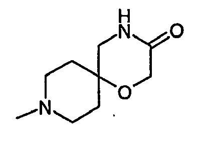 | 404.42 |
| E-10 | -OCH₃ | 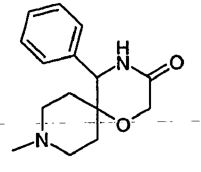 | 480.542 |
| E-11 | -OCH₃ | 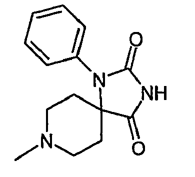 | 465.51 |
| E-12 | -CH₃ | 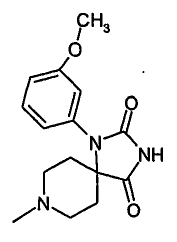 | 493.52 |
| E-13 | -CH₃ | 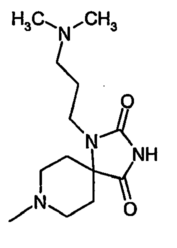 | 472.54 |
| E-14 | -CH₃ | 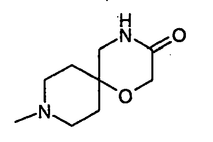 | 388.42 |
| E-15 | -CH₃ | 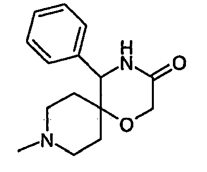 | 464.52 |

### Example F-1: N'-[2-(6,7-Dimethoxy-5-methyl-4-oxo-1,4-dihydro-quinazolin-2-yl)-1,2,3,4-tetrahydro-isoquinolin-5-yl]-N,N-dimethyl-acetamidine

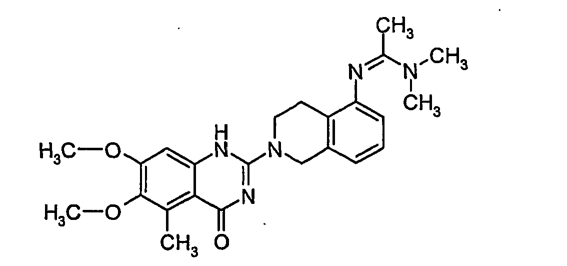

### Step 1: Preparation of N'-isoquinolin-5-yl-N,N-dimethyl-acetamidine

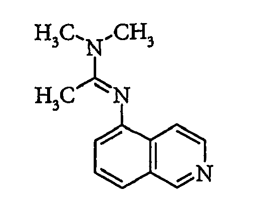

5-Aminoisoquinoline (1.00 g, 6.926 mmol) and *N,N*-dimethylacetamide dimethylacetal (4.00 g) were heated at 80 °C for 16 h. The excess *N,N*-dimethylacetamide. dimethylacetal was evaporated under reduced pressure. The crude *N*'-isoquinolin-5-yl-*N,N*-dimethyl-acetamidine was a brown oil which was taken to the next step.

### Step 2: Preparation of N,N-dimethyl-N'-(1,2,3,4-tetrahydro-isoquinolin-5-yl)-acetamidine

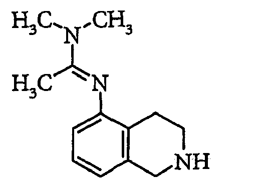

Sodium cyanoborohydride (2.6 g, 41.56 mmol) was added to a solution of *N'-*isoquinolin-5-yl-*N,N*-dimethyl-acetamidine from Step 1 (1.51 g, 6.926 mmol) in 10% HCl in MeOH (10ml) at 0 °C. The ice-bath was removed and the mixture stirred at rt for 1 h. DCM (30ml) was added, the white solid was filtered off, and the filtrate was concentrated to dryness. Purification by flash chromatography (CH₂Cl₂:MeOH:NH₄OH/300:10:1) gave 1.49g (ca. 100%) of a brown solid, *N,N-*dimethyl-*N*'-(1,2,3,4-tetrahydro-isoquinolin-5-yl)-acetamidine.

Step 3 : 2-Chloro-6,7-dimethoxy-5-methyl-1H-quinazolin-4-one (0.300g,1.178 mmol) and the solid (2) from Step 2 (0.269 g, 1.239 mmol) in methoxyethanol (10 ml) were heated at 80°C for 18 h. The solvent was evaporated to dryness under reduced pressure. Purification by flash chromatography (CH₂Cl₂:MeOH:NH₄OH/120:10:1) followed by recrystallization (CH₂Cl₂) gave 0.241 g (47%) of the above Example F-1 as a tan solid. Mp = 270.9-273.5 °C; ¹H NMR (DMSO-*d₆*, 2.49) δ1.74 (s, 3 H), 2.54 (t, 2 H), 2.60 (s, 3H), 2.97 (s, 6 H), 3.62 (s, 3 H), 3.75 (t, 2H), 3.86 (2, 3H), 4.72 (s, 2H), 6.40 (d, 1H), 6.67 (s,1H), 6.75 (d, 2H), 7.05 (t, 1H), 10.99 (s, 1H); IR (KBr) νₘₐₓ 1575 cm⁻¹; MS (ES+) *m*/*z* 436(M + H); Anal. (C₂₄H₂₉N₅O₃•0.8H₂O) C: calcd, 59.16; found, 59.07; H: calcd, 6.13; found, 6.06; N: calcd, 13.91; found, 13.93.

Example F-2:2-[5-(1,3-Dimethyl-imidazolidin-2-ylideneamino)-3,4-dihydro-1*H-*isoquinolin-2-yl]-6,7-dimethoxy-5-methyl-1*H*-quinazolin-4-one

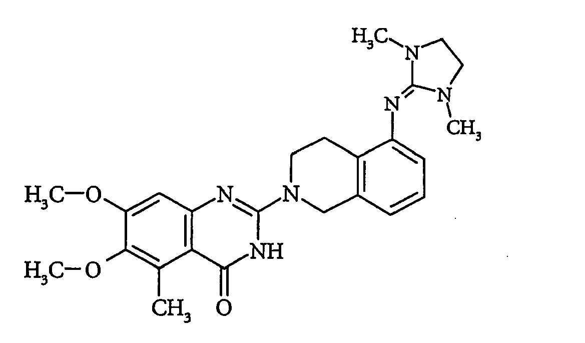

### Step 1: Preparation of (1,3-dimethylimidazolidin-2-ylidene)isoquinolin-5-ylamine

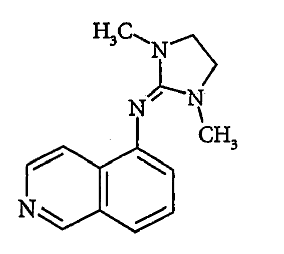

A mixture of 5-aminoisoquinoline (0.3 g, 2.09 mmol) and 2-chloro-1,3-dimethylimidazolidinium hexafluorophosphate (0.7 g, 2.51 mmol) in 15 mL of DCE, was heated to 60°C for 72 hours. The crude was purified by flash column eluting with -CH₂Cl₂:MeOH:NH₄OH (94.5:5:0.5) to afford 0.25 g (50%) of the above titled compound as a dark foam. ¹H NMR (CD₃OD 3.31) δ 2.74 (s, 6 H), 3.79 (s, 4 H), 7.75 (d, 1H, *J=* 1.17), 7.77 (s, 1 H), 8.01 (dt, 1H, *J* = 6.03, 0.96), 8.15 (m, 1H), 8.59 (d, 1H, *J* = 6.03), 9.35 (d, 1H, *J* = 0.99); MS (ES+) *m*/*z* 241.2 (M + H).

### Step 2: Preparation of (1,3-dimethylimidazolidin-2-ylidene)(1,2,3,4-tetrahydroisoquinolin-5-yl)amine.

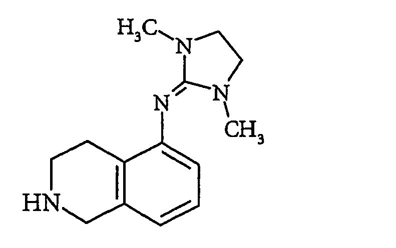

To a solution of (1,3-dimethylimidazolidin-2-ylidene)isoquinolin-5-ylamine from Step 1 (0.25 g, 1.04 mmol) in 10 mL of 10% HCl in MeOH, was added NaBH₃CN (0.4 g, 6.24 mmol), portion-wise over one hour while maintaining the pH acidic by adding 10% HCl in MeOH as required. After the addition was complete, stirring was continued at rt for 18 hours. Solid NaOH was slowly added until pH=10-12, and the insoluble solids removed by filtration. The filtrate was concentrated, dissolved again in 5% MeOH/CH₂Cl₂ and filtered to remove the insoluble materials. Then it was evaporated and purified by flash chromatography eluting with CH₂Cl₂:MeOH:NH₄OH (94.5:5:0.5) to yield the above-titled compound (0.075 g, 29.5%). ¹H NMR (CDCl₃ 7.26) δ 2.58 (t, 2 H, *J* = 5.97), 2.61 (s, 6 H), 2.82 (br s, D₂O, 1H), 3.11 (t, 2 H, *J*= 6.03), 3.28 (s, 4 H), 3.96 (s, 2 H), 5.58 (dt, 1H, *J*= 7.77, 1.17), 6.70 (dt, 1H, *J*= 7.77, 1.17), 6.97 (t, 1H, *J*= 7.65); MS (ES+) *m*/*z* 245.2 (M + H).

### Step 3: 2-[5-(1,3-Dimethylimidazolidin-2-ylideneamino)-3,4-dihydro-1H-isoquinolin-2-yl]-6,7-dimethoxy-5-methyl-1H-quinazolin-4-one (Example F-2).

In a screw capped test tube, a mixture of (1,3-dimethylimidazolidin-2-ylidene) (1,2,3,4-tetrahydroisoquinolin-5-yl)amine (13 mg, 0.052 mmol), 2-chloro-6,7-dimethoxy-5-methyl-1*H*-quinazolin-4-one (13 mg, 0.050 mmol) and TEA (0.014 mL, 0.1 mmol) in 1 mL of DMSO, was heated to 80 °C for 18 hours. The crude mixture was purified by LCMS to afford the above titled compound, Example F-2. MS (ES+) *m*/*z* 463.3 (M + H).

### EXAMPLES F-3 to F-37

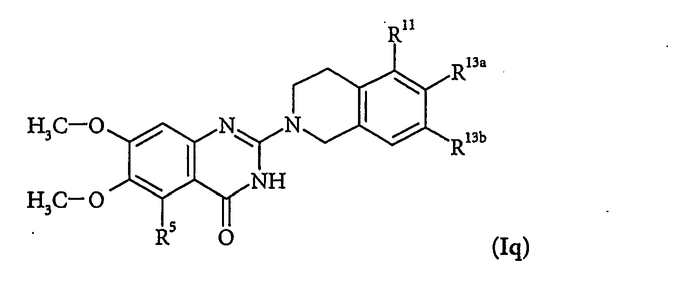

Compounds having the above formula (Iq), wherein R⁵, R¹¹, R^{13a}, and R^{13b} have the values reported in Table 6, were prepared following the same or similar method as described for Example F-1.

**TABLE 6**

| Ex. | R⁵ | R¹¹ | R^{13a} | R^{13b} | MW |
|---|---|---|---|---|---|
| F-3 | -OCH₃ | 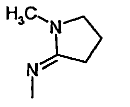 | -H | -H | 463.53 |
| F-4 | -OCH₃ | 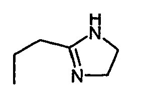 | -H | -H | 463.53 |
| F-5 | -OCH₃ | 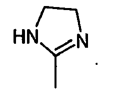 | -H | -H | 435.48 |
| F-6 | -OCH₃ | -H | -H | 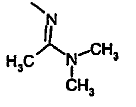 | 451.52 |
| F-7 | -OCH₃ | -H | -OCH₃ | -OCH₃ | 427.46 |
| F-8 | -OCH₂CH₂OCH₃ | -H | -OCH₃ | -OCH₃ | 471.51 |
| F-9 | -OCH₃ | 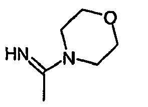 | -H | -H | 479.53 |
| F-10 | 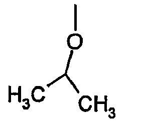 | -H | -OCH₃ | -OCH₃ | 455.51 |
| F-11 | 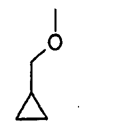 | -H | -OCH₃ | -OCH₃ | 467.52 |
| F-12 | 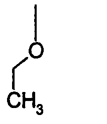 | -H | -OCH₃ | -CH₃ | 441.48 |
| F-13 | -OH | -H | -OCH₃ | -OCH₃ | 413.43 |
| F-14 | -OCH₂CH₂OH | -H | -OCH₃ | -OCH₃ | 457.48 |
| F-15 | 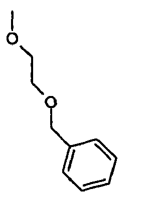 | -H | -OCH₃ | -OCH₃ | 547.61 |
| F-16 | 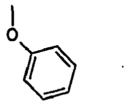 | -H | -OCH₃ | -OCH₃ | 489.53 |
| F-17 | -OCH₂CH₂NH₂ | -H | -OCH₃ | -OCH₃ | 456.50 |
| F-18 | -OCH₃ | 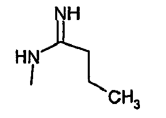 | -H | -H | 451.52 |
| F-19 | -OCH₃ | 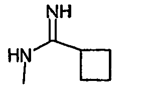 | -H | -H | 463.53 |
| F-20 | -OCH₃ | 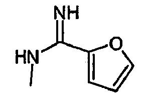 | -H | -H | 475.50 |
| F-21 | -OCH₃ | 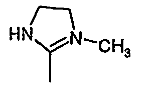 | -H | -H | 449.51 |
| F-22 | 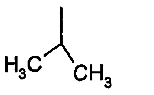 | -H | -OCH₃ | -OCH₃ | 439.51 |
| F-23 | 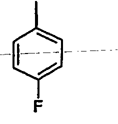 | -H | -OCH₃ | -OCH₃ | 491.52 |
| F-24 | -CH₃ | -H | -OCH₃ | -OCH₃ | 411.46 |
| F-25 | -OCH₃ | 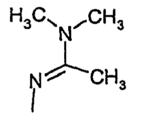 | -H | -H | 451.52 |
| F-26 | -CH₃ | 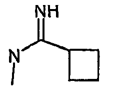 | -H | -H | 447.54 |
| F-27 | -CH₃ | 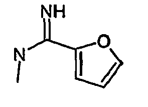 | -H | -H | 459.50 |
| F-28 | -CH₃ | 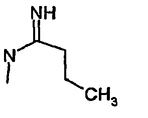 | -H | -H | 435.53 |
| F-29 | -CH₃ | 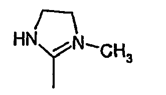 | -H | -H | 433.51 |
| F-30 | -CH₃ | 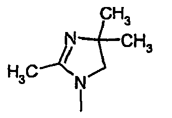 | -H | -H | 461.56 |
| F-31 | -CH₃ | 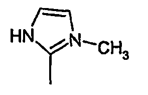 | -H | -H | 431.49 |
| F-32 | -CH₃ | 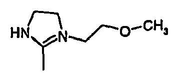 | -H | -H | 477.56 |
| F-33 | -CH₃ | 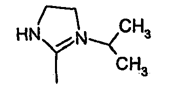 | -H | -H | 461.56 |
| F-34 | -CH₃ | 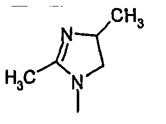 | -H | -H | 447.54 |
| F-35 | -CH₃ | -H | -H | 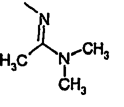 | 435.53 |
| F-36 | -F | -H | -OCH₃ | -OCH₃ | 415.42 |
| F-37 | -Cl | -H | -OCH₃ | -OCH₃ | 431.87 |

Additional compounds prepared according to the procedure of Example A-1 are shown in Table 9.

### Example G-1: 5,6,7-Trimethoxy-2-(4-morpholin-4-yl-5,8-dihydro-6H-pyrido[3,4-d]pyrimidin-7-yl)-1H-quinazolin-4-one

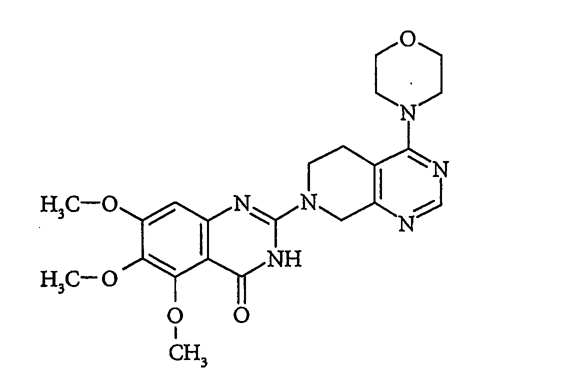

To a sample of 707 mg (2.5 mmol) of 2-methylthiol-5,6,7-trimethoxy-1*H*-quinazolin-4-one in 100 mL of DCE was added 1.3 g of 75% MCPBA (about 5.5 mmol). The mixture was stirred for 2-3 hrs, and then it was treated with 790 mg of 4-morpholin-4-yl-5,8-dihydro-6H-pyrido[3,4-d]pyrimidine 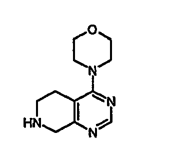 as the dihydrochloride salt (2.7 mmol) and 1.1 g (11 mmol) of TEA. The resulting mixture was stirred at ambient temperature for 4 days and then for an additional 3 h at 80 °C. The solvent was removed and the resulting solid was stirred with EtOAc, collected, stirred with diluted ammonium hydroxide, and extracted with EtOAc again. A hydrochloride salt was prepared as described in WO 02/053558 A1 to furnish 190 mg (13%) of Example G-1. Mp 190-192 °C; ms 455 (M+H).

### Example G-2: 6,7-Dimethoxy-5-methyl-2-(4-morpholin-4-yl-5,8-dihydro-6H-pyrido[3,4-d]pyrimidin-7-yl)-1H-quinazolin-4-one

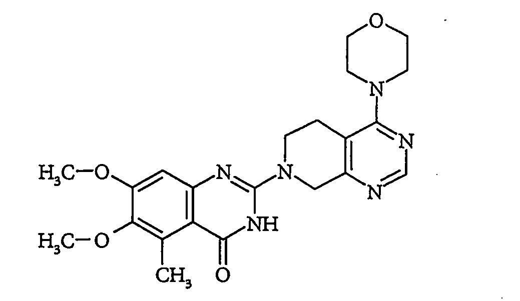

The above compound was prepared in the same fashion as Example G-1, except 6,7-dimethoxy-2-methylthiol-5-methyl-1*H*-quinazolin-4-one was used in place of 2-methylthiol-5,6,7-trimethoxy-1*H*-quinazolin-4-one. MW = 438.48

### Example H-1: 6,7-Dimethoxy-5-methyl-2-[4-(morpholine-4-carbonyl)-[1,4]diazepan-1-yl]-1H-quinazolin-4-one

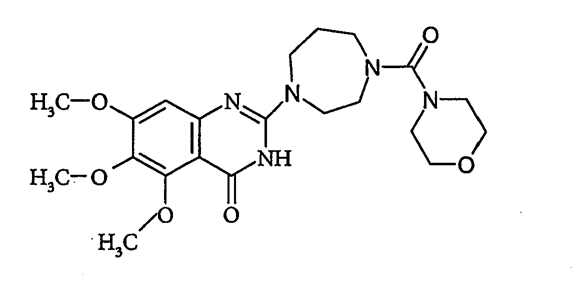

2-Chloro-6,7-dimethoxy-5-methyl-1*H*-quinazolin-4-one (254 mg, 1.0 mmol) and [1,4]diazepan-1-yl-morpholin-4-yl-methanone (256 mg, 1.2 mmol) were combined in 4 mL EtOH in a sealed tube and heated in an oil bath at 105°C for 2.5 hrs. The mixture was cooled in an ice bath. The product was filtered, washed with a little cold EtOH and dried to afford 319 mg (74%) as an off-white solid. Mp 236.6-237.7 °C; MS (ES+) m/z 432 (M + H); IR (KBr) νₘₐₓ 1651, 1588, 1477, 1465, 1410; ¹H NMR (DMSO-*d₆*, 2:50) δ 1.86 (m, 2 H), 2.59 (s, 3 H), 2.95 (t, 2 H, *J* = 4.50), 3.28 (t, 2 H, *J*= 5.5), 3.51 (m, 4 H), 3.61 (s, 3 H), 3.68 (t, 2 H, *J*= 5.8), 3.80 (t, 2 H, *J*= 5.4), 3.85 (s 3 H), 6.60 (s, 1 H). 10.71 (s, 1 H); ¹³C (DMSO-*d₆*, 39.89) δ 163.87,157.59,142.81,131.88,104.73,66.22,60.22, 55.90, 48.15, 47.93, 47.80, 46.97, 46.90, 26.98, 13.88; Anal. (C₂₁H₂₉N₅O₅.HCl) C: calcd. 53.90; found 53.90; H: calcd. 6.46; found 6.10; N: calcd. 14.97; found 14.87 The [1,4]diazepan-1-yl-morpholin-4-yl-methanone intermediate was synthesized as described in EP 9605609.

### EXAMPLES H-2 to H-7

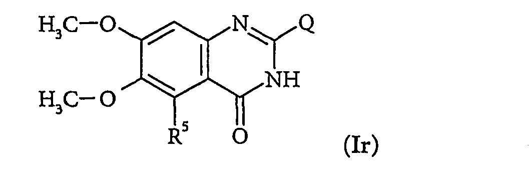

Compounds having the above formula (Ir), wherein R⁵ and Q have the values reported in Table 8, were prepared following the same or similar methods as described for examples detailed above.

**TABLE 8**

| Ex. | R⁵ | Q | MW (M+H⁺) |
|---|---|---|---|
| H-2 | -OCH₃ | 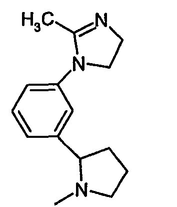 | 463.54 |
| H-3 | -OCH₃ | 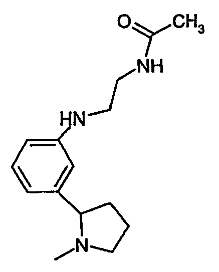 | 481.55 |
| H-4 | -OCH₃ | 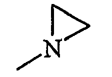 | 277.28 |
| H-5 | -CH₃ | 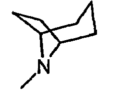 | 329.40 |
| H-6 | -CH₃ | 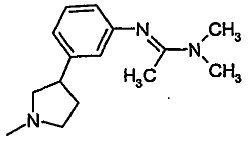 | 449.55 |
| H-7 -OCH₃ | | 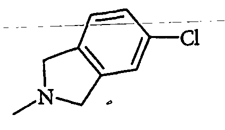 | 387.82 |

Representative compounds in accordance with the invention are shown in Table 9.

| # | Systematic Name | Melting Point | MS (M+H) |
|---|---|---|---|
| 1 | 2-{7-[(Ethyl-methyl-amino)-methyl]-3,4-dihydro-1*H*-isoquinolin-2-yl}-5,6,7-trimethoxy-1*H* quinazolin-4-one | 161.4-163.9 | |
| 2 | 2-{7-[(Ethyl-methyl-amino)-methyl]-3,4-dihydro-1*H*-isoquinolin-2-yl}-6,7-dimethoxy-5-methyl-1*H*-quinazolin-4-one | 189.4-204.6 | |
| 3 | 2-{5-[(Ethyl-methyl-amino)-methyl]-3,4-dihydro-1*H*-isoquinolin-2-yl}-5,6,7-trimethoxy-1*H* quinazolin-4-one | 214.0-216.9 | |
| 4 | 5,6,7-Trimethoxy-2-(7-methylaminomethyl-3,4-dihydro-1*H*-isoquinolin-2-yl)-1*H*-quinazolin-4-one | 178.1-180.9 | |
| 5 | 2-[5-(1,3-Dimethyl-imidazolidin-2-ylideneamino)-3,4-dihydro-1*H*-isoquinolin-2-yl]-5,6,7-trimethoxy-1*H*-quinazolin-4-one | 215-217.5 | |
| 6 | 6,7-Dimethoxy-5-methyl-2-(5-methylaminomethyl-3,4-dihydro-1*H*-isoquinolin-2-yl)-1*H*-quinazolin-4-one | 241.9-245.5 | |
| 7 | 6,7-Dichloro-2-(6,7-dimethoxy-3,4-dihydro-1*H* isoquinolin-2-yl)-1*H*-quinolin-4-one | 266-271 | |
| 8 | *N*-[2-(6,7-Dimethoxy-5-methyl-4-oxo-1,4-dihydro-quinazolin-2-yl)-1,2,3,4-tetrahydro-isoquinolin-5-ylmethyl]-*N*-methyl-2-methylamino-acetamide | 208.8-212.0 | |
| 9 | 5-Isopropyl-6,7-dimethoxy-2-{5-[1-methyl-pyrrolidin-(2E)-ylideneamino]-3,4-dihydro-1*H*-isoquinolin-2-yl}-1*H*-quinazolin-4-one | 196-200 | |
| 10 | 5,6,7-Trimethoxy-2-[4-(4-methyl-pentanoyl)-piperazin-1-yl]-1*H*-quinazolin-4-one hydrochloride | 198-201 | |
| 11 | 2-[4-(Furan-2-carbonyl)-piperazin-1-yl]-5,6,7 trimethoxy-1*H*-quinazolin-4-one | | 415.5 |
| 12 | 4-(5,6,7-Trimethoxy-4-oxo-1,4-dihydro-quinazolin-2-yl)-piperazine-1-carboxylic acid ethyl ester | | 393.4 |
| 13 | [4-(5,6,7-Trimethoxy-4-oxo-1,4-dihydro-quinazolin-2-yl)-piperazin-1-yl]-acetic acid ethyl ester | | 407.4 |
| 14 | 5,6,7-Trimethoxy-2-[4-(3-triffuoromethyl-phenyl)-piperazin-1-yl]-1H-quinazolin-4-one | | 465.4 |
| 15 | *N*,*N*-Dimethyl-2-[4-(5,6,7-trimethoxy-4-oxo-1,4-dihydro-quinazolin-2-yl)-piperazin-1-yl] -acetamide | | 406.4 |
| 16 | 4-(5,6,7-Trimethoxy-4-oxo-1,4-dihydro-quinazolin-2-yl)-piperazine-1-carboxylic acid (3-chloro-phenyl)-amide | | 474.4 |
| 17 | 2-[4-(2-Cyclopentyl-acetyl)-piperazin-1-yl]-5,6,7-trimethoxy-1*H*-quinazolin-4-one | | 431.4 |
| 18 | 5,6,7-Trimethoxy-2-[4-(3-methyl-butyryl)-piperazin-1-yl]-1*H*-quinazolin-4-one | | 405.4 |
| 19 | 4-(5,6,7-Trimethoxy-4-oxo-1,4-dihydro-quinazolin-2-yl)-piperazine-1-carboxylic acid (3-fluoro-phenyl)-amide | | 458.3 |
| 20 | 4-(5,6,7-Trimethoxy-4-oxo-1,4-dihydro-quinazolin-2-yl)-piperazine-1-carboxylic acid (3,4-difluoro-phenyl)-amide | | 476.3 |
| 21 | 4-(5,6,7-Trimethoxy-4-oxo-1,4-dihydro-quinazolin-2-yl)-piperazine-1-carboxylic acid (3-cyano-phenyl)-amide | | 465.3 |
| 22 | 5,6,7-Trimethoxy-2-(4-pyridin-2-yl-piperazin-1-yl)-1*H*-quinazolin-4-one | | 398 |
| 23 | 2-[4-(1*H*-Imidazol-2-yl)-piperazin-1-yl]-5,6,7-trimethoxy-1*H*-quinazolin-4-one | | 387 |
| 24 | 5,6,7-Trimethoxy-2-[4-(6-methyl-pyridin-2-yl)-piperazin-1-yl]-1*H*-quinazolin-4-one | 231.1-232.2 | |
| 25 | 5,6,7-Trimethoxy-2-[4-(4-methyl-pyridin-2-yl)-piperazin-1-yl]-1*H* quinazolin-4-one | 235.3-235.5 | |
| 26 | 5,6,7-Trimethoxy-2-[4-(1-oxy-pyridin-2-yl)-piperazin-1-yl]-1*H*-quinazolin-4-one | 142.8-147.2 | |
| 27 | 5,6,7-Trimethoxy-2-[4-(1-methyl-4,5-dihydro-1*H*-imidazol-2-yl)-piperazin-1-yl]-1*H*-quinazolin-4-one | 181.6-188.8 (HCl Salt) | |
| 28 | 5,6,7-Trimethoxy-2-[4-(1-methyl-1*H*-imidazol-2-yl)-piperazin-1-yl] -1*H*-quinazolin-4-one | 227-229 | |
| 29 | 5,6,7-Trimethoxy-2-[4-(1,4,5-trimethyl-1*H*-imidazol-2-yl)-piperazin-1-yl] -1*H*-quinazolin-4-one | | 429.2 |
| 30 | 5,6,7-Trimethoxy-2-[4-(4-methoxy-pyridin-2-yl)-piperazin-1-yl]-1*H*-quinazolin-4-one | 229-231 | |
| 31 | 4-(6,7-Dimethoxy-5-methyl-4-oxo-1,4-dihydro-quinazolin-2-yl)-piperazine-1-carboxylic acid ethyl ester | | 377.4 |
| 32 | [4-(6,7-Dimethoxy-5-methyl-4-oxo-1,4-dihydro-quinazolin-2-yl)-piperazin-1-yl]-acetic acid ethyl ester | | 391.4 |
| 33 | 2-[4-(Furan-2-carbonyl)-piperazin-1-yl]-6,7-dimethoxy-5-methyl-1*H*-quinazolin-4-one | | 399.4 |
| 34 | 6,7-Dimethoxy-5-methyl-2-[4-(3-trifluoromethyl-phenyl)-piperazin-1-yl]-1*H*-quinazolin-4-one | | 449.4 |
| 35 | 6,7-Dimethoxy-5-methyl-2-[4-(4-methyl-pentanoyl)-piperazin-1-yl]-1*H*-quinazolin-4-one | | 403.5 |
| 36 | 4-(6,7-Dimethoxy-5-methyl-4-oxo-1,4-dihydro-quinazolin-2-yl)-piperazine-1-carboxylic acid (3-chloro-phenyl)-amide | | 458.4 |
| 37 | 2-[4-(2-Cyclopentyl-acetyl)-piperazin-1-yl]-6,7-dimethoxy-5-methyl-1*H*-quinazolin-4-one | | 415.5 |
| 38 | 6,7-Dimethoxy-5-methyl-2-[4-(3-methyl-butyryl)-piperazin-1-yl]-1*H*-quinazolin-4-one | | 389.4 |
| 39 | 2-[4-(4-Chloro-3-trifluoromethyl-phenyl)-piperazin-1-yl] -6,7-dimethoxy-5-methyl-1*H*-quinazolin-4-one | | 483.3 |
| 40 | 6,7-Dimethoxy-5-methyl-2-(4-pyridin-2-yl-piperazin-1-yl)-1*H*-quinazolin-4-one | 286.6-293.1 (HCl Salt) | |
| 41 | 2-[4-(1*H*-Imidazol-2-yl)-piperazin-1-yl]-6,7-dimethoxy-5-methyl-1*H*-quinazolin-4-one | | 371 |
| 42 | 6,7-Dimethoxy-5-methyl-2-[4-(6-methyl-pyridin-2-yl)-piperazin-1-yl]-1*H*-quinazolin-4-one | 284.5-285.5 | |
| 43 | 6,7-Dimethoxy-5-methyl-2-[4-(4-methyl-pyridin-2-yl)-piperazin-1-yl]-1*H*-quinazolin-4-one | 277.7-280(HCl Salt) | |
| 44 | 2-{4-[1-(3-Fluoro-phenyl)-4,5-dihydro-1*H*-imidazol-2-yl]-piperazin-1-yl}-6,7-dimethoxy-5-methyl-1*H*-quinazolin-4-one | 257.9-260 | |
| 45 | 2-[4-(1-Isopropyl-4,5-dihydro-1*H*-imidazol-2-ylmethyl)-piperazin-1-yl]-6,7-dimethoxy-5-methyl-1*H*-quinazolin-4-one | | 429 |
| 46 | 6,7-Dimethoxy-5-methyl-2-[4-(1-methyl-4,5-dihydro-1*H*-imidazol-2-ylmethyl)-piperazin-1-yl]-1*H*-quinazolin-4-one | | 401.2 |
| 47 | 6,7-Dimethoxy-5-methyl-2-[4-(1-methyl-1*H*-imidazol-2-yl)-piperazin-1-yl] -1*H*-quinazolin-4-one | 263-264 | |
| 48 | 6,7-Dimethoxy-5-methyl-2-[4-(1,4,5-trimethyl-1*H*-imidazol-2-yl)-piperazin-1-yl]-1*H*-quinazolin-4-one | | 413 |
| 49 | 6,7-Dimethoxy-2-[4-(4-methoxy-pyridin-2-yl)-piperazin-1-yl]-5-methyl-1*H*-quinazolin-4-one | 273-274 | |
| 50 | 2-(3-Cyclohexyl-5,6-dihydro-8*H*-imidazo[1,5-*a*]-pyrazin-7-yl)-6,7-dimethoxy-5-methyl-1*H*-quinazolin-4-one; compound with trifluoro-acetic acid | | 424.4 |
| 51 | 6,7-Dimethoxy-2-{4-[2-((S)-2-methoxymethyl-pyrrolidin-1-yl)-ethyl]-piperidin-1-yl}-5-methyl-1*H*-quinazolin-4-one | 83.1-110.9 | 445 |
| 52 | 6,7-Dimethoxy-5-methyl-2-[4-(2-oxo-tetrahydro-pyrimidin-1-yl)-piperidin-1-yl]-1*H*-quinazolin-4-one | | 402.4 |
| 53 | 6,7-Dimethoxy-5-methyl-2-[4-(2-oxo-pyrrolidin-1-yl)-piperidin-1-yl]-1*H*-quinazolin-4-one | | 387.4 |
| 54 | 2-(4-Benzyl-piperidin-1-yl)-6,7-dimethoxy-5-methyl-1*H*-quinazolin-4-one | | 394.4 |
| 55 | 2-[4-(4-Chloro-benzoyl)-piperidin-1-yl]-6,7-dimethoxy-5-methyl-1*H*-quinazolin-4-one | | 442.3 |
| 56 | 6,7-Dimethoxy-5-methyl-2-[4-(3-pyrrolidin-1-yl-benzylamino)-piperidin-1-yl]-1*H*-quinazolin-4-one | | 478 |
| 57 | 6,7-Dimethoxy-2-{4-[2-((R)-2-methoxymethyl-pyrrolidin-1-yl)-ethyl]-piperidin-1-yl}-5-methyl-1*H*-quinazolin-4-one | 219.0-221.9 | |
| 58 | 2-{4-[2-(2-Amino-imidazol-1-yl)-ethyl]-piperidin-1-yl}-6,7-dimethoxy-5-methyl-1*H*-quinazolin-4-one | 130.9-133.3 | |
| 59 | 6,7-Dimethoxy-5-methyl-2-{4-[2-(2-methyl-4,5-dihydro-imidazol-1-yl)-ethyl]-piperidin-1-yl}-1*H*-quinazolin-4-one | 266.1-269.5 | |
| 60 | 2-[4-(2-Imidazol-1-yl-ethyl)-piperidin-1-yl]-6,7-dimethoxy-5-methyl-1*H*-quinazolin-4-one | | 398.1 |
| 61 | 2-[1-(6,7-Dimethoxy-5-methyl-4-oxo-1,4-dihydro-quinazolin-2-yl)-piperidin-4-yloxymethyl] -N,N dimethyl-benzenesulfonamide | 217.9-219.8 | |
| 62 | 2-{4-[(2-Fluoro-benzyl)-methyl-amino]-piperidin-1-yl}-6,7-dimethoxy-5-methyl-1*H*-quinazolin-4-one | | 441 |
| 63 | 2-(4-Benzyl-piperidin-1-yl)-5,6,7-trimethoxy-1*H* quinazolin-4-one | 212-215 | |
| 64 | 5,6,7-Trimethoxy-2-[4-(2-oxo-pyrrolidin-1-yl)-piperidin-1-yl]-1*H*-quinazolin-4-one | | 403.4 |
| 65 | 5,6,7-Trimethoxy-2-(4-propyl-piperidin-1-yl)-1*H*-quinazolin-4-one | | 362.4 |
| 66 | 2-[4-(4-Chloro-benzoyl)-piperidin-1-yl]-5,6,7-trimethoxy-1*H*-quinazolin-4-one | | 458.4 |
| 67 | 5,6,7-Trimethoxy-2-(4-pyridin-2-ylmethyl-piperidin-1-yl)-1*H*-quinazolin-4-one | | 411.1 |
| 68 | 5,6,7-Trimethoxy-2-[4-(3-pyrrolidin-1-yl-benzylamino)-piperidin-1-yl]-1*H*-quinazolin-4-one | | 494 |
| 69 | 5,6,7-Trimethoxy-2-{4-[2-((S)-2-methoxymethyl-pyrrolidin-1-yl)-ethyl]-piperidin-1-yl}-1H-quinazolin-4-one | 78.8-91.6 | |
| 70 | 5,6,7-Trimethoxy-2-{4-[2-((R)-2-methoxymethyl-pyrrolidin-1-yl)-ethyl]-piperidin-1-yl}-1*H* quinazolin-4-one | 65.0-79.1 | |
| 71 | 2-{4-[2-(2-Amino-imidazol-1-yl)-ethyl]-piperidin-1-yl}-5,6,7-trimethoxy-1*H*-quinazolin-4-one | 252.4-253.2 | |
| 72 | 2-[4-(2-Fluoro-benzylamino)-piperidin-1-yl]-5,6,7-trimethoxy-1*H*-quinazolin-4-one | | 443 |
| 73 | 2-[4-(2-Chloro-benzylamino)-piperidin-1-yl]-5,6,7-trimethoxy-1*H*-quinazolin-4-one | | 459 |
| 74 | 5,6,7-Trimethoxy-2-[4-(2-methoxy-benzylamino)-piperidin-1-yl]-1*H*-quinazolin-4-one | | 455 |
| 75 | 2-[4-(2-Imidazol-1-yl-ethyl)-piperidin-1-yl]-5,6,7-trimethoxy-1*H*-quinazolin-4-one | | 414.1 |
| 76 | 5,6,7-Trimethoxy-2-[4-(2-methyl-benzylamino)-piperidin-1-yl]-1*H*-quinazolin-4-one | | 439 |
| 77 | *N,N* Dimethyl-2-[1-(5,6,7-trimethoxy-4-oxo-1,4-dihydro-quinazolin-2-yl)-piperidin-4-yloxymethyl]-benzenesulfonamide | 186.0-188.2 | |
| 78 | 3-[1-(5,6,7-Trimethoxy-4-oxo-1,4-dihydro-quinazolin-2-yl)-piperidin-4-ylmethyl] -benzoic acid methyl ester | | 468.2 |
| 79 | 3-[1-(5,6,7-Trimethoxy-4-oxo-1,4-dihydro-quinazolin-2-yl)-piperidin-4-ylmethyl] -benzoic acid | 245-249 | |
| 80 | *N*-Methanesulfonyl-N-{3-[1-(5,6,7-trimethoxy-4-oxo-1,4-dihydro-quinazolin-2-yl)-piperidin-4-yloxymethyl] -phenyl}-methanesulfonamide | 100.7-160.4 | |
| 81 | *N,N*-Dimethyl-*N*'-{3-[1-(5,6,7-trimethoxy-4-oxo-1,4-dihydro-quinazolin-2-yl)-piperidin-4-yloxy-methyl] -phenyl}-acetamidine | 56.0-64.9 | |
| 82 | *N*-{3-[1-(5,6,7-Trimethoxy-4-oxo-1,4-dihydro-quinazolin-2-yl)-piperidin-4-yloxymethyl]-phenyl}-methanesulfonamide | 86.0-92.0 | |
| 83 | 2-(3,4-dihydro-1'H-spiro[chromene-2,4'-piperidin]-1'-yl)-6,7-dimethoxy-5-methylquinazolin-4(1*H*)-one | | 422.4 |
| 84 | 2-(3,4-dihydro-1'*H*-spiro[chromene-2,4'-piperidin]-1'-yl)-5,6,7-trimethoxyquinazolin-4(1*H*)-one | | 438.4 |
| 85 | 6,7-Dimethoxy-5-methyl-2-(4-oxo-1-phenyl-1,3,8-triaza-spiro[4.5]dec-8-yl)-1*H*-quinazolin-4-one | | 450.4 |
| 86 | 8-(6,7-Dimethoxy-5-methyl-4-oxo-1,4-dihydro-quinazolin-2-yl)-1-(2-methoxy-phenyl)-1,3,8-triaza-spiro [4.5] decane-2,4-dione; compound with triffuoro-acetic acid | | 494.4 |
| 87 | 9-(6,7-Dimethoxy-5-methyl-4-oxo-1,4-dihydro-quinazolin-2-yl)-4-(4-ffuoro-phenyl)-1-oxa-4,9-diaza-spiro [5.5] undecan-3-one | | 483.4 |
| 88 | 4-(4-Fluoro-phenyl)-9-(5,6,7-trimethoxy-4-oxo-1,4-dihydro-quinazolin-2-yl)-1-oxa-4,9-diazaspiro [5.5]undecan-3-one | | 499.4 |
| 89 | 1-(2-Methoxy-phenyl)-8-(5,6,7-trimethoxy-4-oxo-1,4-dihydro-quinazolin-2-yl)-1,3,8-triazaspiro [4.5] decane-2,4-dione | | 510.4 |
| 90 | 1-(3-Methoxy-phenyl)-8-(5,6,7-trimethoxy-4-oxo-1,4-dihydro-quinazolin-2-yl)-1,3,8-triazaspiro [4.5] decane-2,4-dione | | 510.5 |
| 91 | 9-(5,6,7-Trimethoxy-4-oxo-1,4-dihydro-quinazolin-2-yl)-1-oxa-4,9-diaza-spiro [5.5] undecan-3-one | | 405.4 |
| 92 | 5-Phenyl-9-(5,6,7-trimethoxy-4-oxo-1,4-dihydro-quinazolin-2-yl)-1-oxa-4,9-diaza-spiro [5.5] undecan-3-one | | 481.4 |
| 93 | 5,6,7-Trimethoxy-2-(4-oxo-1-phenyl-1,3,8-triaza-spiro[4.5]dec-8-yl)-1*H*-quinazolin-4-one | | 466.4 |
| 94 | 8-(6,7-Dimethoxy-5-methyl-4-oxo-1,4-dihydro-quinazolin-2-yl)-1-(3-methoxy-phenyl)-1,3,8-triaza-spiro [4.5] decane-2,4-dione | | 494.4 |
| 95 | 8-(6,7-Dimethoxy-5-methyl-4-oxo-1,4-dihydro-quinazolin-2-yl)-1-(3-dimethylamino-propyl)-1,3,8-triaza-spiro [4.5] decane-2,4-dione | | 473.5 |
| 96 | 9-(6,7-Dimethoxy-5-methyl-4-oxo-1,4-dihydro-quinazolin-2-yl)-1-oxa-4,9-diaza-spiro [5.5]un-decan-3-one | | 389.4 |
| 97 | 9-(6,7-Dimethoxy-5-methyl-4-oxo-1,4-dihydro-quinazolin-2-yl)-5-phenyl-1-oxa-4,9-diaza-spiro[5.5] undecan-3-one | | 465.4 |
| 98 | *N*'-[2-(6,7-Dimethoxy-5-methyl-4-oxo-1,4-dihydro-quinazolin-2-yl)-1,2,3,4-tetrahydro-isoquinolin-5-yl]-*N,N*-climethyl-acetamidine | 270.9-273.5 | 436 |
| 99 | 2-[5-(1,3-Dimethyl-imidazolidin-2-ylideneamino)-3,4-dihydro-1H-isoquinolin-2-yl] -6,7-dimethoxy-5-methyl-1*H*-quinazolin-4-one | | 463.3 |
| 100 | 5,6,7-Trimethoxy-2-{5-[1-methyl-pyrrolidin-(2E)-ylideneamino]-3,4-dihydro-1*H*-isoquinolin-2-yl}-1*H*-quinazolin-4-one | | 464.1 |
| 101 | 2-{5-[2-(4,5-Dihydro-1*H*-imidazol-2-yl)-ethyl]-3,4-dihydro-1*H*-isoquinolin-2-yl}-5,6,7-trimethoxy-1*H*quinazolin-4-one | | 464.1 |
| 102 | 2-[5-(4,5-Dihydro-1*H*-imidazol-2-yl)-3,4-dihydro-1*H*-isoquinolin-2-yl]-5,6,7-trimethoxy-1*H*-quinazolin-4-one | | 436 |
| 103 | *N,N*-Dimethyl-*N*'-[2-(5,6,7-trimethoxy-4-oxo-1,4-dihydro-quinazolin-2-yl)-1,2,3,4-tetrahydro-isoquinolin-7-yl] -acetamidine | 115.8-120.1 | |
| 104 | 2-(6,7-Dimethoxy-3,4-dihydro-1*H*-isoquinolin-2-yl)-5,6,7-trimethoxy-1*H*-quinazolin-4-one | 267-270 (HCl Salt) | |
| 105 | 2-(6,7-Dimethoxy-3,4-dihydro-1*H*-isoquinolin-2-yl)-6,7-dimethoxy-5-(2-methoxy-ethoxy)-1*H*-quinazolin-4-one | 202.4-204.4 | |
| 106 | 2-[5-(Imino-morpholin-4-yl-methyl)-3,4-dihydro-1*H*-isoquinolin-2-yl] -5,6,7-trimethoxy-1*H* quinazolin-4-one | | 480.1 |
| 107 | 2-(6,7-Dimethoxy-3,4-dihydro-1*H*-isoquinolin-2-yl)-5-isopropoxy-6,7-dimethoxy-1*H*-quinazolin-4-one | 253.3-261.9 (HCl Salt) | |
| 108 | 5-Cyclopropylmethoxy-2-(6,7-dimethoxy-3,4-dihydro-1*H*-isoquinolin-2-yl)-6,7-dimethoxy-1*H* quinazolin-4-one | 214-215.8 | |
| 109 | 2-(6,7-Dimethoxy-3,4-dihydro-1*H*-isoquinolin-2-yl)-5-ethoxy-6,7-dimethoxy-1*H*-quinazolin-4-one | 218.5-221 (HCl Salt) | |
| 110 | 2-(6,7-Dimethoxy-3,4-dihydro-1*H*-isoquinolin-2-yl)-5-hydroxy-6,7-dimethoxy-1*H*-quinazolin-4-one | 255-259 (HCl Salt) | |
| 111 | 2-(6,7-Dimethoxy-3,4-dihydro-1*H*-isoquinolin-2-yl)-5-(2-hydroxy-ethoxy)-6,7-dimethoxy-1*H* quinazolin-4-one | 224-229 | |
| 112 | 5-(2-Benzyloxy-ethoxy)-2-(6,7-dimethoxy-3,4-dihydro-1*H*-isoquinolin-2-yl)-6,7-dimethoxy-1*H*-quinazolin-4-one | 197.0-201.9 | |
| 113 | 2-(6,7-Dimethoxy-3,4-dihydro-1*H*-isoquinolin-2-yl)-6,7-dimethoxy-5-phenoxy-1*H*-quinazolin-4-one | 238.7-241.9 | |
| 114 | 5-(2-Amino-ethoxy)-2-(6,7-dimethoxy-3,4-dihydro-1*H*-isoquinolin-2-yl)-6,7-dimethoxy-1*H*-quinazolin-4-one | | 457.1 |
| 115 | *N*-[2-(5,6,7-Trimethoxy-4-oxo-1,4-dihydro-quinazolin-2-yl)-1,2,3,4-tetrahydro-isoquinolin-5-yl]-butyramidine | 192.2-193.5 | |
| 116 | *N*-[2-(5,6,7-Trimethoxy-4-oxo-1,4-dihydro-quinazolin-2-yl)-1,2,3,4-tetrahydro-isoquinolin-5-yl]-cyclobutanecarboxamidine | 199.5-200.8 | |
| 117 | *N*-[2-(5,6,7-Trimethoxy-4-oxo-1,4-dihydro-quinazolin-2-yl)-1,2,3,4-tetrahydro-isoquinolin-5-yl]-furan-2-carboxamidine | 223.9-225.9 | |
| 118 | 5,6,7-Trimethoxy-2-[5-(1-methyl-4,5-dihydro-1*H*-imidazol-2-yl)-3,4-dihydro-1*H*-isoquinolin-2-yl]-1H-quinazolin-4-one | | 450 |
| 119 | 2-(6,7-Dimethoxy-3,4-dihydro-1*H*-isoquinolin-2-yl)-5-isopropyl-6,7-dimethoxy-1*H*-quinazolin-4-one | 243-244 (HCl Salt) | |
| 120 | 2-(6,7-Dimethoxy-3,4-dihydro-1*H*-isoquinolin-2-yl)-5-(4-fluoro-phenyl)-6,7-dimethoxy-1*H*-quinazolin-4-one | 220-225 | |
| 121 | 2-(6,7-Dimethoxy-3,4-dihydro-1*H*-isoquinolin-2-yl)-6;7-dimethoxy-5-methyl-1*H*-quinazolin-4-one | 260-268 (HCl Salt) | |
| 122 | *N,N*-Dimethyl-*N*'-[2-(5,6,7-trimethoxy-4-oxo-1,4-dihydro-quinazolin-2-yl)-1,2,3,4-tetrahydro-isoquinolin-5-yl]-acetamidine | 223.5-226.6 | |
| 123 | *N*-[2-(6,7-Dimethoxy-5-methyl-4-oxo-1,4-dihydro-quinazolin-2-yl)-1,2,3,4-tetrahydro-isoquinolin-5-yl] -cyclobutanecarboxamidine | 252.1-256.1 | |
| 124 | *N*-[2-(6,7-Dimethoxy-5-methyl-4-oxo-1,4-dihydro-quinazolin-2-yl)-1,2,3,4-tetrahydro-isoquinolin-5-yl]-furan-2-carboxamidine | 279.0-282.0 | |
| 125 | *N-*[2-(6,7-Dimethoxy-5-methyl-4-oxo-1,4-dihydro-quinazolin-2-yl)-1,2,3,4-tetrahydro-isoquinolin-5-yl]-butyramidine | 254.3-257.1 | |
| 126 | 6,7-Dimethoxy-5-methyl-2-[5-(1-methyl-4,5-dihydro-1*H*-imidazol-2-yl)-3,4-dihydro-1*H*-isoquinolin-2-yl]-1*H*-quinazolin-4-one | 246-250 | |
| 127 | 6,7-Dimethoxy-5-methyl-2-[5-(2,4,4-trimethyl-4,5-dihydro-imidazol-1-yl)-3,4-dihydro-1*H*-isoquinolin-2-yl]-1*H*-quinazolin-4-one | | 462 |
| 128 | 6,7-Dimethoxy-5-methyl-2-[5-(1-methyl-1*H*-imidazol-2-yl)-3,4-dihydro-1*H*-isoquinolin-2-yl]-1*H*-quinazolin-4-one | | 432 |
| 129 | 6,7-Dimethoxy-2-{5-[1-(2-methoxy-ethyl)-4,5-dihydro-1*H*-imidazol-2-yl]-3,4-dihydro-1*H*-isoquinolin-2-yl}-5-methyl-1*H*-quinazolin-4-one | | 478 |
| 130 | 2-[5-(1-Isopropyl-4,5-dihydro-1*H*-imidazol-2-yl)-3,4-dihydro-1*H*-isoquinolin-2-yl]-6,7-dimethoxy-5-methyl-1H-quinazolin-4-one | | 462.2 |
| 131 | 2-[5-(2,4-Dimethyl-4,5-dihydro-imidazol-1-yl)-3,4-dihydro-1*H*-isoquinolin-2-yl]-6,7-dimethoxy-5-methyl-1*H*-quinazolin-4-one | | 448 |
| 132 | *N*'-[2-(6,7-Dimethoxy-5-methyl-4-oxo-1,4-dihydro-quinazolin-2-yl)-1,2,3,4-tetrahydro-isoquinolin-7-yl]-*N*,*N*-dimethyl-acetamidine | 176-177.1 | |
| 133 | 2-(6,7-Dimethoxy-3,4-dihydro-1*H*-isoquinolin-2-yl)-5-fluoro-6,7-dimethoxy-1*H*-quinazolin-4-one | 260.8-269 | |
| 134 | 5-Chloro-2-(6,7-dimethoxy-3,4-dihydro-1*H*-iso-quinolin-2-yl)-6,7-dimethoxy-1*H*-quinazolin-4-one | 255.4-259.9 | |
| 135 | 5,6,7-Trimethoxy-2-(4-morpholin-4-yl-5,8-dihydro-6*H*-pyrido[3,4-d]pyrimidin-7-yl)-1*H*-quinazolin-4-one | 190-192 (HCl Salt) | 455 |
| 136 | 6,7-Dimethoxy-5-methyl-2-(4-morpholin-4-yl-5,8-dihydro-6*H*-pyrido[3,4-*d*]pyrimidin-7-yl)-1*H*-quinazolin-4-one | >300 | |
| 137 | 6,7-Dimethoxy-5-methyl-2-[4-(morpholine-4-carbonyl)-[1,4]diazepan-1-yl]-1*H*-quinazolin-4-one | 236.6-237.7 (HCl Salt) | 432 |
| 138 | 5,6,7-Trimethoxy-2-{2-[3-(2-methyl-4,5-dihydro-imidazol-1-yl)-phenyl] -pyrrolidin-1-yl}-1*H*-quinazolin-4-one | 205.1-209.1 | |
| 139 | *N*-(2-{3-[1-(5,6,7-Trimethoxy-4-oxo-1,4-dihydro-quinazolin-2-yl)-pyrrolidin-2-yl] -phenylamino}-ethyl)-acetamide | 188.6-191 | |
| 140 | 2-Aziridin-1-yl-5,6,7-trimethoxy-1*H*-quinazolin-4-one | >300. (HCl Salt) | |
| 141 | 2-(8-Aza-bicyclo[3.2.1]oct-8-yl)-6,7-dimethoxy-5-methyl-1*H*-quinazolin-4-one | | 330.3 |
| 142 | *N*¹-{3-[1-(6,7-Dimethoxy-5-methyl-4-oxo-1,4-dihydro-quinazolin-2-yl)-pyrrolidin-3-yl]-phenyl}-*N,N*-dimethyl-acetamidine | 152.2-154.4 | |
| 143 | 2-(5-Chloro-1,3-dihydro-isoindol-2-yl)-5,6,7-trimethoxy-1*H*-quinazolin-4-one hydrochloride | 289-291 | |

### Assay Examples

### EXAMPLE I-1

The potency and selectivity of the inventive compounds as α1A/B antagonists was determined with CHO-K1 cells expressing adrenoceptor subtype α1A-215, α1B or α1D by measuring cAMP accumulation using AlphaScreen.
Cell preparation was accomplished by culturing CHO-α1 cloned cells in Ham's F12 nutrient media supplemented with 10% FBS and G418 (25mg/ml), harvested at 80% confluence, washed with warmed PBS x2, and detached with versene for 5 min. at 37°C. The cultured cells were then resuspended in 40 ml of stimulation buffer (HBSS with 5 mM hepes, 0.1% BSA) and centrifuged at 500-100 rpm for 5 min. The obtained pellet was resuspended in stimulation buffer (with 0.5M IBMX), and the cells were counted. Cells were diluted to the desired number of cells/ml (α1A at 3x10⁶/ml, α1B 15x10⁶/ml, and α1D 20x10⁶/ml).
The compounds being tested were diluted in stimulation buffer (with 0.5M IBMX)₂ from 10⁻⁵ to 10⁻¹¹ (final) dilution, 11 points. 5 µl of each compound was dispensed to 96 well ½ area plates in triplicate. 5 µl of stimulation buffer was dispensed to a norepinephrine (NE) plate. 10 µl of cells were added with anti-cAMP Acceptor beads in stimulation buffer to each plate and incubated for 15 min. at RT (in dark or covered with black plate). Then 5 µl ofNE was added to the antagonist plates, at 1 µM for α1A and 1B and at 100nM for α1D, and then 5 µl serial dilution of NE was added to NE plate. Plates were incubated for 30 min. at RT (in dark or covered with black plate) and 10 µl Donor beads + biotin-cAMP in lysis buffer(5mM Hepes, 0.54%Tween-20, 0.1% BSA) was added. Plates were incubated for 3h. at RT with gentle shaking (in dark or covered with black plate). Plates were read on an AlphaScreen Fusion analyzer, using reagent pursuant to AlphaSreen cAMP detection kit (PerkinElmer Cat#6760600).
Reference compounds norepinephrine, prazosin and vehicle were run in every experiment. In each plate, on column 12, A-D were loaded with only cells to define total count and E-H were loaded with NE 1µM, (total - NE 1µM ), to define 100% NE activity. The values determined for each experimental well on the plate were divided by (total count - NE 1 µM), to determine % of NE activity. All data was plotted using non-liner curve fitting by GraphPad Prism to get pEC₅₀ (pEC₅₀ being the negative logarithm of EC₅₀, *i.e.,* the molar concentration of an agonist which produces 50% of the maximum possible response for that agonist) for norepinephrine and pKb (wherein Kb is the equilibrium dissociation for a competitive antagonist, determined in a functional assay, and being equal to the concentration of antagonist which would occupy 50% of the receptors at equilibrium, units=mol 1⁻¹, and pKb is the negative logarithm of Kb) for prazosin and tested compounds. (AlphaScreen cAMP Detection Kit from PerkinElmer Life Sciences).

Proceeding as in Example I-1, compounds of Formula I were tested and found to be selective alphalA/B-adrenoceptor antagonists.

### Example I-2: [³H]prazosin Binding (Alphal-Adrenoceptor) Assay

AlphalA, alphalB, and alpha1D adrenoceptor transfected CHO-K1 cells, prepared using the methods described by Chang et al., FEBS Lett. 1998, 422:279-283, were grown to confluence in T-162 tissue culture flasks in Ham's F-12 culture medium supplemented with 10% fetal bovine serum, geneticin (150 µg/mL) and streptomycin/penicillin (30 ug/mL/30 µg/mL) at 37° C in 7% CO₂. Cells were harvested by incubating with phosphate-buffered saline (PBS) containing 30 µM EDTA for 5-10 min at 37° C. Cells were pelleted by centrifuging at 500 x g for 5 min, and the pelleted cells were homogenized (Polytron homogenizer) in 10 vols (w/v) of 50 mM Tris, 1 mM EDTA, (homogenisation buffer, pH 7.4 at 4° C). The homogenate was centrifuged at 45,000 x g for 20 min. The pellet was resuspended in the homogenizing buffer and rehomogenized.
The resulting homogenate was centrifuged at 45,000 x g for 20 min. The pellet was resuspended in 50 mM Tris buffer (pH 7.4 at 4° C), aliquoted, frozen, and stored at-80° C for further use.
The membranes were thawed at rt and diluted in assay buffer (50 mM Tris buffer at pH 4) at 37°C and homogenized using the Polytron tissue disrupter. The membranes were incubated with the radioligand ([³H]prazosin, NEN, 0.1- 0.5 nM) and test compound at 37°C for 30 min. The membranes were then filtered over polyethyleneimine-treated GF/B unifilter plates using a Packard Filtermate Harvester and washed with ice-cold 50 mM Tris-HCl, 1 mM EDTA buffer (3 x 3 sec. washes). Scintillation cocktail was added to the filter plates and bound radioligand determined by liquid scintillation spectrophotometry.
For each experiment, total binding (in the absence of any test or reference compounds) and non-specific binding (10 µM phentolamine) were determined. For each sample tested, the concentration producing 50% inhibition of binding (IC₅₀) and Hill Slope (n_{H}) was determined using iterative non-linear curve fitting techniques with Kaleidagraph (Synergy Software) or other appropriate software. If the radioligand K_{D} was known, the inhibition dissociation constant (K_{I}) of each ligand was determined according to the method of Cheng and Prusoff (Cheng and Prusoff, Biochem.Pharmacol., 1973, 22, 3099-3108).

Proceeding as in Example I-2, compounds of Formula I were tested and found to be selective alpha1A/B-adrenoceptor antagonists.

### Example I-3: Dog In Vivo Intraurethral and Blood Pressure Assay

The following describes an in vivo assay for measuring the relative effect of test compounds on hypogastric nerve stimulation-induced increases in intraurethral pressure and phenylephrine-induced increases in diastolic blood pressure in anesthetized dog. Male Mongrel dogs (10 to 20 kg) were fasted for 12 to 18 hours and anesthetized with phenobarbital sodium (36 mg/kg, *i.v.*). An endotracheal tube was inserted and thereafter the lungs were mechanically ventilated with room air. The right femoral vein was isolated and cannulated with two polyethylene cannulae, one for the administration of a continuous infusion of phenobarbital sodium (5 to 10 mg/kg/hr) and the other for bolus administration of test substances. The right femoral artery was isolated and cannulated to the abdominal aorta with a fluid filled polyethylene cannula connected to an external pressure transducer for monitoring diastolic aortic pressure (DAP). The bladder was exposed via a ventral midline abdominal incision and emptied of urine through a 22 gauge needle. The bladder was cannulated through a stab incision with a water filled balloon catheter connected to an external pressure transducer for monitoring prostatic intraurethral pressure (IUP). The right hypogastric nerve (HGN) was carefully isolated and attached to a Dastre's electrode for nerve stimulation.
The preparation was allowed to stabilize for at least 20-30 minutes and must have had a stable basal IUP for not less than 15 minutes prior to commencement of the assay protocol. The HGN was stimulated (20-50V, 10Hz, 10 msec pulse train for 10 sec) to induce a measurable increase in IUP and then phenylephrine (PE) was administered by bolus injection (6 µg/kg, i.v.) to induce a measurable increase in DAP. The HGN stimulation and PE bolus injection were repeated every 5 minutes until three consecutive reproducible increases in IUP and DAP were achieved. Test compound was administered and 10 minutes later the HGN stimulation and PE bolus injection were repeated. Test compound was administered approximately every 20 minutes, increasing the dose until maximal or near maximal inhibition of the increases in IUP and DAP is attained.

Proceeding as in Example I-3, compounds of Formula I were tested and found to selectively inhibit the HGN stimulation-induced increases in IUP. In contrast, prazosin inhibited increases in IUP and DAP in similar fashion.

Compounds of formula I are active in the above assays. For some examplary compounds the following table shows corresponding data.

| Compound | pKi | | |
|---|---|---|---|
| | alpha 1A | alpha 1B | alpha 1D |
| 2-[4-(1H-Imidazol-2-yl)-piperazin-1-yl]-5,6,7-tri-methoxy-1H-quinazolin-4-one | 8.95 | 8.60 | 6.34 |
| 2-[4-(2-Imidazol-1-yl-ethyl)-piperidin-1-yl]-6,7-di-methoxy-5-methyl-1H-quinazolin-4-one | 8.28 | 8.66 | 7.06 |
| 4-(4-Fluoro-phenyl)-9-(5,6,7-trimethoxy-4-oxo-1,4-dihydro-quinazolin-2-yl)-1-oxa-4,9-diazaspiro [5.5]undecan-3-one | 8.21 | 7.86 | 6.59 |
| *N*-[2-(6,7-Dimethoxy-5-methyl-4-oxo-1,4-dihydro-quinazolin-2-yl)-1,2,3,4-tetrahydro-isoquinolin-5-ylmethyl]-*N*-methyl-2-methylamino-acetamide | 8.8 | 8.8 | 7.1 |

## Claims

1. A compound of the Formula I 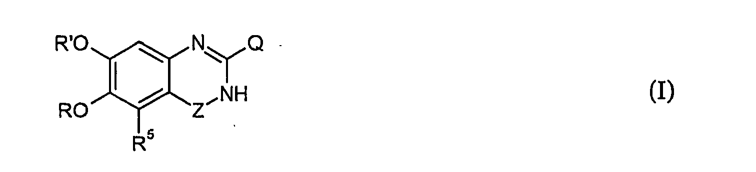 wherein
Z is-C(=O)-or-S(=O)₂-;
R and R' are lower alkoxy;
R⁵ is selected from halogen, cyano, hydroxy, optionally substituted phenyl, -R⁶, and -OR⁶;
R⁶ is alkyl, alkoxyalkyl, hydroxyalkyl, optionally substituted benzyloxyalkoxy, optionally substituted phenoxy, aminoalkyl, optionally substituted aryl, optionally substituted heteroaryl, cycloalkyl, or cycloalkyloxy; and
Q is a monocyclic or bicyclic heterocyclic ring selected from (S), (T), (U), and (V) 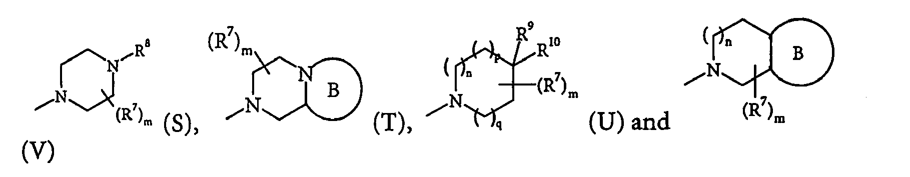
wherein
B is an optionally-substituted fused aryl or heteroaryl ring;
R⁷ is attached to any available carbon atom of the piperidinyl or piperazinyl ring and at each occurrence is independently selected from alkyl, substituted alkyl, halogen, cyano, hydroxy, alkoxy, haloalkoxy, amino, and alkylamino; or alternatively, wherein Q is ring (T), two R⁷ groups attached to different carbon atoms may be taken together to form a carbon-carbon bridge of one to two bridgehead carbon atoms;
R⁸ is -K-R¹⁴;
R⁹ and R¹⁰ are (i) independently selected from -L-R¹⁵, or alternatively, (ii) R⁹ and R¹⁰ are taken together to form an optionally-substituted spirocyclic ring;
K and L are independently selected from a bond, optionally-substituted C₁₋₄akylene, -M₁-O-M₂-, -M₁-C(=O)-M₂-, -M₁-C(O)₂-M₂-, -M₁ C(=O)NR¹⁶-M₂-, and -M₁-NR¹⁶-M₂-, wherein M₁ and M₂ are selected from a bond and optionally-substituted C₁₋₄alkylene;
R¹⁴ and R¹⁵ are independently selected from hydrogen, optionally-substituted alkyl, aryl, heteroaryl, cycloalkyl, or heterocyclo, provided that if K or L is a bond or -NR¹⁶-, then R¹⁴ and R¹⁵ are not selected from phenyl, pyridyl, or pyrimidinyl having a para substituent that is CO₂R²², wherein R²² is selected from hydrogen, alkyl, aryl, araylalkyl, guanidinyl, hydroxy, alkoxy, aryloxy, and arylalkyloxy;
R¹⁶ is selected from hydrogen and alkyl;
*m* is 0, 1, 2, 3, or 4;
*n* is 0 or 1;
*p* is 0, 1 or 2; and
*q* is 0 or 1; or
an isomer or pharmaceutically-acceptable salt, hydrate, or prodrug thereof.

2. The compound of claim 1 selected from
2-[4- (1H-imidazol-2-yl)-piperazin-1-yl] -5,6,7-trimethoxy-1H-quinazolin-4-one;
2- [4-(2-imidazol-1-yl-ethyl)-piperidin-1-yl]-6,7-dimethoxy-5-methyl- 1H-quinazolin-4one;
4-(4-fluoro-phenyl)-9-(5,6,7-trimethoxy-4-oxo-1,4-dihydro-quinazolin-2-yl)-1-oxa-4,9-diaza-spiro[5.5]undecan-3-one; and
N-[2-(6,7-dimethoxy-5-methyl-4-oxo-1,4-dihydro-quinazolin-2-yl)-1,2,3,4-tetrahydro-isoquinolin-5-ylmethyl]-*N*-methyl-2-methylamino-acetamide.

3. The compound of claim 1 for use as therapeutic active substance.

4. A pharmaceutical composition comprising a therapeutically effective amount of at least one compound of claim 1 and at least one pharmaceutically acceptable carrier.

5. Use of a compound of claim 1 for the manufacture of a medicament comprising a compound according to claim 1 for treating a disease state in a patient modulated via an alpha-1A/B adrenoceptor.

6. The use of claim 5 wherein the disease state is selected from disorders and symptoms of the urinary tract, sexual dysfunction, benign prostatic hypertrophy, and pain.

## Patentansprüche

1. Verbindung der Formel I 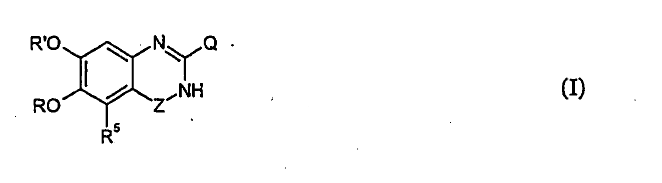 wobei
Z -C(=O)- oder -S(=O)₂- ist;
R und R' Niederalkoxy sind;
R⁵ aus Halogen, Cyano, Hydroxy, gegebenenfalls substituiertem Phenyl, -R⁶ und -OR⁶ ausgewählt ist;
R⁶ Alkyl, Alkoxyalkyl, Hydroxyalkyl, gegebenenfalls substituiertes Benzyloxyalkoxy, gegebenenfalls substituiertes Phenoxy, Aminoalkyl, gegebenenfalls substituiertes Aryl, gegebenenfalls substituiertes Heteroaryl, Cycloalkyl oder Cycloalkyloxy ist; und
Q ein monocyclischer oder bicyclischer heterocyclischer Ring ist, der aus (S), (T), (U) und (V) ausgewählt ist 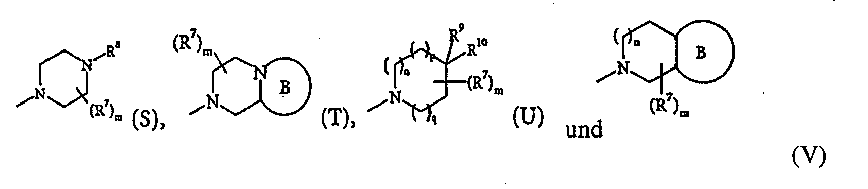 wobei
B ein gegebenenfalls substituierter kondensierter Aryl- oder Heteroarylring ist;
R⁷ an ein beliebiges verfügbares Kohlenstoffatom des Piperidinyl- oder Piperazinylrings gebunden ist und bei jedem Vorkommen unabhängig aus Alkyl, substituiertem Alkyl, Halogen, Cyano, Hydroxy, Alkoxy, Halogenalkoxy, Amino und Alkylamino ausgewählt ist; oder alternativ dazu, wenn Q der Ring (T) ist, zwei Reste R⁷, die an verschiedene Kohlenstoffatome gebunden sind, zusammengenommen werden können, um eine Kohlenstoff-Kohlenstoffbrücke aus ein oder zwei Brückenkopf-Kohlenstoffatomen zu bilden;
R⁸ -K-R¹⁴ ist;
R⁹ und R¹⁰ (i) unabhängig aus -L-R¹⁵ ausgewählt ist, oder alternativ dazu (ii) R⁹ und R¹⁰ zusammengenommen werden, um einen gegebenenfalls substituierten spirocyclischen Ring zu bilden;
K und L unabhängig aus einer Bindung, gegebenenfalls substituiertem C₁₋₄-Alkylen, -M₁-O-M₂-, -M₁-C(=O)-M₂-, -M₁-C(O)₂-M₂-, -M₁-C(=O)NR¹⁶-M₂- und -M₁-NR¹⁶-M₂- ausgewählt sind, wobei M₁ und M₂ aus einer Bindung und gegebenenfalls substituiertem C₁₋₄-Alkylen ausgewählt sind;
R¹⁴ und R¹⁵ unabhängig aus Wasserstoff, gegebenenfalls substituiertem Alkyl, Aryl, Heteroaryl, Cycloalkyl oder Heterocyclo ausgewählt sind, mit der Maßgabe, dass wenn K oder L eine Bindung oder -NR¹⁶- ist, R¹⁴ und R¹⁵ nicht aus Phenyl, Pyridyl oder Pyrimidinyl mit einem para-Substituenten, welcher CO₂R²² ist, ausgewählt sind, wobei R²² aus Wasserstoff, Alkyl, Aryl, Arylalkyl, Guanidinyl, Hydroxy, Alkoxy, Aryloxy und Arylalkyloxy ausgewählt ist;
R¹⁶ aus Wasserstoff und Alkyl ausgewählt ist;
m 0, 1, 2, 3 oder 4 ist;
n 0 oder 1 ist;
p 0, 1 oder 2 ist; und
q 0 oder 1 ist; oder
ein Isomer oder pharmazeutisch verträgliches Salz, Hydrat oder Prodrug davon.

2. Verbindung gemäß Anspruch 1, ausgewählt aus
2-[4-(1H-Imidazol-2-yl)piperazin-1-yl]-5,6,7-trimethoxy-1H-chinazolin-4-on;
2-[4-(2-Imidazol-1-yl-ethyl)piperidin-1-yl]-6,7-dimethoxy-5-methyl-1H-chinazolin-4-on;
4-(4-Fluorphenyl)-9-(5,6,7-trimethoxy-4-oxo-1,4-dihydrochinazolin-2-yl)-1-oxa-4,9-diaza-spiro[5.5]undecan-3-on; und
N-[2-(6,7-Dimethoxy-5-methyl-4-oxo-1,4-dihydro-chinazolin-2-yl)-1,2,3,4-tetrahydroisochinolin-5-yl-methyl]-N-methyl-2-methylamino-acetamid.

3. Verbindung gemäß Anspruch 1 zur Verwendung als therapeutischen Wirkstoff.

4. Arzneimittel, umfassend eine therapeutisch wirksame Menge mindestens einer Verbindung gemäß Anspruch 1 und mindestens einen pharmazeutisch verträglichen Träger.

5. Verwendung einer Verbindung gemäß Anspruch 1 zur Herstellung eines Medikaments, umfassend eine Verbindung gemäß Anspruch 1 zur Behandlung eines Erkrankungszustands bei einem Patienten, welcher durch einen Alpha-1A/B-Adrenozeptor moduliert wird.

6. Verwendung gemäß Anspruch 5, wobei der Erkrankungszustand aus Störungen und Symptomen des Harntraktes, sexueller Funktionsstörung, gutartiger Prostatahypertrophie und Schmerz ausgewählt ist.

## Revendications

1. Composé de Formule I 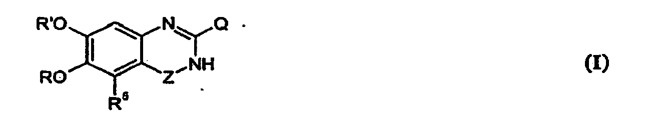 dans laquelle
Z est -C(=O)- ou -S(=O)₂-;
R et R' sont des groupes alkoxy inférieurs;
R⁵ est choisi parmi les groupes halogéno, cyano, hydroxy, phényle facultativement substitué, -R⁶ et -OR⁶;
R⁶ est un groupe alkyle, alkoxyalkyle, hydroxyalkyle, benzyloxyalkoxy facultativement substitué, phénoxy facultativement substitué, aminoalkyle, aryle facultativement substitué, hétéroaryle facultativement substitué, cycloalkyle ou cycloalkyloxy; et
Q est un hétérocycle monocyclique ou bicyclique choisi parmi (S), (T), (U) et (V) 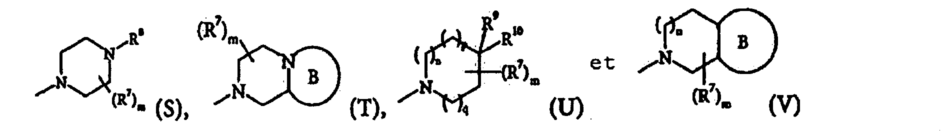 où
B est un cycle aryle ou hétéroaryle condensé facultativement substitué ;
R⁷ est attaché à tout atome de carbone disponible du cycle pipéridinyle ou pipérazinyle et est choisi indépendamment, à chaque occurrence, parmi les groupes alkyle, alkyle substitué, halogéno, cyano, hydroxy, alkoxy, halogénalkoxy, amino et alkylamino ; ou, en variante, lorsque Q est le cycle (T), deux groupes R⁷ attachés à des atomes de carbone différents peuvent être pris ensemble pour former un pont carbone-carbone d'un à deux atomes de carbone de pontage ;
R⁸ est -K-R¹⁴;
R⁹ et R¹⁰ sont (i) choisis indépendamment parmi -L-R¹⁵, ou bien (ii) R⁹ et R¹⁰ sont pris ensemble pour former un cycle spirocyclique facultativement substitué ;
K et L sont choisis indépendamment parmi une liaison, un groupe alkylène en C₁ à C₄ facultativement substitué, -M₁-O-M₂-, -M₁-C (=O)-M₂-, -M₁-C(O)₂-M₂-, -M₁-C (=O) NR¹⁶-M₂et -M₁-NR¹⁶-M₂-, où M₁ et M₂ sont choisis parmi une liaison et un groupe alkylène en C₁ à C₄ facultativement substitué ;
R¹⁴ et R¹⁵ sont choisis indépendamment parmi l'hydrogène, les groupes alkyle, aryle, hétéroaryle, cycloalkyle ou hétérocyclo facultativement substitués, à condition que si K ou L est une liaison ou -NR¹⁶-, alors R¹⁴ et R¹⁵ ne soient pas choisis parmi les groupes phényle, pyridyle et pyrimidinyle ayant un substituant en *para* qui est CO₂R²², où R²² est choisi parmi l'hydrogène, les groupes alkyle, aryle, arylalkyle, guanidinyle, hydroxy, alkoxy, aryloxy et arylalkyloxy ;
R¹⁶ est choisi parmi l'hydrogène et les groupes alkyle ;
m est 0, 1, 2, 3 ou 4 ;
n est 0 ou 1 ;
p est 0, 1 ou 2 ; et
q est 0 ou 1 ; ou
un isomère ou un sel, hydrate ou promédicament pharmaceutiquement acceptable de celui-ci.

2. Composé selon la revendication 1, choisi parmi
la 2-[4-(1H-imidazole-2-yl)pipérazine-1-yl]-5,6,7-triméthoxy-1H-quinazoline-4-one ;
la 2-[4-(2-imidazole-1-yléthyl)pipéridine-1-yl]-6,7-diméthoxy-5-méthyl-1H-quinazoline-4-one ;
la 4-(4-fluorophényl)-9-(5,6,7-triméthoxy-4-oxo-1,4-dihydroquinazoline-2-yl)-1-oxa-4,9-diazaspiro[5.5]-undécane-3-one ; et
le *N*-[2-(6,7-diméthoxy-5-méthyl-4-oxo-1,4-dihydro-quinazoline-2-yl)-1,2,3,4-tétrahydroisoquinoléine-5-yl-méthyl]-*N*-méthyl-2-méthylamino-acétamide.

3. Composé selon la revendication 1, pour son utilisation comme substance active thérapeutique.

4. Composition pharmaceutique comprenant une quantité thérapeutiquement efficace d'au moins un composé de la revendication 1 et au moins un support pharmaceutiquement acceptable.

5. Utilisation d'un composé de la revendication 1, pour la fabrication d'un médicament comprenant un composé selon la revendication 1 destiné au traitement chez un patient d'un état pathologique modulé par un récepteur adrénergique alpha-1A/B.

6. Utilisation selon la revendication 5, dans laquelle l'état pathologique est choisi parmi des troubles et symptômes des voies urinaires, un dysfonctionnement sexuel, l'hypertrophie bénigne de la prostate et la douleur.
